(19) Europäisches Patentamt
European Patent Office
Office européen des brevets

(11) **EP 3 750 995 B1**

(12) **EUROPEAN PATENT SPECIFICATION**

(45) Date of publication and mention of the grant of the patent:
**25.12.2024 Bulletin 2024/52**

(21) Application number: **19751530.7**

(22) Date of filing: **08.01.2019**

(51) International Patent Classification (IPC):
***C12N 15/11*** *(2006.01)* ***C07K 14/00*** *(2006.01)*
***A61K 47/54*** *(2017.01)*

(52) Cooperative Patent Classification (CPC):
**C12N 15/111; A61K 47/6455; A61P 35/00; C07K 14/003;** C12N 15/113; C12N 15/1136; C12N 15/1137; C12N 15/1138; C12N 2310/3181; C12N 2310/331; C12N 2310/3513; C12N 2310/53; C12N 2320/32; C12Y 301/04017

(86) International application number:
**PCT/KR2019/000245**

(87) International publication number:
**WO 2019/156365 (15.08.2019 Gazette 2019/33)**

(54) **PEPTIDE NUCLEIC ACID COMPLEX HAVING ENDOSOMAL ESCAPE CAPACITY, AND USE THEREOF**

PEPTID-NUKLEINSÄURE-KOMPLEX FÄHIGKEIT ZUR FREISETZUNG AUS DEN ENDOSOMEN UND SEINE VERWENDUNG

COMPLEXE D’ACIDE NUCLÉIQUE PEPTIDIQUE AYANT UNE CAPACITÉ D’ÉCHAPPEMENT ENDOSOMAL ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.02.2018 KR 20180015750**

(43) Date of publication of application:
**16.12.2020 Bulletin 2020/51**

(73) Proprietor: **Seasun Therapeutics, Inc.**
**Daejon 34015 (KR)**

(72) Inventors:
- **KIM, Hye Joo**
  **Daejeon 34080 (KR)**
- **YU, Ji-Yeon**
  **Cheongju-si Chungcheongbuk-do 28193 (KR)**
- **LEE, Dong In**
  **Daejeon 34696 (KR)**
- **KANG, Yusun**
  **Daejeon 34014 (KR)**
- **PARK, Hee Kyung**
  **Daejeon 34034 (KR)**

(74) Representative: **Schüssler, Andrea**
**Kanzlei Huber & Schüssler**
**Truderinger Strasse 246**
**81825 München (DE)**

(56) References cited:
**EP-A1- 3 498 844**
**WO-A1-2017/015109**
**WO-A1-2018/030789**
**WO-A1-95/01369**
**WO-A1-99/13719**
**JP-A- 2008 220 366**
**KR-A- 20120 079 104**
**KR-B1- 101 255 149**
**US-A- 5 719 262**
**US-A1- 2005 250 148**

- **S. D. LAUFER ET AL: "Peptide-Mediated Cellular Delivery of Oligonucleotide-Based Therapeutics In Vitro: Quantitative Evaluation of Overall Efficacy Employing Easy to Handle Reporter Systems", CURRENT PHARMACEUTICAL DESIGN, vol. 14, no. 34, December 2008 (2008-12-01), pages 3637 - 3655, XP055035059, ISSN: 1381-6128, DOI: 10.2174/138161208786898806**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **TAKEHIKO SHIRAISHI: "Peptide nucleic acid (PNA) cell penetrating peptide (CPP) conjugates as carriers for cellular delivery of antisense oligomers", 1 July 2011 (2011-07-01), XP055333188, Retrieved from the Internet <URL:https://www.ncbi.nlm.nih.gov/pmc/articles/PMC3324339/pdf/adna-2-90.pdf> [retrieved on 20170109], DOI: 10.4161/adna.2.3**
- **DE COSTA N TILANI S ET AL: "Differential DNA and RNA sequence discrimination by PNA having charged side chains", BIOORGANIC & MEDICINAL CHEMISTRY LETTERS, ELSEVIER, AMSTERDAM, NL, vol. 24, no. 10, 28 March 2014 (2014-03-28), pages 2360 - 2363, XP028646710, ISSN: 0960-894X, DOI: 10.1016/J.BMCL.2014.03.059**
- **TORU SUGIYAMA ET AL: "Chiral Peptide Nucleic Acids with a Substituent in the N-(2-Aminoethy) glycine Backbone", MOLECULES, vol. 18, no. 1, 27 December 2012 (2012-12-27), pages 287 - 310, XP055411282, DOI: 10.3390/molecules18010287**
- **N. TILANI S. DE COSTA ET AL: "Evaluating the Effect of Ionic Strength on Duplex Stability for PNA Having Negatively or Positively Charged Side Chains", PLOS ONE, vol. 8, no. 3, 6 March 2013 (2013-03-06), pages e58670 - 1, XP055638961, DOI: 10.1371/journal.pone.0058670**
- **DRAGULESCU-ANDRASI ANCA ET AL: "Cell-permeable GPNA with appropriate backbone stereochemistry and spacing binds sequence-specifically to RNA", CHEMICAL COMMUNICATIONS, ROYAL SOCIETY OF CHEMISTRY, UK, no. 2, 2 December 2004 (2004-12-02), pages 244 - 246, XP002439659, ISSN: 1359-7345, DOI: 10.1039/B412522C**
- **CHERAGHI NASIM ET AL: "Pore formation and the key factors in antibacterial activity of aurein 1.2 and LLAA inside lipid bilayers, a molecular dynamics study", BBA - BIOMEMBRANES ACTA, ELSEVIER, AMSTERDAM, NL, vol. 1860, no. 2, 10 October 2017 (2017-10-10), pages 347 - 356, XP085304063, ISSN: 0005-2736, DOI: 10.1016/J.BBAMEM.2017.10.009**
- **XING-LIANG ZHAO ET AL: "Delivery of cell-penetrating peptide-peptide nucleic acid conjugates by assembly on an oligonucleotide scaffold", SCIENTIFIC REPORTS, vol. 5, no. 1, 27 November 2015 (2015-11-27), XP055669937, DOI: 10.1038/srep17640**
- **MARTIN, M. E. ET AL.: "Peptide-Guided Gene Delivery", THE AAPS JOURNAL, vol. 9, no. 1, 9 February 2007 (2007-02-09), pages E18 - E29, XP035718877, doi:10.1208/aapsj0901003**


Remarks:

The complete document including Reference Table(s) and the Sequence Listing(s) can be downloaded from the EPO website

## Description

### Technical Field

The present invention relates to a nucleic acid complex having a novel structure, which may introduce a bioactive nucleic acid into cells, a composition for treating or diagnosing a disease comprising the same, and more particularly to a nucleic acid complex which comprises a material for facilitating endosomal escape and in which a bioactive nucleic acid and a carrier peptide nucleic acid are complementarily bound to each other and a composition for use in treating or diagnosing a disease comprising the same.

### Background Art

US Patent 5,719,262 concerns a novel class of compounds, known as peptide nucleic acids, which bind complementary DNA and RNA strands more strongly than the corresponding DNA and RNA strands, and exhibit increased sequence specificity and solubility.

WO 99/13719 A1 concerns the chemical modification of DNA using peptide nucleic acid conjugates. These are complexes comprising a nucleic acid molecule and a conjugated peptide nucleic acid (PNA). These complexes increase the efficiency of expression of a particular gene.

WO 2017/015109 A1 relates to compounds, compositions and methods useful for modulating gene expression of multiple targets.

Conventionally, the exploration of new drugs is based on screening various compounds through computer research, and the majority of screened compounds target proteins.

Unlike traditional drugs, nucleic acid drugs inhibit the expression of target-specific messenger RNA (mRNA), making it possible to address research areas in which diseases could not be treated by conventional drugs that target proteins (Kole R. et al., Nature Rev. Drug Discov. 2012; 11; 125-140., Wilson C. et al., Curr. Opin. Chem. Bio. 2006; 10: 607-614.).

Despite the excellent effects and various applications of gene expression regulation based on oligonucleic acid, there are many obstacles to overcome in the development of nucleic acid-based therapeutic agents. For example, oligonucleic acid can be damaged by nuclease or the like, and the passage of oligonucleic acids through the cell membrane by passive diffusion is impossible due to the electrical properties (charges) and size of these oligonucleic acids. In order to overcome these problems, efforts have been continuously made to ensure biological stability through modification of nucleic acids. For modified artificial nucleic acids, it becomes possible to increase their affinity for target nucleic acids without loss of biological activity.

Peptide nucleic acid (PNA), a type of modified artificial nucleic acid, is an artificial nucleic acid having a (2-aminoethyl)-glycine peptide backbone introduced therein, and has the property of strongly binding to RNA and DNA, each having a nucleotide sequence complementary thereto. Particularly, the peptide nucleic acid is resistant to nuclease and has high biological stability, and studies on therapeutic agents based on various oligonucleic acids have been conducted. However, the peptide nucleic acid has the disadvantage of being difficult to introduce into cells, because it is electrically neutral (Joergensen M. et al., Oligonucleotides 2011, 21; 29-37).

Owing to the performance and advantages of nucleic acids as drugs, various clinical trials using nucleic acids have been conducted. Despite the increasing applications of nucleic acid-based therapeutic agents, the use of carriers for intracellular introduction is extremely limited. For example, clinical trials have been performed using a strategy (method) that delivers oligonucleic acid-based drugs into cells or tissues by the use of nanoparticles, cationic liposomes and polymeric nanoparticles. However, most of these clinical trials do not include delivery systems, and rely mainly on direct introduction of nucleic acids by parenteral administration routes, including intramuscular injection, intraocular administration and subcutaneous injection.

In addition, the cell membrane permeability of oligonucleic acids is considerably low, and in particular, DNA or RNA is negatively charged. For this reason, these oligonucleic acids cannot pass through the hydrophobic phospholipid bilayer of the cell membrane, and thus delivery thereof into cells through simple diffusion is difficult. The use of a virus carrier such as retrovirus or AAV (adeno-associated virus) makes it possible to introduce oligonucleic acids into cells, but has risks, such as unintended immune activity and the possible recombination of oncogenes (Couto L. B. et al., Curr. Opin. Pharmacol. 2010, 5; 534-542.).

For this reason, development of nucleic acid carriers based on non-viral oligonucleic acids having low cytotoxicity and low immune activity is of increasing importance. As a result, techniques of introducing nucleic acids using cationic lipids, liposomes, stable nucleic acid lipid particles (SNALPs), polymers and cell-penetrating peptides have been developed (Zhi D. et al., Bioconjug. Chem. 2013, 24; 487-519., Buyens K. et al., J. Control Release, 2012, 158; 362-70., ROSSI, J. J. et al., Gene Ther. 2006, 13: 583-584., Yousefi A. et al., J. Control Release, 2013, 170; 209-18., Trabulo S. et al., Curr. Pharm. Des. 2013, 19; 2895-923.).

These nucleic acid delivery techniques have functional moieties by direct binding, include a complex formation step, and have problems associated with the endosomal escape efficiency of liposome structures, in vivo toxicity, and the like. Consequently, it is required to improve the function of introducing oligonucleic acids and overcome problems associated with production procedures and side effects.

Meanwhile, bioactive nucleic acids generally enter cells through the formation of cell organelles called endosomes via receptor-mediated endocytosis. For effective expression of bioactive nucleic acids and treatment, bioactive nucleic acids in endosomes should be capable of escaping the endosomes and moving to the nucleus or performing their functions in the cytoplasm. Therefore, the process of escaping from the endosome to the cytoplasm, that is, "endosomal escape" is essential (D. W. Pack, A. S. Hoffman, S. Pun, P. S. Stayton, "Design and development of polymers for gene delivery," Nat. Rev. Drug. Discov., 4, 581-593, 2005).

In connection with this, the present inventors found that a nucleic acid complex comprising a bioactive nucleic acid complementarily bound to a carrier peptide nucleic acid modified to be generally positively charged has surprisingly increased cell permeability, and expression of a target gene can be very efficiently regulated using the nucleic acid complex. Based on this finding, the present inventors filed an application for a patent for a new structure having low cytotoxicity, an ability to allow a bioactive nucleic acid to permeate into cells, and an increased ability to regulate gene expression (PCT/KR2017/008636)..

Since then, the present inventors have conducted continued studies, and as a result, have found that, when a material for facilitating endosomal escape is bound to the above-described structure, the intracellular activity of the bioactive nucleic acid contained in the structure is significantly increased, thereby completing the present invention.

The above information disclosed in this Background section is only for enhancement of understanding of the background of the present invention. Therefore, it may not contain information that forms the conventional art that is already known in the art to which the present invention pertains.

### Summary of the Invention

An object of the present invention is to provide a nucleic acid complex in which a bioactive nucleic acid comprising a material for facilitating endosomal escape and a carrier peptide nucleic acid modified to be generally positively charged are complementarily bound to each other, and a composition for diagnosing or treating disease comprising the same.

To achieve the above object, the present invention provides a nucleic acid complex as defined in claim 1. Preferred embodiments are defined in the dependent claims.

Thus, the present invention provides a nucleic acid complex having a structure of the following Structural Formula (1):

$$[\ mA \equiv mC^{(+)}\ ]$$

wherein,

- A represents a bioactive nucleic acid having either a sequence capable of binding to a target gene or a target gene sequence, preferably wherein the bioactive nucleic acid is selected from the group consisting of DNA, RNA, LNA, PNA, and modified nucleic acids;
- C represents a carrier peptide nucleic acid capable of binding to the bioactive nucleic acid;
- '≡' represents complementary binding between the bioactive nucleic acid and the carrier peptide nucleic acid;
- 'm' represents a material for facilitating endosomal escape of the bioactive nucleic acid and the carrier peptide nucleic acid, preferably wherein the material for facilitating endosomal escape is bound to the 5'-end and/or 3'-end of each of the bioactive nucleic acid and the carrier peptide nucleic acid;
- the material for facilitating endosomal escape is GLFDIIKKIAESF (SEQ ID NO: 86) or histidine(10);
- the bioactive nucleic acid represented by A is generally negatively charged or neutral;
- $C^{(+)}$ indicates that the carrier peptide nucleic acid is generally positively charged; and
- the carrier peptide nucleic acid comprises one or more peptide nucleic acid monomers modified such that the carrier peptide nucleic acid is generally positively charged.

The present invention also provides a composition for use in diagnosing a disease comprising the nucleic acid complex of Structural Formula (1), and a composition for preventing or treating a disease comprising the nucleic acid complex of Structural Formula (1).

The present invention also provides the nucleic acid complex of Structural Formula (1) for use in preventing or treating a disease.

The present application also describes the use of the nucleic acid complex of Structural Formula (1) in the manufacture of a medicament for preventing or treating a disease.

**Brief Description of Drawings**

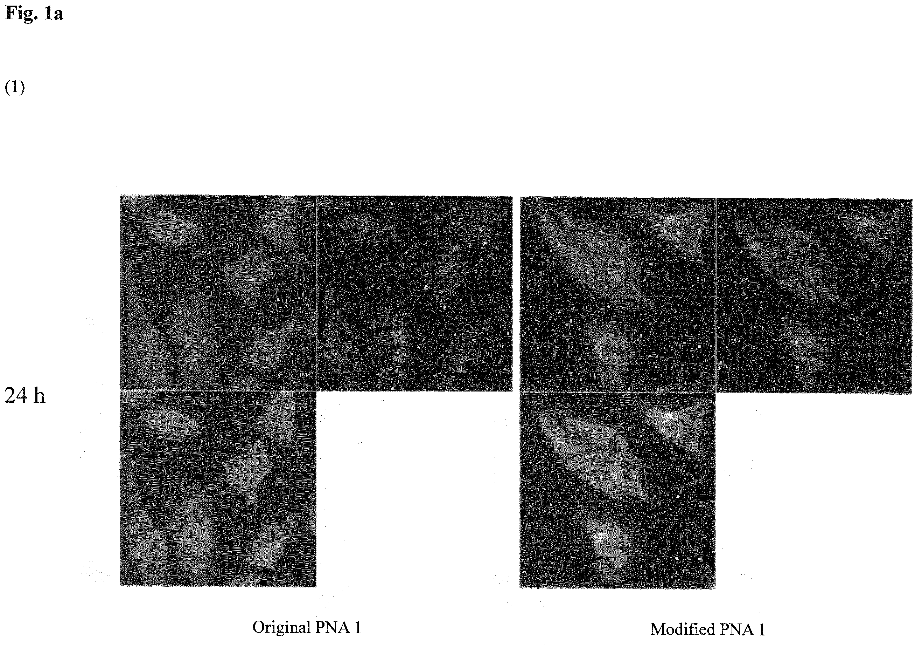

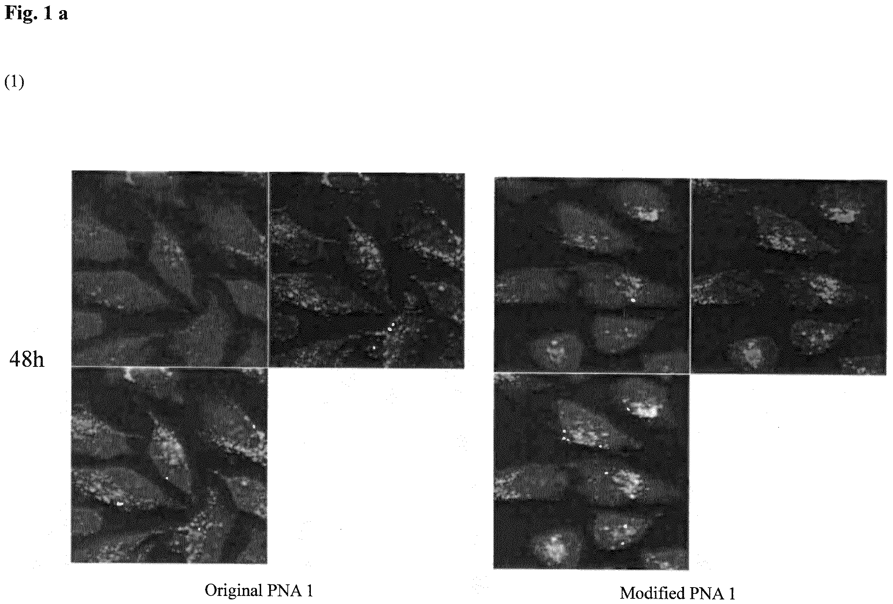

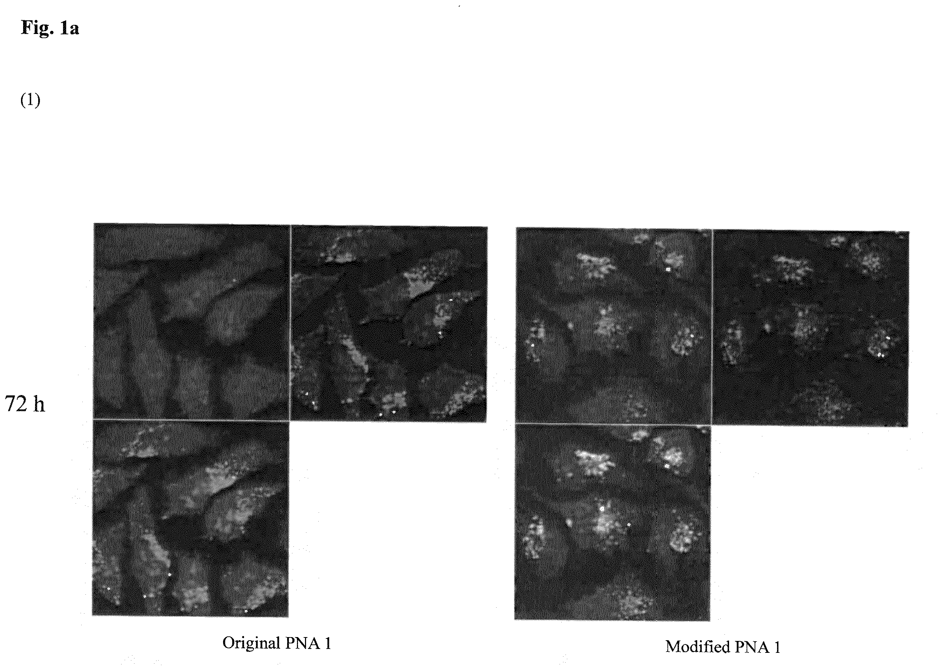

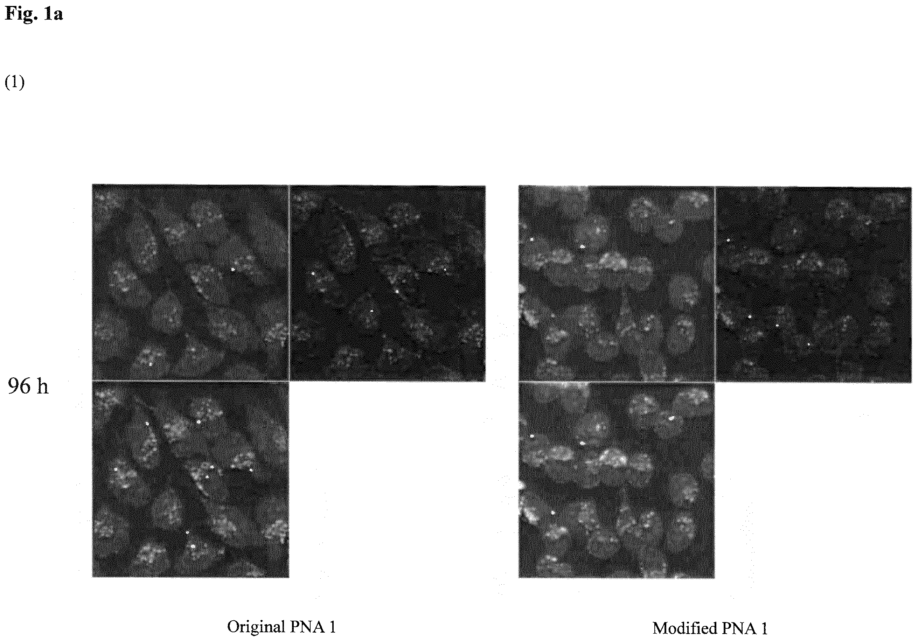

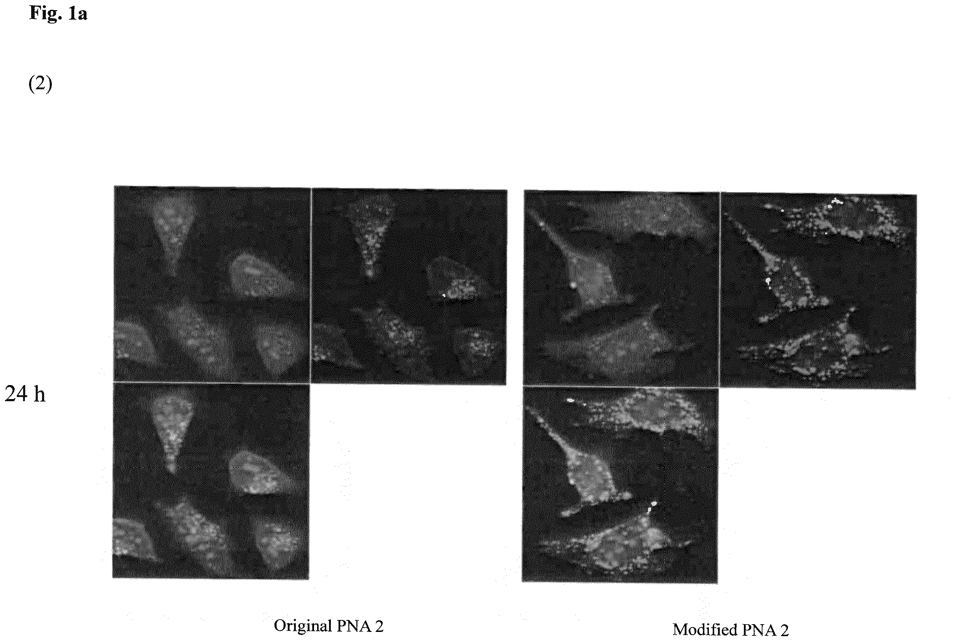

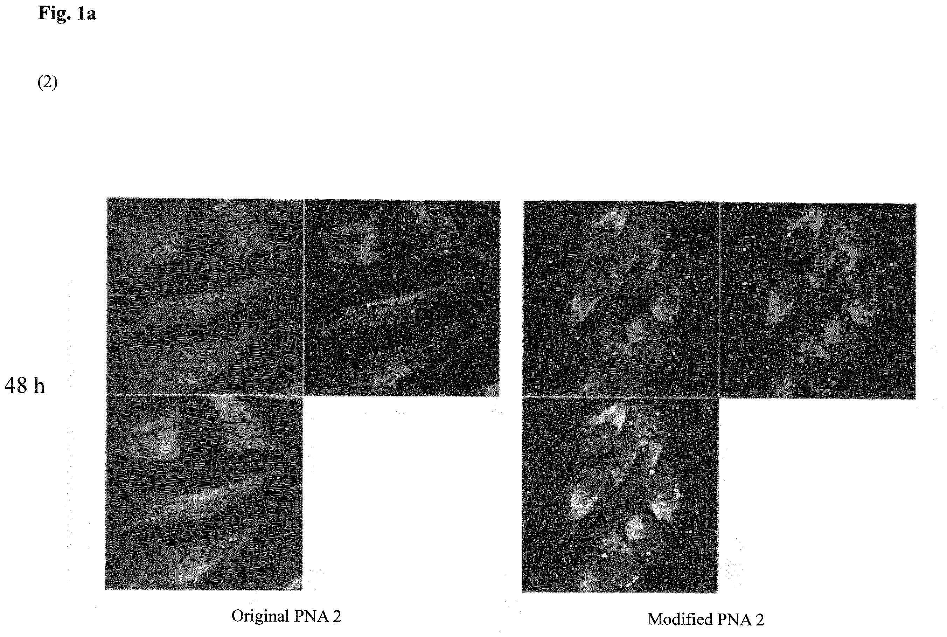

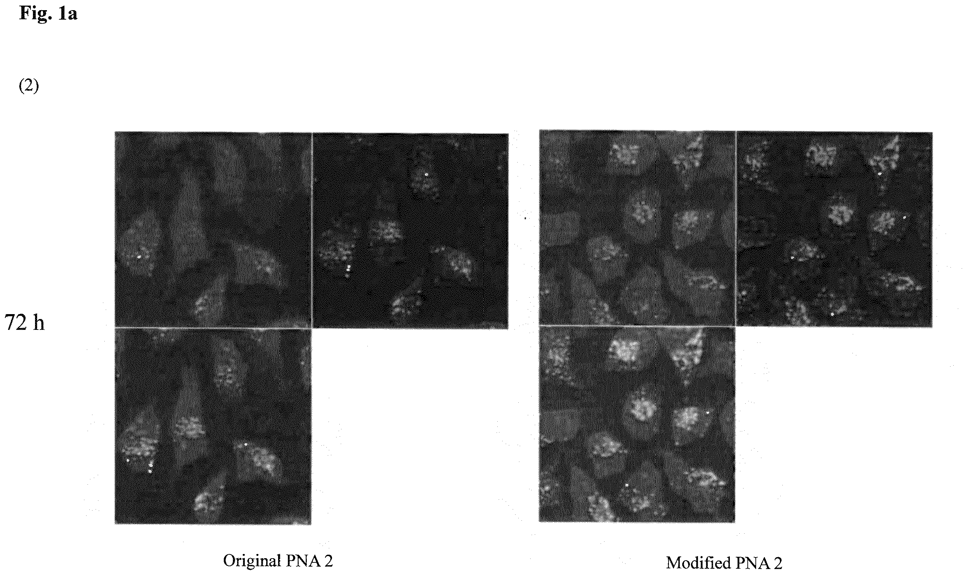

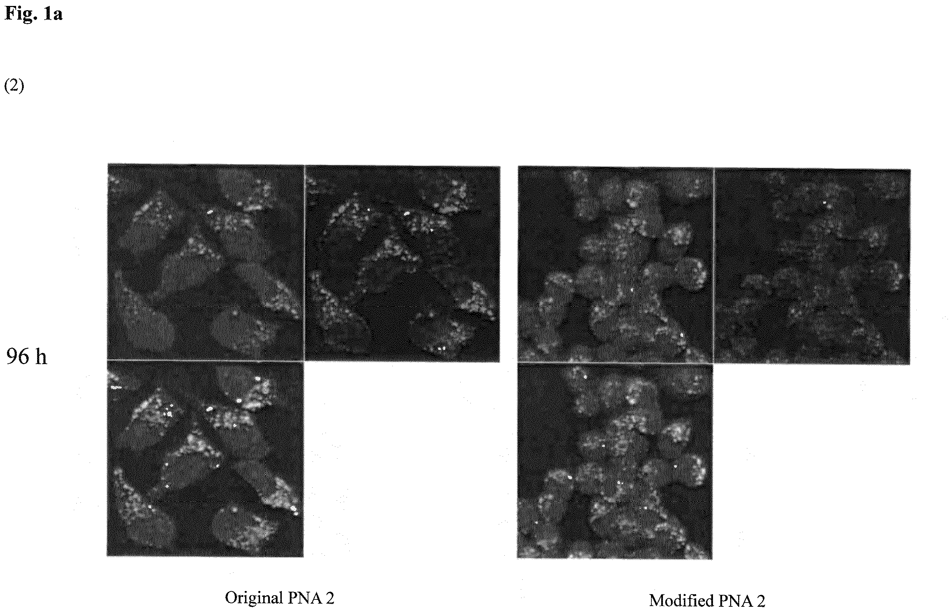

FIGS. 1a to 1e show the results of confirming that the use of a nucleic acid complex of Structural Formula (1), which comprises a VEGF-specific bioactive peptide nucleic acid, is released into the cytoplasm after penetration into human uterine cancer cell lines, and show the results of analyzing changes in the cell viabilities of human breast cancer cell lines and lung cancer cell lines, inhibition of expression of VEGF and downstream proteins thereof, and induction of apoptosis.

(a) Changes in cell permeability and endosomal escape of the nucleic acid complex in HeLa;
(b) change in cell viability of MDA-MB-231 cells;
(c) change in cell viability of A549 cells;
(d) changes in expression of VEGF and downstream proteins thereof; and
(e) analysis of apoptosis induced by VEGF inhibition.

FIGS. 2a to 2g show the results of evaluating the tumor growth inhibitory effect of a nucleic acid complex of Structural Formula (1), which comprises a VEGF-specific bioactive peptide nucleic acid, in mice transplanted with human breast cancer cell lines.

(a) *In vivo* animal test design;
(b) changes in mouse body weight;
(c) changes in tumor volume;
(d) changes in tumor weight;
(e) changes in tumor appearance;
(f) changes in hepatotoxicity markers;
(g) changes in expression of VEGF and downstream proteins thereof.

FIG. 3 shows the results of evaluating the tumor growth inhibitory effect of a nucleic acid complex of Structural Formula (1), which comprises a PD-L1-specific bioactive peptide nucleic acid, in mice transplanted with human breast cancer cell lines.

FIG. 4 shows the results of confirming that the use of a nucleic acid complex of Structural Formula (1), which comprises an androgen receptor-specific bioactive peptide nucleic acid, inhibits expression of androgen receptor and downstream proteins thereof in human prostate cancer cell lines.

(A) the case in which a complex comprising a bioactive peptide nucleic acid (SEQ ID NO: 12 or SEQ ID NO: 13) and each of carrier peptide nucleic acids (SEQ ID NO: 16 to SEQ ID NO: 19 or SEQ ID NO: 24 to SEQ ID NO: 27) was used;
(B) the case in which a complex comprising a bioactive peptide nucleic acid (SEQ ID NO: 14 or SEQ ID NO: 15) and each of carrier peptide nucleic acids (SEQ ID NO: 20 to SEQ ID NO: 23 or SEQ ID NO: 28 to SEQ ID NO: 31) was used.

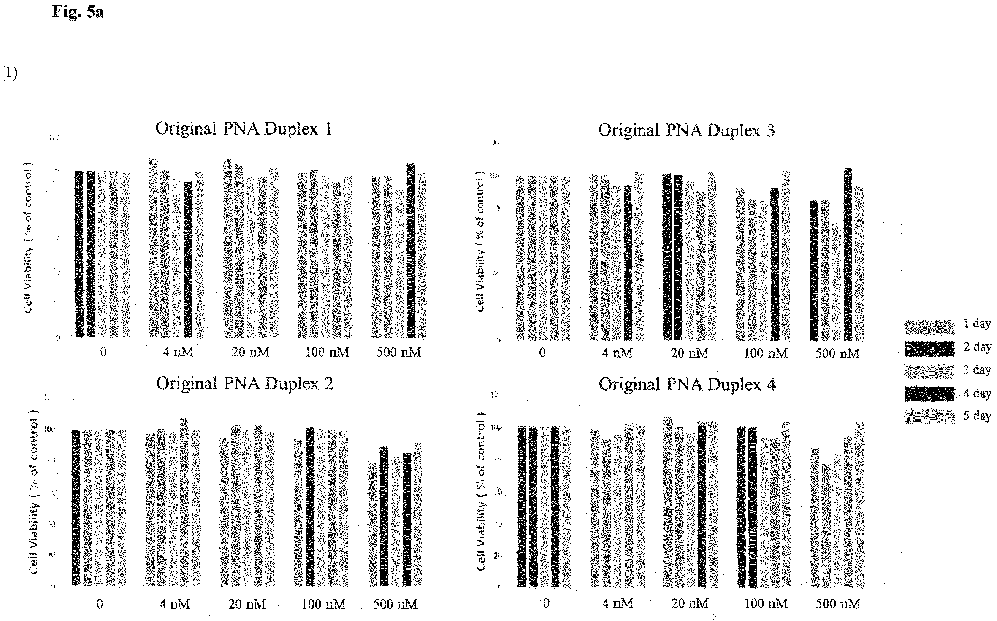

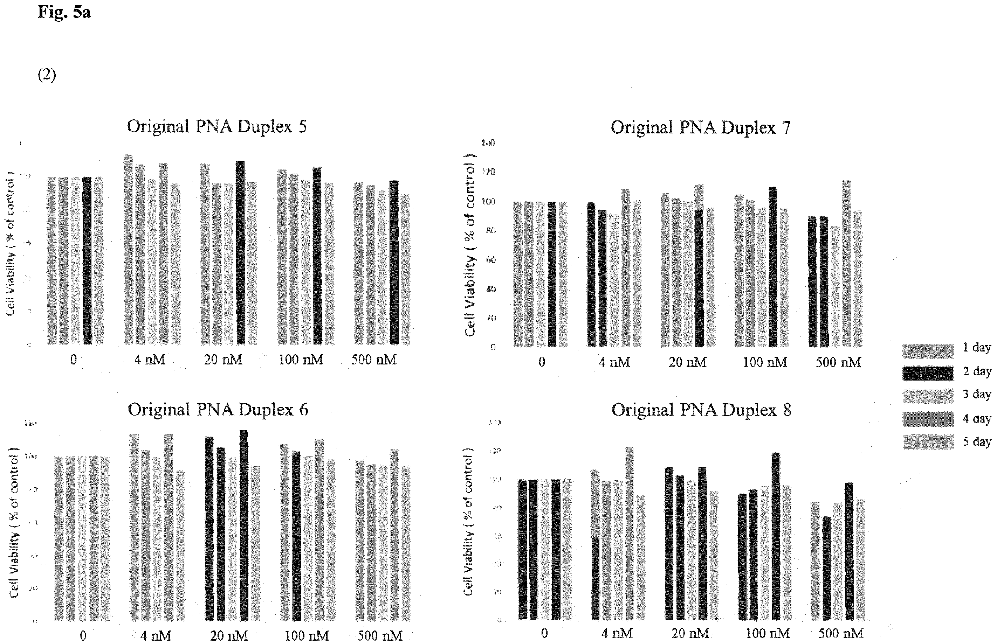

FIGS. 5a to 5c show the results of confirming that the use of a nucleic acid complex of Structural Formula (1), which comprises a clusterin-specific bioactive peptide nucleic acid, changes the cell viability of human prostate cancer cells and inhibits the expression of clusterin-associated proteins.

(a) change in cell viability of PC-3 cells in the case in which a complex comprising a bioactive peptide nucleic acid (SEQ ID NO: 32 or SEQ ID NO: 33) and each of carrier peptide nucleic acids (SEQ ID NO: 36 to SEQ ID NO: 39 or SEQ ID NO: 44 to SEQ ID NO: 47) was used;
(b) change in cell viability of PC-3 cells in the case in which a complex comprising a bioactive peptide nucleic acid (SEQ ID NO: 34 or SEQ ID NO: 35) and each of carrier peptide nucleic acids (SEQ ID NO: 40 to SEQ ID NO: 43 or SEQ ID NO: 48 to SEQ ID NO: 51) was used;
(c) changes in expression of clusterin and related proteins;

(1) the case in which a complex comprising a bioactive peptide nucleic acid (SEQ ID NO: 32 or SEQ ID NO: 33) and each of carrier peptide nucleic acids (SEQ ID NO: 36 to SEQ ID NO: 39 or SEQ ID NO: 44 to SEQ ID NO: 47) was used;
(2) the case in which a complex comprising a bioactive peptide nucleic acid (SEQ ID NO: 34 or SEQ ID NO:

35) and each of carrier peptide nucleic acids (SEQ ID NO: 36 to SEQ ID NO: 39 or SEQ ID NO: 44 to SEQ ID NO: 47) was used.

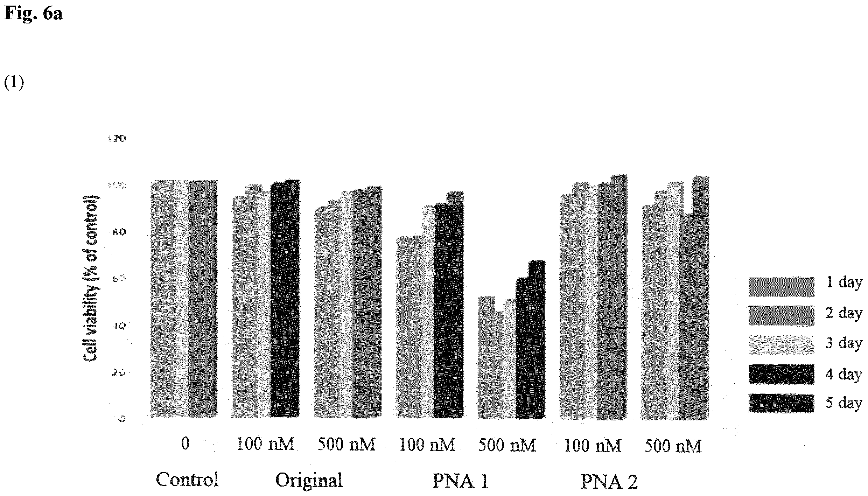

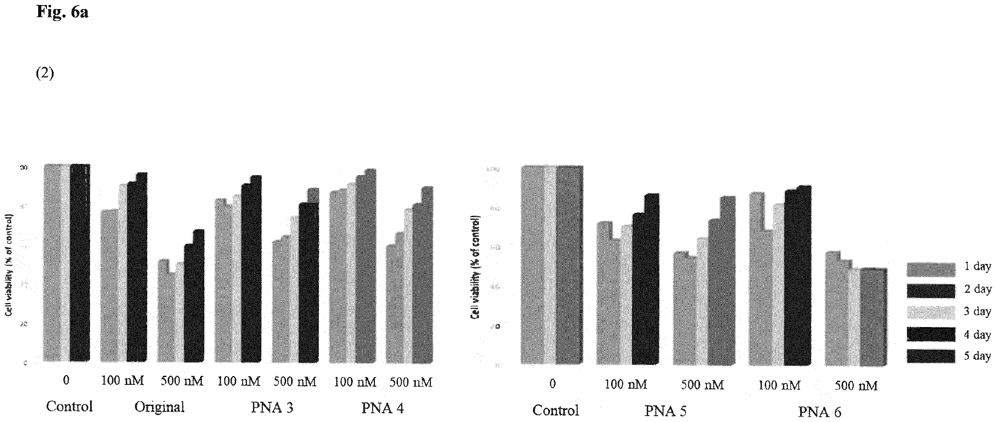

FIGS. 6a to 6f show the results of confirming that the use of a nucleic acid complex of Structural Formula (1), which comprises a VEGF-specific bioactive peptide nucleic acid, changes the cell viability of human retinal pigment epithelium cell lines, inhibits expression of VEGF and downstream proteins thereof, and inhibits angiogenesis in the mouse retina through intravitreal injection and ocular administration.

(a) Change in cell viability of ARPE-19 cells;
(b) changes in expression of VEGF and downstream proteins thereof;
(c) analysis of angiogenesis in the mouse retina one week after intravitreal injection in the case in which a complex comprising a bioactive peptide nucleic acid (SEQ ID NO: 2) and a carrier peptide nucleic acid (SEQ ID NO: 8) was used;
(d) and (e) analysis of angiogenesis in the mouse retina through intravitreal injection in the case in which a complex comprising a bioactive peptide nucleic acid (SEQ ID NO: 2) and a carrier peptide nucleic acid (SEQ ID NO: 6 or SEQ ID NO: 8) was used;
(f) analysis of angiogenesis in the mouse retina through ocular administration in the case in which a complex comprising a bioactive peptide nucleic acid (SEQ ID NO: 2) and a carrier peptide nucleic acid (SEQ ID NO: 8) was used.

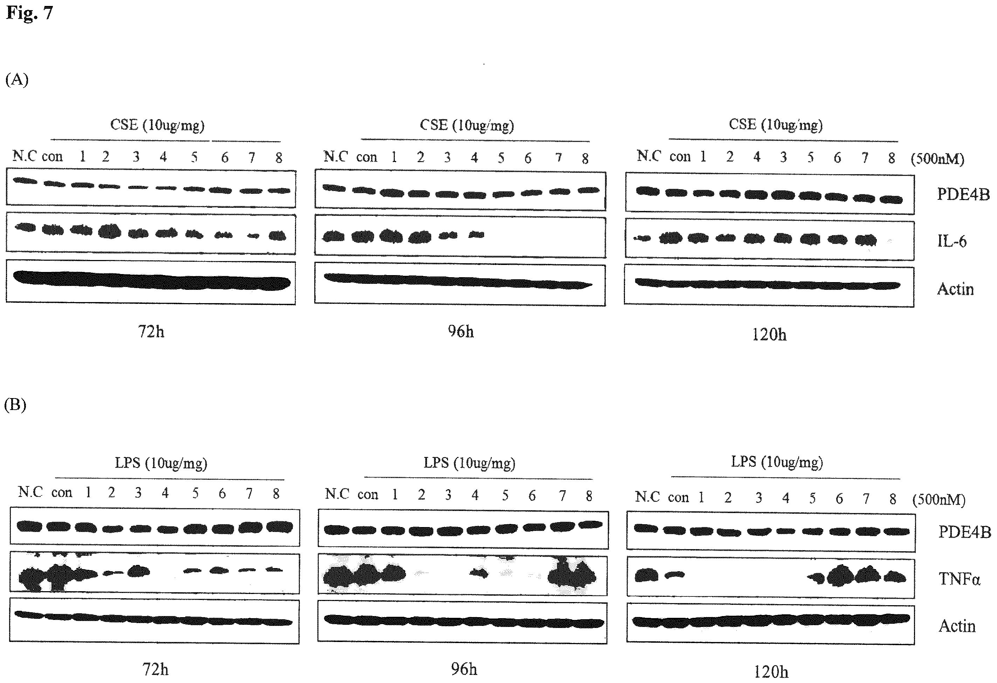

FIG. 7 shows the results of confirming that the use of a nucleic acid complex of Structural Formula (1), which comprises a PDE4B-specific bioactive peptide nucleic acid, inhibits expression of PDE4B and inflammation-related proteins in human respiratory epithelial cells and human lung cancer cell lines.

(A) Analysis of expression of PDE4B and inflammatory proteins in NCI-H292 in the case in which a complex comprising a bioactive peptide nucleic acid (SEQ ID NO: 52 or SEQ ID NO: 55) and each of carrier peptide nucleic acids (SEQ ID NO: 56 to SEQ ID NO: 63) was used;
(B) analysis of expression of PDE4B and inflammatory proteins in A549 in the case in which a complex comprising a bioactive peptide nucleic acid (SEQ ID NO: 52 or SEQ ID NO: 55) and each of carrier peptide nucleic acids (SEQ ID NO: 56 to SEQ ID NO: 63) was used.

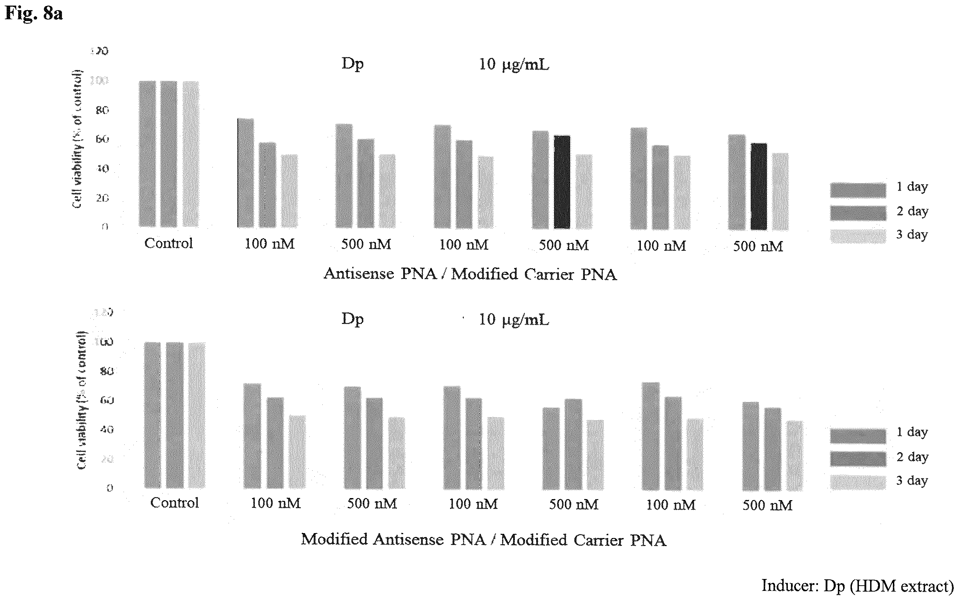

FIGS. 8a to 8f show the results of confirming that the use of a nucleic acid complex of Structural Formula (1), which comprises a TLR2-specific bioactive peptide nucleic acid, exhibits expression of TLR2 associated with atopy.

(a) Change in cell viability of HaCaT in the case in which a complex comprising a bioactive peptide nucleic acid (SEQ ID NO: 64 or SEQ ID NO: 65) and each of carrier peptide nucleic acids (SEQ ID NO: 67 to SEQ ID NO: 71) was used;
(b) inhibition of expression of TLR2 and downstream gene proteins thereof in HaCaT in the case in which a complex comprising a bioactive peptide nucleic acid (SEQ ID NO: 64 or SEQ ID NO: 65) and each of carrier peptide nucleic acids (SEQ ID NO: 67 to SEQ ID NO: 71) was used;
(c) change in atopic dermatitis phenotype in animal model in the case in which a complex comprising a bioactive peptide nucleic acid (SEQ ID NO: 65) and each of carrier peptide nucleic acids (SEQ ID NO: 67 or SEQ ID NO: 70) was used;
(d) changes in IgE and TARC concentrations in sera of animal model;
(e) change in atopy phenotype of animal model through H&E staining;
(f) changes in inflammatory markers of animal model through immunostaining.

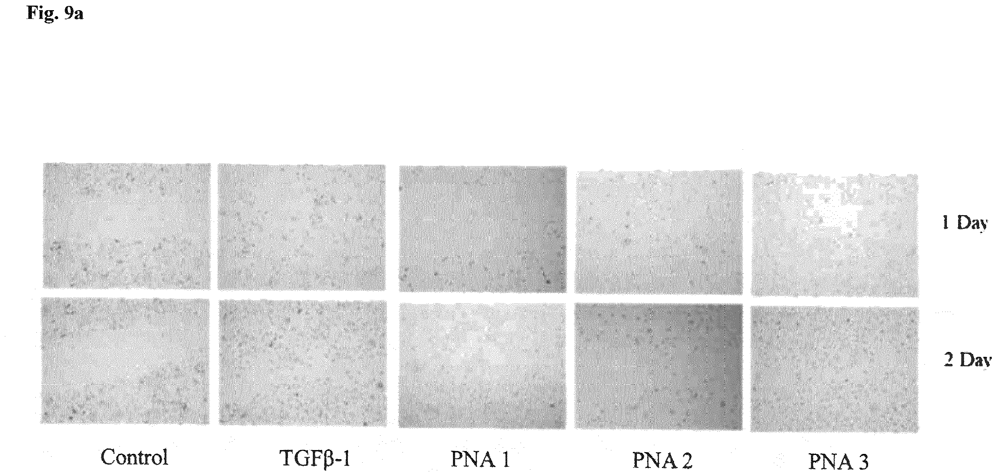

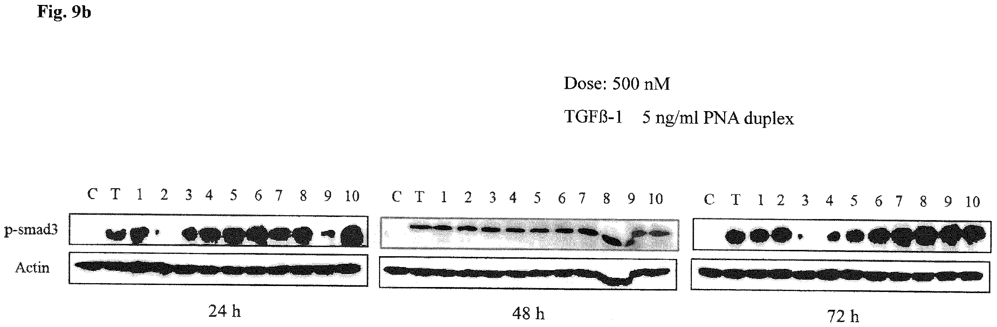

FIGS. 9a and 9b show the results of confirming that the use of a nucleic acid complex of Structural Formula (1), which comprises a Smad3-specific bioactive peptide nucleic acid, inhibits expression of Smad3 associated with skin regeneration.

(a) Wound healing assay in HaCaT in the case in which a complex comprising a bioactive peptide nucleic acid (SEQ ID NO: 73) and each of carrier peptide nucleic acids (SEQ ID NO: 75 or SEQ ID NO: 77 and SEQ ID NO: 78) was used;
(b) inhibition of protein expression of Smad3 in the case in which a complex comprising a bioactive peptide nucleic acid (SEQ ID NO: 72 or SEQ ID NO: 73) and each of carrier peptide nucleic acids (SEQ ID NO: 74 to SEQ ID NO: 78) was used.

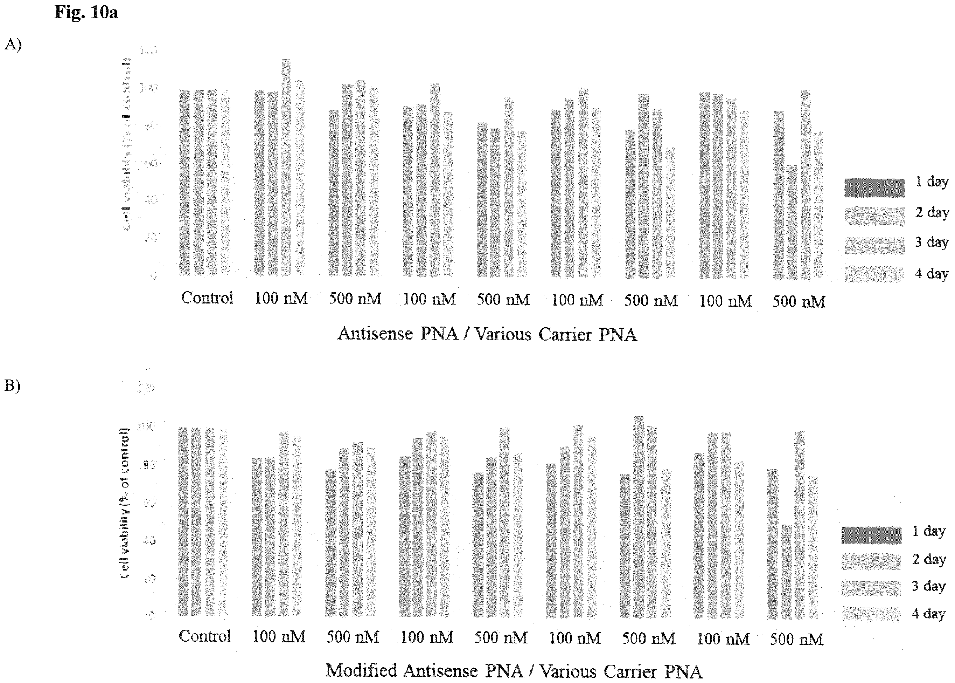

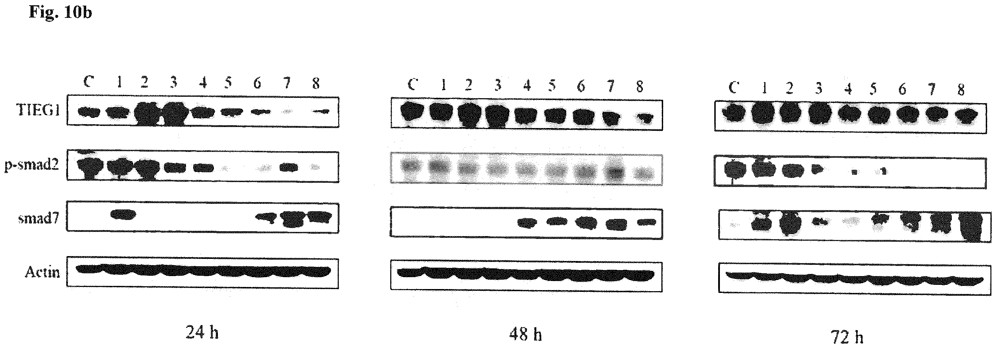

FIGS. 10a and 10b show the results of confirming that the use of a nucleic acid complex of Structural Formula (1), which comprises a TIEG1-specific bioactive peptide nucleic acid, inhibits expression of TIEG1 associated with keloids.

(a) Change in cell viability of KEL-FIB cells in the case in which a complex comprising a bioactive peptide nucleic acid (SEQ ID NO: 79 or SEQ ID NO: 80) and each of carrier peptide nucleic acids (SEQ ID NO: 81 to SEQ ID NO: 84) was used;
(b) changes in expression of TIEG1 gene and downstream genes thereof in KEL-FIB in the case in which a complex comprising a bioactive peptide nucleic acid (SEQ ID NO: 79 or SEQ ID NO: 80) and each of carrier peptide nucleic acids (SEQ ID NO: 81 to SEQ ID NO: 84) was used.

**Detailed Description**

Unless otherwise defined, all technical and scientific terms used in the present specification have the same meanings as commonly understood by those skilled in the art to which the present disclosure pertains. In general, the nomenclature used in the present specification is well known and commonly used in the art.

In one example of the present invention, it has been found that the use of a nucleic acid complex, in which a bioactive nucleic acid, comprising a material for facilitating endosomal escape, and a carrier peptide nucleic acid, are complementarily bound to each other, may increase the intracellular delivery efficiency of the bioactive nucleic acid and easily regulate target gene expression.

Therefore, in one aspect, the present invention is directed to a nucleic acid complex having a structure as defined in claim 1.

Particularly, the present invention is directed to a nucleic acid complex having a structure of the following Structural Formula (1):

$$[\ mA \equiv mC^{(+)}\ ]$$

wherein,

A represents a bioactive nucleic acid having either a sequence capable of binding to a target gene or a target gene sequence, preferably wherein the bioactive nucleic acid is selected from the group consisting of DNA, RNA, LNA, PNA, and modified nucleic acids;
C represents a carrier peptide nucleic acid capable of binding to the bioactive nucleic acid;
'≡' represents complementary binding between the bioactive nucleic acid and the carrier peptide nucleic acid;
'm' represents a material for facilitating endosomal escape of the bioactive nucleic acid and the carrier peptide nucleic acid, preferably wherein the material for facilitating endosomal escape is bound to the 5'-end and/or 3'-end of each of the bioactive nucleic acid and the carrier peptide nucleic acid;
the material for facilitating endosomal escape is GLFDIIKKIAESF (SEQ ID NO: 86) or histidine(10);
the bioactive nucleic acid represented by A is generally negatively charged or neutral;
$C^{(+)}$ indicates that the carrier peptide nucleic acid is generally positively charged; and
the carrier peptide nucleic acid comprises one or more peptide nucleic acid monomers modified such that the carrier peptide nucleic acid is generally positively charged.

In the present invention, "bioactive nucleic acid" refers to a nucleic acid having a complementary sequence capable of binding to a target gene whose expression is to be reduced, particularly a complementary sequence capable of binding to the mRNA of the target gene, or comprising a sequence that promotes expression of a target gene to be expressed. Specifically, it refers to a nucleic acid which is involved in gene expression regulation, such as inhibiting or promoting expression of the gene of interest. The bioactive nucleic acid may be a nucleic acid having a sequence complementary to a target gene whose expression is to be decreased or increased, or may be a nucleic acid having a sequence complementary to the sequence of a single-stranded RNA, such as pre-mRNA, miRNA or mRNA.

In particular, "bioactive nucleic acid" in the present invention may bind to a target gene or a nucleotide sequence comprising the same *in vitro* or *in vivo*, thereby activating or inhibiting the characteristic function of the target gene (e.g., transcript expression or protein expression) or regulating splicing of pre-mRNA (e.g., exon skipping). Here, the nucleotide sequence may be a gene regulatory sequence, or a gene coding sequence, or a splicing regulatory sequence. The gene regulatory sequence may be selected from among a promoter, a transcriptional enhancer, a 5' untranslated region, a 3' untranslated region, a viral packaging sequence, and a selection marker. The gene coding sequence may be an exon or an intron, and the gene coding sequence may be located within 10, 5, 3 or 1 kb or 500, 300 or 200 bp from the transcription initiation site of the gene. For example, the gene coding sequence may be located upstream or downstream of the initiation site. Furthermore, the splicing regulatory sequence may comprise a sequence associated with exon skipping, cryptic splicing, pseudo-splice site activation, intron retention, or alternative splicing deregulation.

In the present invention, "carrier peptide nucleic acid" refers to a nucleic acid whose bases partially or completely bind complementarily to the bioactive nucleic acid, thereby imparting functionality. Carrier peptide nucleic acids that may be used in the present invention include not only peptide nucleic acid (PNA), but also modified nucleic acids similar thereto. The peptide nucleic acid is preferable, but is not limited thereto.

In the present invention, a material for facilitating endosomal escape may be bound to the 5'-end or 3'-end of each of the bioactive nucleic acid and the carrier peptide nucleic acid. Preferably, it is possible to use a form in which a material for facilitating endosomal escape is bound to the 5'-end of each of the bioactive nucleic acid and the carrier peptide nucleic acid.

The material for facilitating endosomal escape is linked by covalent bonds to the bioactive nucleic acid and the carrier peptide nucleic acid, but is not limited thereto.

In the present invention, the "material for facilitating endosomal escape" may facilitate endosomal escape of the bioactive nucleic acid by increasing the osmotic pressure in endosomes or destabilizing the endosomal membrane. This means that the material helps the bioactive nucleic acid move more efficiently and quickly to the nucleus or cytoplasm so as to meet and act on a target gene (D. W. Pack, A. S. Hoffman, S. Pun, P. S. Stayton, "Design and development of polymers for gene delivery," Nat. Rev. Drug. Discov., 4, 581-593 (2005)).

The material for facilitating endosomal escape may be bound via a linker to the 5'-end or 3'-end of the bioactive nucleic acid or the carrier peptide nucleic acid, but is not limited thereto.

According to the present invention the material for facilitating endosomal escape is selected from the peptides GLFDIIKKIAESF (SEQ ID NO: 86) and histidine(10).

In the present invention, each of the bioactive nucleic acid and the carrier peptide nucleic acid may comprise 2 to 50, preferably 5 to 30, more preferably 10 to 25, most preferably 15 to 17 nucleic acid monomers.

Moreover, the bioactive nucleic acid may be composed of natural nucleic acid bases and/or modified nucleic acid monomers.

In the present invention, the bioactive nucleic acid may be selected from the group consisting of DNA, RNA, and modified nucleic acids, i.e., PNA (peptide nucleic acid), PMO (phosphorodiamidate morpholino oligonucleotide), LNA (locked nucleic acid), GNA (glycol nucleic acid), TNA (threose nucleic acid), antisense oligonucleotide, aptamer, siRNA (small interfering RNA), shRNA (short hairpin RNA), ribozyme, and DNAzyme. Preferably, the bioactive nucleic acid may be selected from the group consisting of DNA, RNA, and modified nucleic acids, i.e., PNA, PMO, LNA, GNA, and TNA, but is not limited thereto.

In the present invention, when a monomer used in the bioactive nucleic acid is PNA, the bioactive nucleic acid is referred to as bioactive peptide nucleic acid, and when another monomer is used, the bioactive nucleic acid is also referred to in the same manner.

In the present invention, the carrier peptide nucleic acid may have a nucleotide sequence which is partially or completely complementary to the bioactive nucleic acid. In particular, the carrier peptide nucleic acid may comprise one or more universal bases, and the carrier peptide nucleic acid may also be completely composed of universal bases.

In the present invention, the bioactive nucleic acid and the carrier peptide nucleic acid may further comprise one or more functional groups selected from the group consisting of phosphodiester, 2'0-methyl, 2' methoxy-ethyl, phosphoramidate, methylphosphonate, and phosphorothioate.

Each of the bioactive nucleic acid and the carrier peptide nucleic acid of the nucleic acid complex may be generally positively charged (cationic), negatively charged (anionic) or neutral.

The term "generally" as used when expressing electrical charge does not mean the electrical property of individual bases, but means the overall electrical properties of the bioactive nucleic acid or the carrier peptide nucleic acid when viewed externally. For example, if the number of negatively charged monomers in the bioactive nucleic acid is larger even though some monomers in the bioactive nucleic acid are positively charged, the bioactive nucleic acid is negatively charged when "generally" viewing the electrical property. If the number of positively charged bases and/or backbones in the carrier peptide nucleic acid is larger even though some bases and/or backbones in the carrier peptide nucleic acid are negatively charged, the carrier peptide nucleic acid is positively charged when "generally" viewing the electrical property.

In this regard, the nucleic acid complex having a structure of Structural Formula (1) according to the present invention may be generally positively charged. In the nucleic acid complex of Structural Formula (1), the bioactive nucleic acid is negatively charged or neutral when generally viewing the electrical property, and the carrier peptide nucleic acid is positively charged when generally viewing the electrical property.

The electrical property of each of the bioactive nucleic acid and the carrier peptide nucleic acid may be imparted using a modified peptide nucleic acid monomer. The modified peptide nucleic acid monomer may comprise, as positively charged carrier peptide nucleic acids, any one or more positively charged amino acids selected from the group consisting of lysine (Lys, K), arginine (Arg, R), histidine (His, H), diamino butyric acid (DAB), ornithine (Orn), and an amino acid analogue. In addition, the modified peptide nucleic acid monomer may comprise, as a negatively charged carrier peptide nucleic acid, glutamic acid (Glu, E), which is a negatively charged amino acid, or a negatively charged amino acid analogue.

In the present invention, the carrier peptide nucleic acid may comprise one or more gamma- or alpha-backbone-modified peptide nucleic acid monomers so as to be generally positively charged.

The gamma- or alpha-backbone-modified peptide nucleic acid monomers may comprise, in the backbone thereof, one or more positively charged amino acids selected from the group consisting of lysine (Lys, K), arginine (Arg, R), histidine (His, H), diamino butyric acid (DAB), ornithine (Orn), and an amino acid analogue, so as to be electrically positive.

In the present invention, modification of the peptide nucleic acid monomers to impart charges may be performed using nucleobase-modified peptide nucleic acid monomers besides the backbone modification. Preferably, the carrier peptide nucleic acid may comprise an amine, triazole or imidazole moiety in its nucleobase so as to be electrically positive, or may comprise carboxylic acid in its base so as to be electrically negative.

In the present invention, the modified nucleic acid monomers of the carrier peptide nucleic acid may further comprise negative charges in the backbone or nucleobase, but the modified peptide nucleic acid monomers preferably comprise a larger number of positively charged monomers than negatively charged monomers such that the carrier peptide nucleic acid is generally positively charged.

Preferably, the nucleic acid complex of Structural Formula (1) according to the present invention may be generally positively charged.

In the nucleic acid complex of Structural Formula (1) according to the present invention, at least one substance selected from the group consisting of a hydrophobic moiety, a hydrophilic moiety, a target antigen-specific antibody, an aptamer, and a fluorescent/luminescent marker may be bound to the bioactive nucleic acid and/or the carrier peptide nucleic acid. Preferably, one or more substances selected from the group consisting of the hydrophobic moiety, the hydrophilic moiety, the target antigen-specific antibody, the aptamer, and the fluorescent/luminescent marker for imaging may be bound to the carrier peptide nucleic acid.

In the present invention, the binding of at least one substance, selected from the group consisting of the hydrophobic moiety, the hydrophilic moiety, the target antigen-specific antibody, the aptamer, the quencher, the fluorescent marker, and the luminescent marker, to the bioactive nucleic acid and/or the carrier peptide nucleic acid, may be via a single covalent bond or a linker-mediated covalent bond, but is not limited thereto (see Table 1). Preferably, cell permeation-, solubility-, stability-, delivery- and imaging-related substances (e.g., hydrophobic moiety, etc.) bound to the nucleic acid carrier are present independently of the bioactive nucleic acid that regulates target gene expression.

In the present invention, complementary binding of the bioactive nucleic acid to the carrier peptide nucleic acid may largely be classified into antiparallel binding and parallel binding. The complementary binding is configured such that the bioactive nucleic acid is released in the presence of a sequence targeted by the bioactive nucleic acid, that is, a sequence complementary to the bioactive nucleic acid.

Antiparallel binding and parallel binding are determined according to 5'-directionality and 3'-directionality in DNA-DNA or DNA-PNA binding. Antiparallel binding is a general DNA-DNA or DNA-PNA binding method. Taking the nucleic acid complex of Structural Formula (1) according to the present invention as an example, antiparallel binding means that the bioactive nucleic acid in the 5' to 3' direction and the carrier peptide nucleic acid in the 3' to 5' direction are bound to each other. Parallel binding shows a somewhat lower binding affinity than antiparallel binding, and means that the bioactive nucleic acid and the carrier peptide nucleic acid are bound to each other in the 5' to 3' direction or the 3' to 5' direction.

In the nucleic acid complex of Structural Formula (1) according to the present invention, the binding affinity between the bioactive nucleic acid and the carrier peptide nucleic acid may preferably be lower than the binding affinity between the bioactive nucleic acid and a gene targeted by the bioactive nucleic acid, particularly the mRNA of the target gene. The binding affinity is determined by melting temperature (Tm).

As a specific example of a method for allowing the binding affinity (melting temperature (Tm)) between the bioactive nucleic acid and the carrier peptide nucleic acid to be lower than the binding affinity between the bioactive nucleic acid and a gene targeted by the bioactive nucleic acid, particularly the mRNA of the target gene, the bioactive nucleic acid and the carrier peptide nucleic acid may be bound to each other by parallel binding or partial specific binding, but the present invention is not limited thereto.

As another example, the carrier peptide nucleic acid may have at least one peptide nucleobase selected from the group consisting of a linker, a universal base, and a peptide nucleobase which has base not complementary to the corresponding base of the bioactive nucleic acid, but the present invention is not limited thereto (see Table 1).

The universal base used in the present invention may be one or more selected from the group consisting of inosine PNA, indole PNA, nitroindole PNA, and abasic PNA, which are bases that bind to natural bases, including adenine, guanine, cytosine, thymine, and uracil, without selectivity, and have lower binding affinity than complementary binding affinity. Preferably, inosine PNA may be used as the universal base.

[Table 1]

| Examples of binding between bioactive nucleic acid and carrier peptide nucleic acid | | | |
|---|---|---|---|
| Type | Complex structure | | Features |
| I | Carrier peptide nucleic acid | 5'-[NNNNN*NNNNNNNNNN*NNNNN]-3' | Partial match 1 (Substitution) |
| | Bioactive nucleic acid | 3'-[NNNNNNNNNNNNNNNNNNNNN]-5' | |
| II | Carrier peptide nucleic acid | 5'-[NNNNNNNNNNNNNNNNNNN]-3' | Partial match 2 (Insertion/Deletion) |
| | Bioactive nucleic acid | 3'-[NNNNNNNNNNNNNNNNNNNNN]-5' | |
| III | Carrier peptide nucleic acid | 5'-[NNNNNNN$$$$NNSNNNNNNNN]-3' | Universal base |
| | Bioactive nucleic acid | 3'-[NNNNNNNNNNNNNNNNNNNNN]-5' | |
| IV | Carrier peptide nucleic acid | 5'-[NNNNNNNNN=NNNNNNNNN]-3' | Linker |
| | Bioactive nucleic acid | 3'-[NNNNNNNNNNNNNNNNNNNNN]-5' | |
| V | Carrier peptide nucleic acid | 5'-[NNNNNNNNNNNNNNNNNNNNN]-3' | Parallel binding |
| | Bioactive nucleic acid | 5'-[NNNNNNNNNNNNNNNNNNNNN]-3' | |

In Table 1 above, N represents nucleobases (ATGC); * represents a sequence which is not complementary to an antisense nucleic acid sequence; $ represents a universal base; = represents a linker; and 5'- and 3'- represent the directionalities of nucleic acid (bases).

The present invention provides a combination of binding form and electrical property of nucleic acids for regulating the function of the nucleic acid complex, may control the particle size and the time of action through the combination of binding form and electrical property of nucleic acids, and may increase cell permeability, solubility and specificity.

In the present invention, the time point at which the bioactive peptide nucleic acid binds to a target sequence in the presence of a target gene (the time of strand displacement of the bioactive nucleic acid to the target sequence, and the time of target specific release and binding of the bioactive nucleic acid) may be controlled by controlling the binding affinity between the carrier peptide nucleic acid and the bioactive peptide nucleic acid.

In the nucleic acid complex of Structural Formula (1) according to the present invention, the time of strand displacement of the bioactive nucleic acid to a target gene and the time of the target specific release and binding of the bioactive nucleic acid may be controlled by the nonspecific bases of the carrier peptide nucleic acid for nonspecific binding of the complex, the presence or absence of universal bases and a linker, and the number and position of the bases. In addition, these may also be controlled by a combination of these conditions with parallel or antiparallel binding which is complementary binding in the complex.

In the present invention, the particle size of the nucleic acid complex of Structural Formula (1) may be 5 nm to 300 nm, preferably 10 nm to 80 nm, most preferably 15 nm to 70 nm.

In the present invention, the particle size of the nucleic acid complex may be controlled by controlling the charge balance between the bioactive nucleic acid and the carrier peptide nucleic acid. Specifically, as the positive charges of the carrier peptide nucleic acid increase, the particle size becomes smaller, but if the positive charges of the carrier peptide nucleic acid exceed a certain level, the particle size becomes larger. In addition, the particle size of the nucleic acid complex is determined by proper charge balance between the bioactive nucleic acid and the carrier peptide nucleic acid depending on the charges of the bioactive peptide nucleic acid of the complex, which is another factor that determines the particle size.

The number of positive charges of the carrier peptide nucleic acid according to the present invention is 1 to 7 (indicating that 1 to 7 positively charged monomers are included), preferably 2 to 5, most preferably 2 to 3, and the net charge of charge balance of the bioactive nucleic acid is negative charge 0 to 5, preferably 0 to 3.

In the present invention, the nucleic acid complex of Structural Formula (1) may be produced by hybridization between the bioactive nucleic acid and the carrier peptide nucleic acid under proper conditions.

As used herein, the term "hybridization" means that complementary single-stranded nucleic acids form a doublestranded nucleic acid. Hybridization can occur when the complementarity between two nucleic acid strands is perfect (perfect match) or when some mismatched residues exist. The degree of complementarity necessary for hybridization may vary depending on hybridization conditions, particularly may be controlled by binding temperature.

In the present invention, a reporter and a quencher capable of quenching the fluorescence of the reporter may be bound to both ends of the bioactive nucleic acid or the carrier peptide nucleic acid. The reporter may be one or more selected from the group consisting of FAM (6-carboxyfluorescein), Texas red, HEX (2',4',5',7'-tetrachloro-6-carboxy-4,7-dichlorofluorescein), and Cy5. Preferably, Cy5 is used. The quencher may be one or more selected from the group consisting of TAMRA (6-carboxytetramethyl-rhodamine), BHQ1, BHQ2 and Dabcyl, but is not limited thereto.

In another aspect, the present invention is directed to a composition for diagnosing a disease comprising the nucleic acid complex having the structure of Structural Formula (1).

In still another aspect, the present invention is directed to a composition comprising the nucleic acid complex having the structure of Structural Formula (1) for use in preventing or treating a disease.

In yet another aspect, the present invention is directed to the nucleic acid complex having the structure of Structural Formula (1) for use in a method for preventing or treating disease, the method comprising administering the nucleic acid complex having the structure of Structural Formula (1) to a patient in need of prevention or treatment.

In yet another aspect, the present invention is directed to the nucleic acid complex having the structure of Structural Formula (1) for use in preventing or treating a disease.

In addition, the use of the nucleic acid complex having the structure of Structural Formula (1) in the manufacture of a medicament for preventing or treating a disease is described.

As used herein, the term "target gene" refers to a nucleic acid sequence (nucleotide sequence) to be activated, inhibited or labeled, and is not different from and is used interchangeably with the term "target nucleic acid".

If the target nucleic acid (nucleotide sequence) comprising the target gene contacts (binds) the complex *in vitro* or *in vivo,* then the bioactive nucleic acid is separated from the carrier peptide nucleic acid and exhibits biological activity.

In the present invention, the disease that can be diagnosed, prevented or treated using the nucleic acid complex having the structure of Structural Formula (1) may be determined depending on a target gene to which the bioactive nucleic acid in the nucleic acid complex binds. Preferably, the disease is cancer or a tumor, but is not limited thereto.

In the present invention, the term "composition for treatment" may be used interchangeably with "pharmaceutical composition", and the composition comprises, as an active ingredient, the nucleic acid complex of the present invention, which comprises a bioactive nucleic acid and a carrier bioactive nucleic acid bound to the bioactive nucleic acid.

The composition for treatment according to the present invention may be formulated in an oral or parenteral dosage form according to standard pharmaceutical practices. This formulation may contain additives such as a pharmacologically acceptable carrier, an excipient, a supplement, or a diluent besides the active ingredient.

The term "physiologically acceptable" refers to not abrogating the biological activity and properties of the compound.

The term "carrier" is defined as a compound which facilitates the addition of the complex into cells or tissue. For example, dimethylsulfoxide (DMSO) is a carrier which is commonly used to facilitate the penetration of a number of organic compounds into the cells or tissue of an organism.

The term "diluent" is defined as a compound that not only stabilizes the biologically active form of the target compound, but also a compound that is diluted in water in which it was dissolved. Salts dissolved in buffer solution are used as diluents in the related art. A commonly used buffer solution is phosphate buffered saline, which mimics the concentrations of salts in the human body. Since the buffer salts can control the pH of solution at low concentration, biological activity of compounds is rarely altered by buffer diluents.

The compounds containing the complex used herein can be administered to a human patient *per se*, or in pharmaceutical compositions where they are mixed with other active ingredients, as in combination therapy, or suitable carriers or excipients.

Pharmaceutical compositions suitable for use in the present invention include compositions where the active ingredients are contained in an amount effective to achieve intended purposes. More specifically, a therapeutically effective amount means an amount of a compound effective to prevent, stabilize, alleviate or ameliorate symptoms of a disease, or prolong the survival of the subject being treated. Determination of a therapeutically effective amount is well within the capability of those skilled in the art, especially in light of the detailed disclosure provided herein.

As used herein, the term "preventing" or "prevention" refers to all actions that exhibit anticancer activity and inhibit the growth of cancer or delay the development of cancer by administering (or applying) a pharmaceutical composition comprising the complex or a pharmaceutically acceptable salt thereof. As used herein, the term "treating" or "treatment" refers to all actions that alleviate or perfectly cure cancer by administering (or applying) a pharmaceutical composition comprising the complex or a pharmaceutically acceptable salt thereof.

In the present invention, diseases that can be prevented or treated by a composition comprising the nucleic acid complex of the present invention include, but are not limited to, tumors or cancers, inflammatory diseases, age-related macular degeneration, diabetic retinopathy, rare and severe diseases, cardiovascular diseases, metabolic diseases, and skin diseases.

In the present invention, diseases that can be treated by a composition comprising the nucleic acid complex of the present invention are determined by a target gene to which the bioactive nucleic acid contained in the nucleic acid complex binds. Examples of a target gene to which the bioactive nucleic acid binds, for cancer therapy, include VEGF, Androgen receptor, Clusterin, TGFβR2, ERBB3, ABCB1 and PD-L1. A target gene for inflammatory diseases is DE4B or Pellino-1, and a target gene for rare diseases and severe diseases is SMN2, ApoB-100, ICAM-1, ApoCIII, TTR, HTT, GHr, SOD1, ANGPTL3, PKK, miR-21, TMPRSS6, FMR1, or Connexin 26. A gene that targets cardiovascular diseases is Factor XI, Apo(a), ApoCIII, or AGT, and a target gene for metabolic diseases is GCGR, ANGPTL3, miR-103/107, or DGAT2. A target gene for skin diseases is IFI16, TLR6, or TIEG1, but examples of the genes are not limited thereto.

In the present invention, the composition for use in treatment may be formulated alone or together with a pharmaceutically acceptable carrier or excipient as described below, into a parenteral or oral dosage form by a known method. Specific examples of such a formulation include oral formulations such as an injectable formulation, a soft capsule formulation, a hard capsule formulation, a tablet formulation, and a syrup formulation, or external preparations.

Preferably, the composition for use in treatment comprising the nucleic acid complex may be prepared and used in a parenteral dosage form. Examples of suitable parenteral dosage forms include, but are not limited to, solution or freeze-dried formulations suitable for subcutaneous injection, intravenous injection, intramuscular injection or intrathoracic injection.

In the present invention, a composition comprising the nucleic acid complex may be administered transdermally (skin delivery). The formulation for transdermal delivery may be selected from among aqueous solutions, creams, and ointments, but is not limited thereto, and it is possible to use all types of formulations for skin delivery, which are known in the related art.

In order to formulate the composition for use in treatment in a parenteral dosage form, the composition comprises the nucleic acid complex of the present invention, and may further comprise one selected from among physiological saline, sterile water, Ringer's solution, buffered saline, dextrose solution, maltodextrin solution, glycerol, ethanol, and a mixture of two or more thereof. If necessary, the composition may contain other conventional additives such as an antioxidant, a buffer, and a bacteriostatic agent. In addition, a diluent, a dispersing agent, a surfactant, a binder and a lubricant may additionally be added to the composition to be prepared into an injectable formulation such as an aqueous solution, a suspension or an emulsion. Particularly, the composition is preferably provided as a lyophilized formulation. For preparation of a lyophilized formulation, a conventional method known in the technical field to which the present invention pertains may be used, and a stabilizer for lyophilization may also be added. Furthermore, the composition can preferably be formulated according to diseases or components by a suitable method known in the art or by a method disclosed in Remington's Pharmaceutical Science, Mack Publishing Company, Easton PA.

In addition, the composition for use in treatment according to the present invention may be formulated in oral dosage forms, including powder, tablet, capsule, liquid, injectable solution, ointment and syrup formulations. In this case, one or more pharmaceutically acceptable excipients may be added to the composition.

In the present invention, the composition comprising the nucleic acid complex may be a composition for use in preventing or treating cancer. Tumors and cancers that can be treated by the composition are not particularly limited, and include both solid cancers and blood cancers. Preferably, the cancers may be breast cancer, prostate cancer or lung cancer.

Preferably, a target gene, to which the bioactive nucleic acid contained in the nucleic acid complex for use in cancer treatment according to the present invention binds, may be, for example, any one or more selected from the group consisting of VEGF, PD-L1, Androgen receptor, Clusterin, TGFβR2, ERBB3, ABCB1, and PDE4B, without being limited thereto.

In the present invention, the composition for use in preventing or treating cancer comprising the nucleic acid complex having the structure of Structural Formula (1) comprises: a VEGF-specific bioactive peptide nucleic acid represented by any one of SEQ ID NO: 1 to SEQ ID NO: 3, or a PD-L1-specific bioactive peptide nucleic acid represented by SEQ ID NO: 10; and a carrier peptide nucleic acid complementary thereto. The carrier peptide nucleic acid may preferably have the sequence of any one of SEQ ID NO: 4 to SEQ ID NO: 9 (VEGF) or the sequence of SEQ ID NO: 11 (PD-L1), and a portion of the sequence may also be substituted with universal bases.

In the present invention, the composition for use in preventing or treating cancer comprising the nucleic acid complex having the structure of Structural Formula (1) comprises: an androgen receptor-specific bioactive peptide nucleic acid represented by any one of SEQ ID NO: 12 to SEQ ID NO: 15; and a carrier peptide nucleic acid complementary thereto. The carrier peptide nucleic acid may preferably have the sequence of any one of SEQ ID NO: 16 to SEQ ID NO: 31, and a portion of the sequence may also be substituted with universal bases.

In the present invention, the composition for use in preventing or treating cancer comprising the nucleic acid complex having the structure of Structural Formula (1) comprises: a clusterin-specific bioactive peptide nucleic acid represented by any one of SEQ ID NO: 32 to SEQ ID NO: 35; and a carrier peptide nucleic acid complementary thereto. The carrier peptide nucleic acid may preferably have the sequence of any one of SEQ ID NO: 36 to SEQ ID NO: 51, and a portion of the sequence may also be substituted with universal bases.

In the present invention, the composition comprising the nucleic acid complex having the structure of Structural Formula (1) may be a composition for use in preventing or treating age-related macular degeneration or diabetic retinopathy. A target gene, to which the bioactive nucleic acid contained in the nucleic acid complex for preventing or treating aged-related macular degeneration or diabetic retinopathy according to the present invention binds, may be, for example, VEGF, but is not limited thereto.

The composition for use in preventing or treating age-related macular degeneration or diabetic retinopathy comprises: a VEGF-specific bioactive peptide nucleic acid represented by any one of SEQ ID NO: 1 to SEQ ID NO: 3; and a carrier peptide nucleic acid complementary thereto. The carrier peptide nucleic acid may preferably have the sequence of any one of SEQ ID NO: 4 to SEQ ID NO: 9, and a portion of the sequence may also be substituted with universal bases.

The composition for use in preventing or treating age-related macular degeneration or diabetic retinopathy may have any one formulation selected from among aqueous solutions, injections, ophthalmic solutions, and eye drops.

In the present invention, the composition comprising the nucleic acid complex may be a composition for use in preventing or treating chronic obstructive pulmonary disease (COPD). A target gene, to which the bioactive nucleic acid contained in the nucleic acid complex binds, may be PDE4B, but is not limited thereto.

The composition for use in preventing or treating COPD comprises: a PDE4B-specfic bioactive peptide nucleic acid represented by any one of SEQ ID NOs: 52 to SEQ ID NO: 55; and a carrier peptide nucleic acid complementary thereto. The carrier peptide nucleic acid may preferably have the sequence of any one of SEQ ID NO: 56 to SEQ ID NO: 63, and a portion of the sequence may also be substituted with universal bases.

In the present invention, the composition comprising the nucleic acid complex may be a composition for use in preventing or treating skin disease. A target gene, to which the bioactive nucleic acid contained in the nucleic acid complex binds, may be, for example, any one or more selected from the group consisting of TLR2, Smad3, IFI16 and TIEG1, but is not limited thereto. Examples of the skin disease include, but are not limited to, psoriasis, pigmentation-related skin diseases, atopic dermatitis, skin damage, and keloids.

Preferably, the present invention provides a composition for use in treating atopic dermatitis. The composition for use in treating atopic dermatitis comprises: a TLR2-specific bioactive peptide nucleic acid represented by SEQ ID NO: 64 or SEQ ID NO: 65; and a carrier peptide nucleic acid complementary thereto. The carrier peptide nucleic acid may preferably have the sequence of any one of SEQ ID NO: 66 to SEQ ID NO: 71, and a portion of the sequence may also be substituted with universal bases.

The present invention also provides a composition for use in treating skin damage. The composition for use in treating skin damage is for skin regeneration including skin wound healing, but is not limited thereto. The composition for treating skin damage according to the present invention comprises: a Smad3-specific bioactive peptide nucleic acid represented by SEQ ID NO: 72 or SEQ ID NO: 73; and a carrier peptide nucleic acid complementary thereto. The carrier peptide nucleic acid may preferably have the sequence of any one of SEQ ID NOs: 74 to SEQ ID NO: 78, and a portion of the sequence may also be substituted with universal bases.

The present invention also provides a composition for use in treating keloids. The composition for use in treating keloids according to the present invention comprises: a TIEG1-specific bioactive peptide nucleic acid represented by SEQ ID NO: 79 or SEQ ID NO: 80; and a carrier peptide nucleic acid complementary thereto. The carrier peptide nucleic acid may preferably have the sequence of any one of SEQ ID NOs: 81 to SEQ ID NO: 84, and a portion of the sequence may also be substituted with universal bases.

The composition for use in preventing or treating skin disease may be prepared in the form of an aqueous solution, a gel, a paste, a lotion or an ointment, but is not limited thereto.

In the present invention, the composition comprising the nucleic acid complex may be a composition for use in preventing or treating inflammatory disease. A target gene, to which the bioactive nucleic acid contained in the nucleic acid complex binds, may be PDE4B or Pellino-1, but is not limited thereto.

In the present invention, the composition comprising the nucleic acid complex may be a composition for use in preventing or treating rare and severe disease. A target gene, to which the bioactive nucleic acid contained in the nucleic acid complex binds, may be, for example, SMN2, ApoB-100, ICAM-1, ApoCIII, TTR, HTT, GHr, SOD1, ANGPTL3, PKK, miR-21, TMPRSS6 or Connexin 26, but is not limited thereto.

Preferably, the rare and severe disease according to the present invention may be hearing loss, and a target gene, to which the bioactive nucleic acid contained in the nucleic acid complex binds, may be Connexin 26.

In the present invention, the complex may be administered (or applied) via a carrier such as a liposome. The liposome may aid in targeting the complex toward a specific tissue, such as lymphoid tissue, or specifically targeting the complex toward infected cells, and may also help to increase the half-life of the composition comprising the complex. Examples of the liposome include emulsions, foams, micelles, insoluble monolayers, liquid crystals, phospholipid dispersions, lamellar layers, and the like. In these preparations, the complex to be delivered is incorporated as part of a liposome, alone or in conjunction with a molecule which binds to, e.g., a receptor prevalent among lymphoid cells, such as monoclonal antibodies which bind to the CD45 antigen, or with other therapeutic compositions. Thus, liposomes either filled or decorated with a desired complex of the present invention can be directed to the site of lymphoid cells.

Liposomes for use in the present invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes. For example, methods as disclosed in literature [Szoka, et al., Ann. Rev. Biophys. Bioeng., 9:467, 1980, and U.S. Pat. Nos. 4,235,871, 4,501,728, 4,837,028, and 5,019,369] can be used.

In the following a method of treating and suppressing (or alleviating) disease by administering (or applying) the complex or the composition comprising the complex to a subject is described.

A disease that can be treated using the complex of the present invention is determined according to the characteristics of the bioactive nucleic acid used, and is not particularly limited.

Examples of diseases that can be treated using the complex of the present invention include, but are not limited to, cancer, abnormal blood vessel growth-related disease such as macular degeneration, skin diseases, inflammatory diseases and autoimmune diseases.

A composition comprising the complex according to the present invention may be administered (or applied) in a pharmaceutically effective amount in order to treat cancer or suppress (or alleviate) cancer symptoms. The dose/application amount of the pharmaceutical composition of the present invention may vary depending on various factors such as the kind of pigmentation-related skin diseases, the patient's age and body weight, the characteristics and degree of symptoms, the kind of current treatment method, the frequency of treatment, the mode and route of administration (application), and may be easily determined by those of ordinary skill in the related art. The composition of the present invention may be administered (applied) together with the pharmacological or physiological ingredient, or sequentially administered (applied). In addition, the composition of the present invention may also be administered (applied) in combination with conventional additional therapeutic agents, and sequentially or simultaneously with the conventional therapeutic agent. The administration (application) may be single dose administration (application) or multi-dose administration (application).

In the present invention, the term "subject" refers to a mammal suffering from a condition or disease which can be alleviated, suppressed or treated by administering (applying) the complex of the present invention, or being at risk of developing this condition or disease. Preferably, it refers to a human being.

In addition, the dose (application amount) of the compound of the present invention to the human body may vary depending on the patient's age, body weight and gender, the mode of administration (application), the patient's health condition, and the severity of the disease. Based on an adult patient weighing 70 kg, the dose is generally 0.001 to 1,000 mg/day, preferably 0.01 to 500 mg/day. Depending on the judgment of a doctor or a pharmacist, the dose may be administered (applied) once or several times a day at predetermined time intervals.

For any compound or a mixture comprising the same used in the methods , the therapeutically effective amount can be estimated initially from cell culture assays. For example, a dose can be formulated in animal models to achieve a circulating plasma concentration range that includes the $IC_{50}$ (half maximal inhibitory concentration) or the $EC_{50}$ (half maximal effective concentration) as determined in cell culture. Such information can be used to more accurately determine useful doses in humans.

Toxicity and therapeutic efficacy of the complex described herein or a mixture comprising the same can be determined by standard pharmaceutical procedures in cell cultures or experimental animals, e.g., by determining the $LD_{50}$ (the dose lethal to 50% of the population) and the $ED_{50}$ (the dose therapeutically effective in 50% of the population) . The dose ratio between therapeutic and toxic effects is the therapeutic index and it can be expressed as the ratio $ED_{50}$ (or $IC_{50}$)/$LD_{50}$. Compounds that exhibit large therapeutic indices are preferred. The data obtained from these cell culture assays can be used in formulating a range of doses for use in humans. The dosages of these compounds lay preferably within a range of circulating concentrations that include the $ED_{50}$ (or $IC_{50}$) with little or no toxicity.

In the following a kit for diagnosing cancer or tumor is described, wherein the kit comprises the complex.

In the present invention, a sample for diagnosing cancer or tumor may be derived from specific tissues or organs of mammals, including humans. Representative examples of the tissues include connective tissue, muscle, or nerve tissue. Representative examples of the organs include eyes, brain, lung, liver, spleen, bone marrow, thymus, heart, lymph, blood, bone, cartilage, pancreas, kidney, gallbladder, stomach, small intestine, testes, ovaries, uterus, rectum, nervous system, gland, and internal blood vessels.

The sample includes any cell, tissue or fluid that is derived from a biological origin, or any other medium that can be well analyzed by the present invention. The sample also includes a sample obtained from foods produced for consumption of humans and/or animals. In addition, the sample to be analyzed includes a body fluid sample, which includes, but not limited to, blood, serum, plasma, lymph, breast milk, urine, feces, ocular fluid, saliva, semen, brain extracts (e.g., ground brain), spinal fluid, and appendix, spleen, and tonsil tissue extracts.

The kit may optionally include reagents required for performing a target nucleic acid amplification reaction (e.g., PCR reaction), such as buffer, DNA polymerase cofactor, and deoxyribonucleotide-5-triphosphate. Alternatively, the kit may also include an antibody that inhibits the activities of various polynucleotide molecules, a reverse transcriptase, buffers and reagents, and a DNA polymerase. In addition, in the kit, the optimal amount of the reagent used in a specific reaction can be easily determined by those skilled in the art who have acquired the disclosure set forth herein. Typically, the kit may be manufactured as a separate package or compartment containing the above-mentioned ingredients.

In the present invention, the carrier peptide nucleic acid may form a complementary hydrogen bond with the bioactive nucleic acid and deliver the bioactive nucleic acid into cells, and the bioactive nucleic acid may bind to a target gene and regulate expression of the target gene.

In addition, a method of regulating target gene expression using the nucleic acid complex having the structure of Structural Formula (1) is described.

The method of regulating target gene expression may comprise steps of: (a) forming a complex by binding between a bioactive nucleic acid, which comprises a material for facilitating endosomal escape, and a carrier peptide nucleic acid; and (b) introducing the complex into target cells by bringing the complex into contact with the target cells.

The target cells may be the above-described cancer or tumor cells, without being limited thereto. After the complex is introduced into the cells in step (b) and moves, the bioactive nucleic acid may bind to a target nucleic acid having a nucleotide sequence complementary thereto and may be separated from the carrier peptide nucleic acid, and the bioactive nucleic acid may bind to the target gene and thereby regulate expression of the target gene.

In the absence of a target nucleic acid (target sequence), the bioactive nucleic acid and the carrier peptide nucleic acid of the complex maintain complementary binding therebetween, whereas, in the presence of a target nucleic acid complementary to the nucleotide sequence of the bioactive nucleic acid, the bioactive nucleic acid is separated from the carrier peptide nucleic acid and binds to the target nucleic acid by "strand displacement of the bioactive nucleic acid to the target sequence" and "target-specific release and binding". The time of release and binding can be controlled by controlling the hydrogen bonding strength between nucleobases of the bioactive nucleic acid according to the complementarity between the nucleotide sequence of the carrier peptide nucleic acid and the nucleotide sequence of the target sequence.

Therefore, the target-specific release and binding may be achieved by: i) constructing a "single nucleotide polymorphism (SNP)" or a "sequence shorter than the bioactive nucleic acid" as a carrier peptide nucleic acid structure having a partial specific sequence; or ii) replacing a portion of the carrier peptide nucleic acid sequence with universal bases; or iii) replacing a portion of the carrier peptide nucleic acid sequence with a linker; or iv) providing a carrier peptide nucleic acid structure which binds parallel to the bioactive nucleic acid such that the binding affinity between the carrier peptide nucleic acid and the bioactive nucleic acid is lower than the binding affinity between the target nucleic acid and the bioactive nucleic acid. These methods may be used in combination of two or more, and the parallel binding method is preferably used.

The universal base used in the present invention may be one or more selected from the group consisting of inosine PNA, indole PNA, nitroindole PNA, and abasic PNA, which are bases that bind to natural bases, including adenine, guanine, cytosine, thymine, and uracil, without selectivity, and have lower binding affinity than complementary binding affinity. Preferably, inosine PNA may be used as the universal base.

There is an advantage in that the time of separation between the bioactive nucleic acid and the carrier peptide nucleic acid and the time of binding between the bioactive nucleic acid and the gene targeted by the bioactive nucleic acid can be controlled by controlling the binding affinity between the bioactive nucleic acid and the carrier peptide nucleic acid.

In the present invention, the efficacy of the nucleic acid complex having the structure of Structural Formula (1) was verified using a nucleic acid complex having a structure of the structural formula **[ A ≡ C (+) ]**, which does not comprise the material for facilitating endosomal escape (see PCT/KR2017/008636) .

The carrier peptide nucleic acid (i.e., modified carrier peptide nucleic acid) according to the present invention overcomes a precipitation problem caused by the self-aggregation property of a conventional non-modified naked-PNA, and may increase cell permeability, solubility and intracellular diffusion effects.

Hereinafter, the present invention will be described in more detail with reference to examples. It will be obvious to those skilled in the art that these examples are merely to illustrate the present invention.

### Example 1: Examination of Anticancer Pharmacological Effect by Inhibition of Vascular Endothelial Growth Factor in Human Breast Cancer Cells and Lung Cancer Cells Using Novel Complex Having Structure of Structural Formula (1)

It is known that vascular endothelial growth factor (VEGF) found to be highly expressed in many types of cancer cells induces cellular and vascular proliferation in cancer cells. Thus, the novel complex was examined to determine whether it could exhibit an anticancer pharmacological effect by inhibiting VEGF.

Vascular endothelial growth factor (VEGF) is involved in the development and maintenance of homeostasis of vascular and lymphatic vessels, and also has an important effect on neurons. Such VEGF is known to be an important mediator of disease-related angiogenesis in tumors and eyes. In addition, VEGF mRNA is overexpressed by tumors in the majority of subjects investigated (Berkman et al., J Clin Invest., 91:153-159 (1993)). Cancer requires new capillaries as a passage for nutrient supply and waste discharge for its growth, and to this end, cancer cells and cancerous stromal cells continuously secrete VEGF, and the secreted VEGF diffuses through tissue and stimulates the migration of vascular endothelial cells (Ferrara.N. et al., Nat Rev Cancer, 2:795-803, (2002)). The neovasculatures induced by cancer cells are characterized by being incomplete as compared to normally

formed capillaries because they are unaided by surrounding cells. Although VEGF binds to receptors VEGFR1, 2 and 3, it is through VEGFR2 that VEGF delivers a signal leading to proliferation, migration and permeability of endothelial cells (Zeng H. et al., J Biol. Chem. , 276:26969-26976 (2001)). Therefore, by controlling angiogenesis using drugs targeting VEGF, the proliferation of cancer cells and diseases associated with angiogenesis can be treated.

Example 1-1. Cell Culture

Human breast cancer cells (MDA-MB-231), human lung cancer cells (A549) and human uterine cancer cells (HeLa), obtained from the ATCC (American Type Culture Collection, USA), were cultured in DMEM culture medium (Dulbecco Modified Eagle Medium, Welgene, Korea) containing 10% (v/v) fetal bovine serum, 100 units/ml penicillin and 100 μg/ml streptomycin at 37°C under 5% (v/v) $CO_2$.

Example 1-2. Analysis of Changes in Intracellular Introduction, Cell Permeability and Endosomal Escape of Nucleic Acid Complex

Novel complexes for inhibiting vascular endothelial growth factor were produced by preparing bioactive peptide nucleic acids having sequences (SEQ ID NO: 1 to SEQ ID NO: 3) complementary to VEGF mRNA, an essential gene for vascular

growth, preparing carrier peptide nucleic acids (SEQ ID NO: 4 to SEQ ID NO: 9) complementary to the bioactive peptide nucleic acids comprising a material for facilitating endosomal escape, and then hybridizing each bioactive peptide nucleic acid with each carrier peptide nucleic acid in equal amounts (see Table 2). A method for treating the complex in which the bioactive peptide nucleic acid and the carrier peptide nucleic acid are bound to each other is as described in PCT/KR2017/008636.

[Table 2]

| Sequences of bioactive nucleic acids and carrier peptide nucleic acids for inhibiting VEGF activity | | | |
|---|---|---|---|
| Component | SEQ ID NO | Nucleotide sequence | Monomer modification |
| Bioactive nucleic acid | SEQ ID NO: 1 | 5'-AT$^{(-)}$GA$^{(+)}$TTC$^{(-)}$TG$^{(+)}$CCC$^{(-)}$TCC-O-K-3' | -+-+- |
| | SEQ ID NO: 2 | 5'-GLFDIIKKIAESF-O-AT$^{(-)}$GA$^{(+)}$TTC$^{(-)}$TG$^{(+)}$CCC$^{(-)}$TCC-O-K-3' | -+-+- |
| | SEQ ID NO: 3 | 5'-Histidine(10)-O-AT$^{(-)}$GA$^{(+)}$TTC$^{(-)}$TG$^{(+)}$CCC$^{(-)}$TCC-O-K-3' | -+-+- |
| Carrier peptide nucleic acid | SEQ ID NO: 4 | 5'-K-O-GG$^{(+)}$AG$^{(+)}$G-3' | ++ |
| | SEQ ID NO: 5 | 5'-K-O-GG$^{(+)}$AG$^{(+)}$G-O-GLFDIIKKIAESF-3' | ++ |
| | SEQ ID NO: 6 | 5'-K-O-GG$^{(+)}$AG$^{(+)}$G-O-Histidine(10)-3' | ++ |
| | SEQ ID NO: 7 | 5'-GLFDIIKKIAESF-O-TAC$^{(+)}$TAAGA$^{(+)}$CGG$^{(+)}$GAGG-O-K-3' | +++ |
| | SEQ ID NO: 8 | 5'-Histidine(10)-O-TAC$^{(+)}$TAAGA$^{(+)}$CGG$^{(+)}$GAGG-O-K-3' | +++ |
| | SEQ ID NO: 9 | 5'-TAC$^{(+)}$TAAGA$^{(+)}$CGG$^{(+)}$GAGG-O-K-3' | +++ |

The cells cultured in Example 1-1 were seeded into an 8-well plate at a density of $5\times10^3$ cells/well, treated with the complex, and then cultured for 24, 48, 72 and 96 hours. After each culture time, changes in cell permeability and endosomal escape of the nucleic acid complex were analyzed using acridine orange staining.

The nucleic acid complexes used in this Example are shown in Table 3 below.

[Table 3]

| Nucleic acid complexes for analysis of endosomal escape | |
|---|---|
| Name | Nucleic acid complex |
| Original PNA 1 | SEQ ID NO: 1 and SEQ ID NO: 4 |
| Original PNA 2 | SEQ ID NO: 1 and SEQ ID NO: 9 |
| Modified PNA 1 | SEQ ID NO: 2 and SEQ ID NO: 6 |
| Modified PNA 2 | SEQ ID NO: 2 and SEQ ID NO: 8 |

As shown in FIG. 1a, it was confirmed that the nucleic acid complex containing the endosomal escape moiety was released into the cytoplasm faster than the nucleic acid complex not containing the endosomal escape moiety in the human uterine cancer cells.

Example 1-3. Analysis of Cell Viability in Human Breast Cancer Cells and Lung Cancer Cells

To analyze the degree to which the novel complex inhibits vascular endothelial growth factor, the cells cultured in Example 1-1 were seeded into a 96-well plate at a density of $6\times10^3$ cells/well, treated with the complex, and then cultured for 24, 48, 72 and 96 hours. After each culture time, cell viability was analyzed under the experimental conditions disclosed in PCT/KR2017/008636.

The nucleic acid complexes used in this Example are shown in Table 4 below.

[Table 4]

| Nucleic acid complexes for analysis of cell viability in breast cancer cells and lung cancer cells | |
|---|---|
| | Nucleic acid complex |
| Original | SEQ ID NO: 1 and SEQ ID NO: 4 |
| PNA 1 | SEQ ID NO: 2 and SEQ ID NO: 4 |
| PNA 2 | SEQ ID NO: 3 and SEQ ID NO: 4 |
| PNA 3 | SEQ ID NO: 2 and SEQ ID NO: 5 |
| PNA 4 | SEQ ID NO: 2 and SEQ ID NO: 6 |
| PNA 5 | SEQ ID NO: 2 and SEQ ID NO: 7 |
| PNA 6 | SEQ ID NO: 2 and SEQ ID NO: 8 |

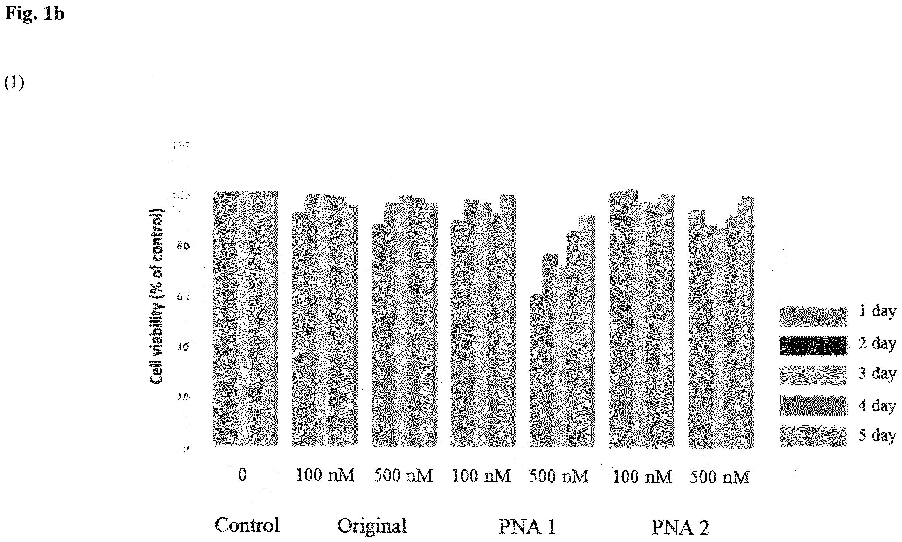

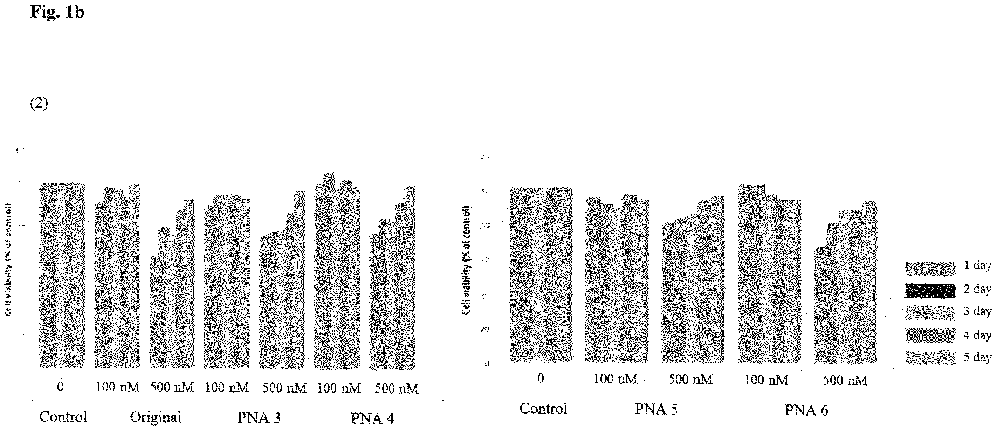

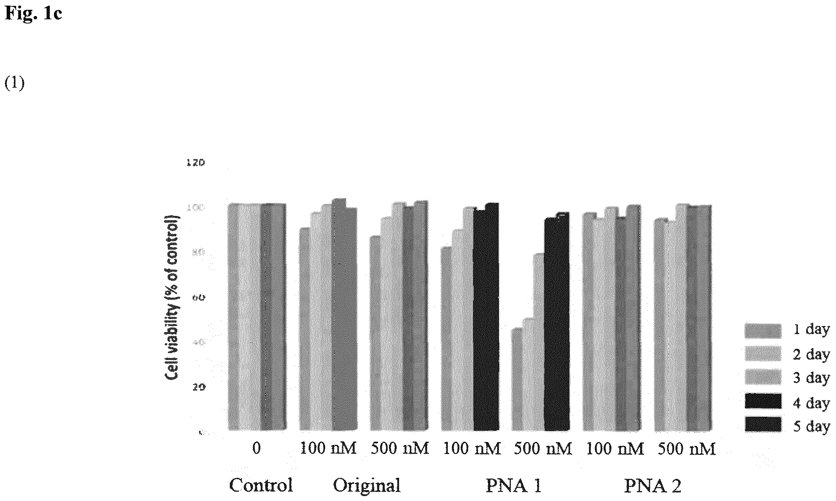

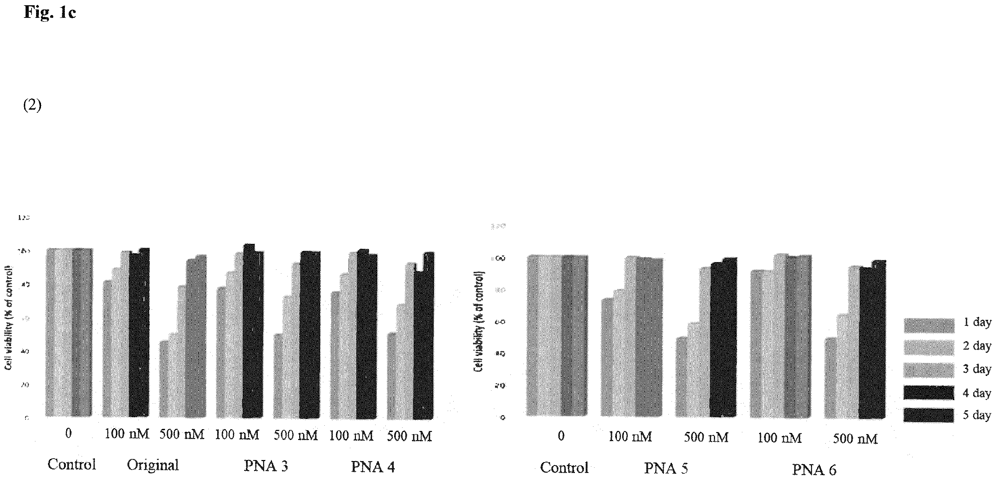

As a result, as shown in FIGS. 1b and 1c, it was confirmed in the human breast cancer cells and lung cancer cells that the novel complex inhibited cell viability of the cells by inhibiting vascular endothelial growth factor.

Example 1-4. Analysis of Gene Expression by Western Blot Assay

To analyze the degree to which the novel complex inhibits vascular endothelial growth factor, the cells cultured in Example 1-1 were seeded into a 6-well plate at a density of $1\times10^5$ cells/well, treated with the complex, and then cultured for 24, 48, 72 and 96 hours. After each culture time, protein expression was analyzed using anti-VEGF antibody (SantaCruz Biotech., USA) and anti-p-Akt1 (Cell Signaling, USA) under the conditions disclosed in PCT/KR2017/008636.

The nucleic acid complexes used in this Example are shown in Table 5 below.

[Table 5]

| Nucleic acid complexes for analysis of expression of VEGF and p-Akt1 genes | |
|---|---|
| Name | Nucleic acid complex |
| PNA 1 | SEQ ID NO: 1 and SEQ ID NO: 4 |
| PNA 2 | SEQ ID NO: 1 and SEQ ID NO: 9 |
| PNA 3 | SEQ ID NO: 2 and SEQ ID NO: 6 |

(continued)

| Nucleic acid complexes for analysis of expression of VEGF and p-Akt1 genes | |
|---|---|
| Name | Nucleic acid complex |
| PNA 4 | SEQ ID NO: 2 and SEQ ID NO: 8 |

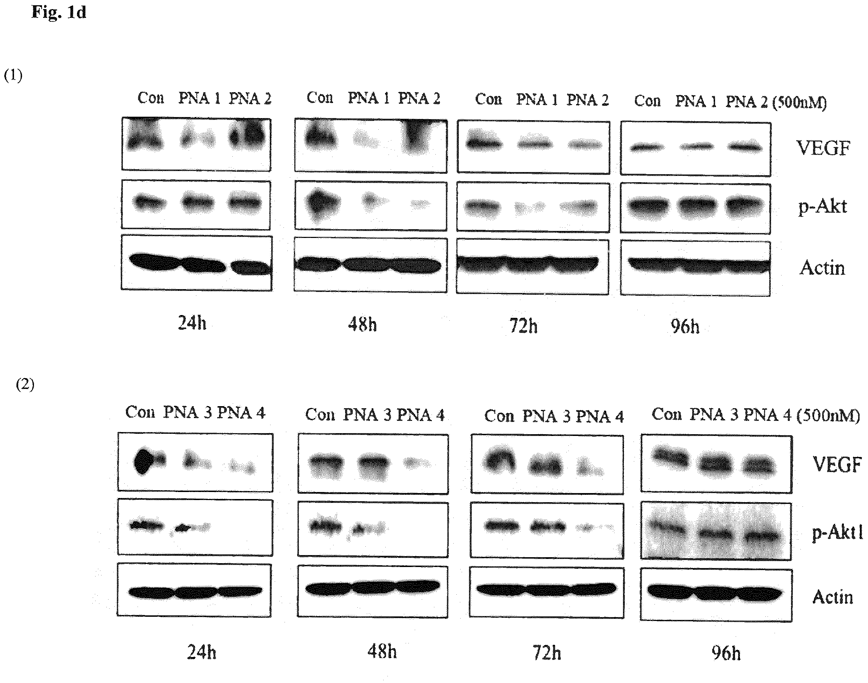

As shown in FIG. 1d, it was confirmed that protein expression of the vascular endothelial growth factor (VEGF) and its downstream gene p-Akt1 was inhibited.

Example 1-5. Analysis of Apoptosis by Flow Cytometry (fluorescence activated cell sorter; FACS)

To analyze apoptosis induced by inhibition of vascular endothelial growth factor using the novel complex, the cells cultured in Example 1-1 were seeded into a 6-well plate at a density of $1\times10^5$ cells per well, treated with the complex, cultured for 72 hours, and then harvested. Next, the cells were suspended well in 500 μL of Annexin V binding buffer of an FITC Annexin V Apoptosis Detection Kit (BD, USA) and treated with 5 μL of each of FITC Annexin V and propidium iodide staining solution. Then, the cells were transferred into a tube for flow cytometry, and then analyzed by FACS CantoII (BD, USA).

The nucleic acid complexes used in this Example are shown in Table 6 below.

[Table 6]

| Nucleic acid complexes for analysis of apoptosis induced by inhibition of vascular endothelial growth factor | |
|---|---|
| Name | Nucleic acid complex |
| Original PNA duplex | SEQ ID NO: 1 and SEQ ID NO: 4 |
| Modified PNA duplex | SEQ ID NO: 2 and SEQ ID NO: 6 |
| Modified PNA duplex 1 | SEQ ID NO: 2 and SEQ ID NO: 6 |
| Modified PNA duplex 2 | SEQ ID NO: 2 and SEQ ID NO: 8 |

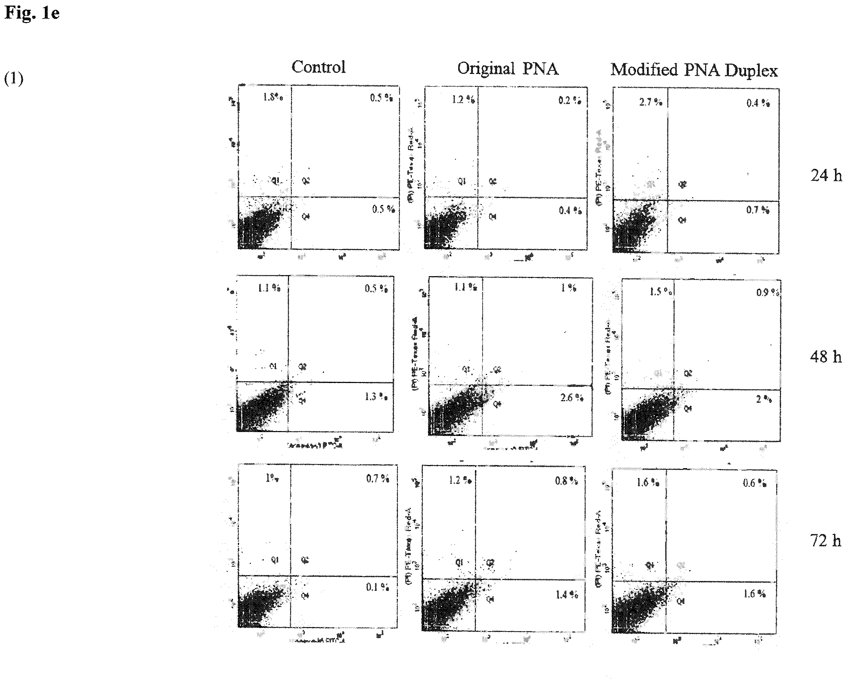

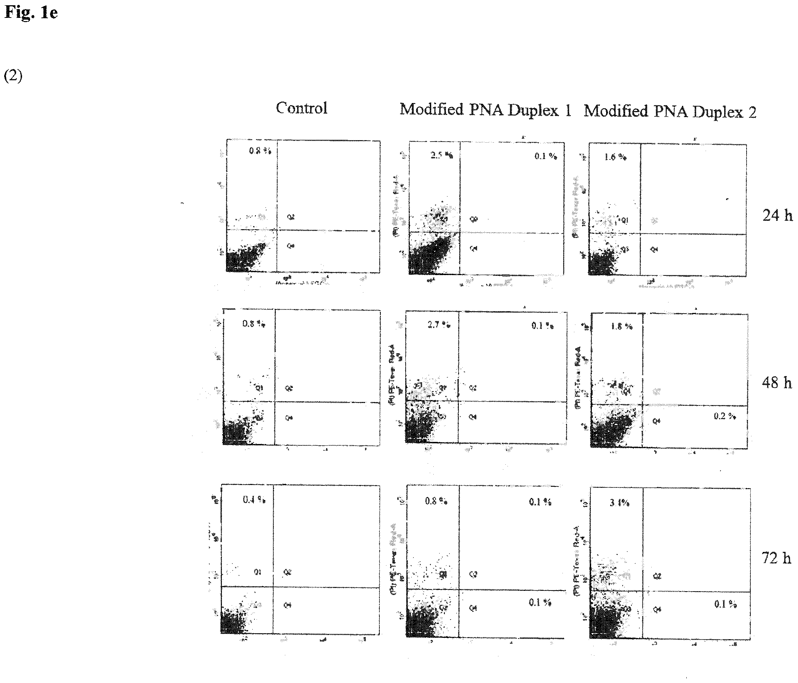

As a result, it was confirmed that analysis of the complexes, comprising the bioactive peptide nucleic acid and the carrier peptide nucleic acid bound thereto and having different charges and lengths, indicated that apoptosis of 0.6 to 3.1% was induced (FIG. 1e).

**Example 2: Evaluation of Anticancer Efficacy of Candidate Complex Comprising Vascular Endothelial Growth Factor-Targeting Bioactive Peptide Nucleic Acid and Carrier Peptide Nucleic Acid**

Candidate complexes, each comprising a VEGF-targeting bioactive peptide nucleic acid and a carrier peptide nucleic acid as shown in Table 7 below, were used to analyze whether each complex would inhibit tumors by inhibiting expression of the target gene VEGF in animal models transplanted with human breast cancer tumor cell lines and tumor cells. In addition, analysis was performed to examine whether cancer cell growth

would be inhibited by treatment with a combination of a novel complex comprising a bioactive peptide nucleic acid, which targets PD-L1 that is expressed on the surface of cancer cells and continuously induces proliferation of the cancer cells, and a carrier peptide nucleic acid, and a candidate complex that targets VEGF.

[Table 7]

| Sequences for analyzing tumor inhibition induced by inhibition of target gene expression in animal model | | | |
|---|---|---|---|
| Name | Component | SEQ ID NO | Nucleotide sequence |
| Sample 1 | Bioactive nucleic acid | SEQ ID NO: 2 | 5'-GLFDIIKKIAESF-O-<br>AT$^{(-)}$GA$^{(+)}$TTC$^{(-)}$TG$^{(+)}$CCC$^{(-)}$TCC-O-K-3' |
| | Carrier peptide nucleic acid | SEQ ID NO: 5 | 5'-K-O-GG$^{(+)}$AG$^{(+)}$G-O-GLFDIIKKIAESF-3' |

(continued)

| Sequences for analyzing tumor inhibition induced by inhibition of target gene expression in animal model | | | |
|---|---|---|---|
| Name | Component | SEQ ID NO | Nucleotide sequence |
| Sample 2 | Bioactive nucleic acid | SEQ ID NO: 2 | 5'-GLFDIIKKIAESF-O-AT$^{(-)}$GA$^{(+)}$TTC$^{(-)}$TG$^{(+)}$CCC$^{(-)}$TCC-O-K-3' |
| | Carrier peptide nucleic acid | SEQ ID NO: 8 | 5'-Histidine(10)-O-TAC$^{(+)}$TAAGA$^{(+)}$CGG$^{(+)}$GAGG-O-K-3' |
| Sample 3 | Bioactive nucleic acid | SEQ ID NO: 2 | 5'-GLFDIIKKIAESF-O-AT$^{(-)}$GA$^{(+)}$TTC$^{(-)}$TG$^{(+)}$CCC$^{(-)}$TCC-O-K-3' |
| | Carrier peptide nucleic acid | SEQ ID NO: 5 | 5'-K-O-GG$^{(+)}$AG$^{(+)}$G-3' |
| | Bioactive nucleic acid | SEQ ID NO: 10 | 5'-GLFDIIKKIAESF-O-AT$^{(-)}$GA$^{(+)}$AAGC$^{(-)}$AA$^{(+)}$TGA$^{(-)}$T-O-K-3' |
| | Carrier peptide nucleic acid | SEQ ID NO: 11 | 5'-K-O-AT$^{(+)}$CA$^{(+)}$T-GLFDIIKKIAESF-3' |
| Sample 4 | Bioactive nucleic acid | SEQ ID NO: 2 | 5'-GLFDIIKKIAESF-O-AT$^{(-)}$GA$^{(+)}$TTC$^{(-)}$TG$^{(+)}$CCC$^{(-)}$TCC-O-K-3' |
| | Carrier peptide nucleic acid | SEQ ID NO: 8 | 5'-Histidine(10)-O-TAC$^{(+)}$TAAGA$^{(+)}$CGG$^{(+)}$GAGG-O-K-3' |
| | Bioactive nucleic acid | SEQ ID NO: 10 | 5'-GLFDIIKKIAESF-O-AT$^{(-)}$GA$^{(+)}$AAGC$^{(-)}$AA$^{(+)}$TGA$^{(-)}$T-O-K-3' |
| | Carrier peptide nucleic acid | SEQ ID NO: 11 | 5'-K-O-AT$^{(+)}$CA$^{(+)}$T-GLFDIIKKIAESF-3' |

Example 2-1. Evaluation of Anticancer Efficacy by Tail Vein Administration of Candidate Complex Comprising Bioactive Peptide Nucleic Acid and Carrier Peptide Nucleic Acid in Mice Transplanted with Human Breast Cancer Cells (MDA-MB-231)

For evaluation of anticancer efficacy, human breast cancer cell lines (MDA-MB-231) were cultured and adjusted to a cell concentration of $3 \times 10^7$ cells/ml. 0.3 ml (9x106 cells/mouse) of the cell culture was injected subcutaneously into the axillary region between the right scapular portion and chest wall of each of specific pathogen-free (SPF) BALB/C female nude mice (Nara Biotech Co., Korea). Each of candidate complex samples 1, 2, 3 and 4 (1 and 2 mg/kg), each comprising the bioactive peptide nucleic acid and the carrier peptide nucleic acid, and a negative control (1 and 2 mg/kg), was

administered into the tail vein of each mouse in an amount of 0.1 ml, twice a week (days 0, 3, 7, 10, 14 and 17). For all the animals, general conditions and body weight were measured at the start of injection and immediately before administration during the test period. After cancer cell transplantation, three directions (length x width x height) of each tumor were measured using Vernier calipers for each animal, a total of 10 times up to 21 days from a time point when the average tumor volume of each group reached 37.7 $mm^3$, and then the tumor volume was calculated using the equation "length x width x height/2". 21 days after the start of drug administration, blood was collected from the orbital vein by a heparin tube and centrifuged at 5000 rpm for 5 minutes, the supernatant plasma was isolated and dispensed in vials and stored at -70°C. Next, the mice were euthanized with $CO_2$ gas, and then the tumor was isolated and weighed in a chemical balance, and the tumor tissue was imaged.

Example 2-2: Analysis of Hepatotoxicity Marker in Blood from Mice Transplanted with Human Breast Cancer Cells (MDA-MB-231)

Plasma was separated from the blood collected from the mice used in anticancer efficacy evaluation, and was diluted at a suitable ratio in the assay solution of an alanine aminotransferase (ALT) and aspartate aminotransferase (AST) assay kit (Biovision, USA), and 20 μL of the dilution was added to each well. In addition, standard materials that help quantify the amounts of alanine aminotransferase (ALT) and aspartate aminotransferase (AST) in serum were also prepared and added to each well. Next, a reaction solution (an enzyme/dye agent mixture including the assay solution) was prepared according to the method provided in the assay kit, and 100 μL of the reaction solution was added to each well and shaken well. Next, the OD at 570 nm was measured by a spectrophotometer.

Example 2-3. Analysis of Expression of VEGF and Downstream Gene Proteins in Tumor Tissue from Mice Transplanted with Human Breast Cancer Cells (MDA-MB-231)

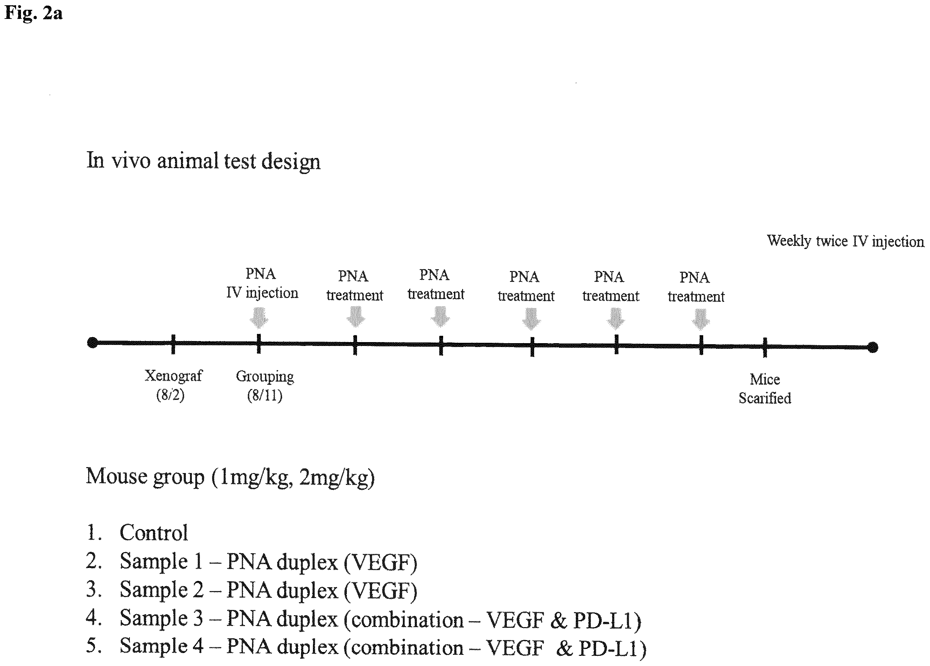

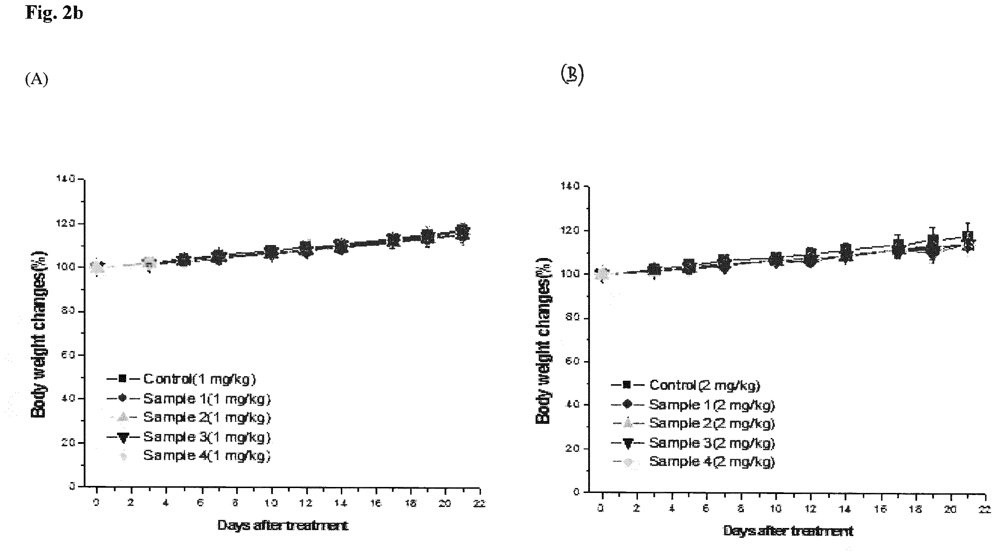

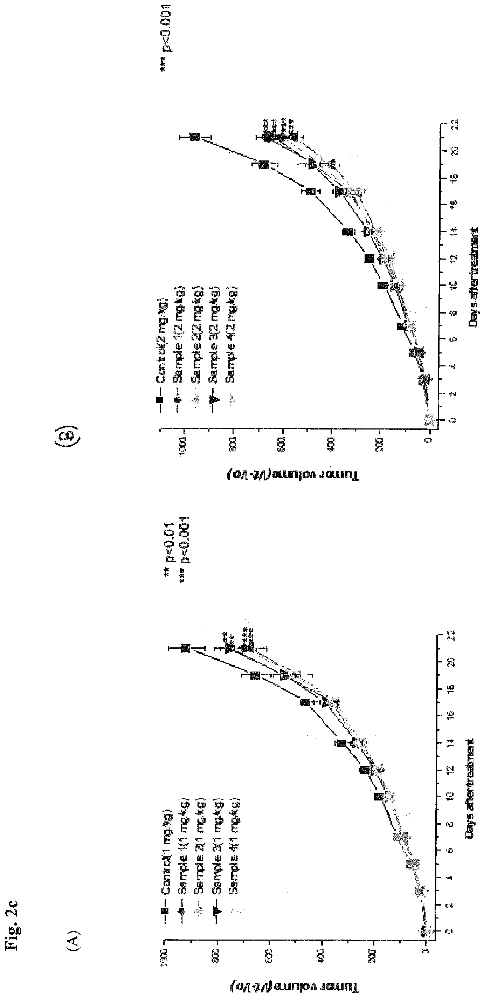

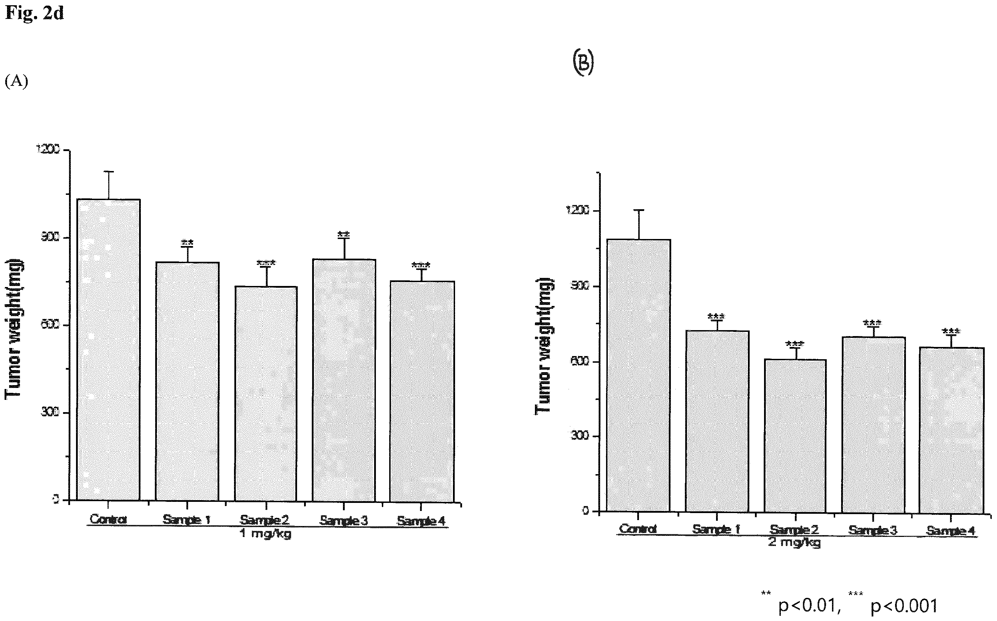

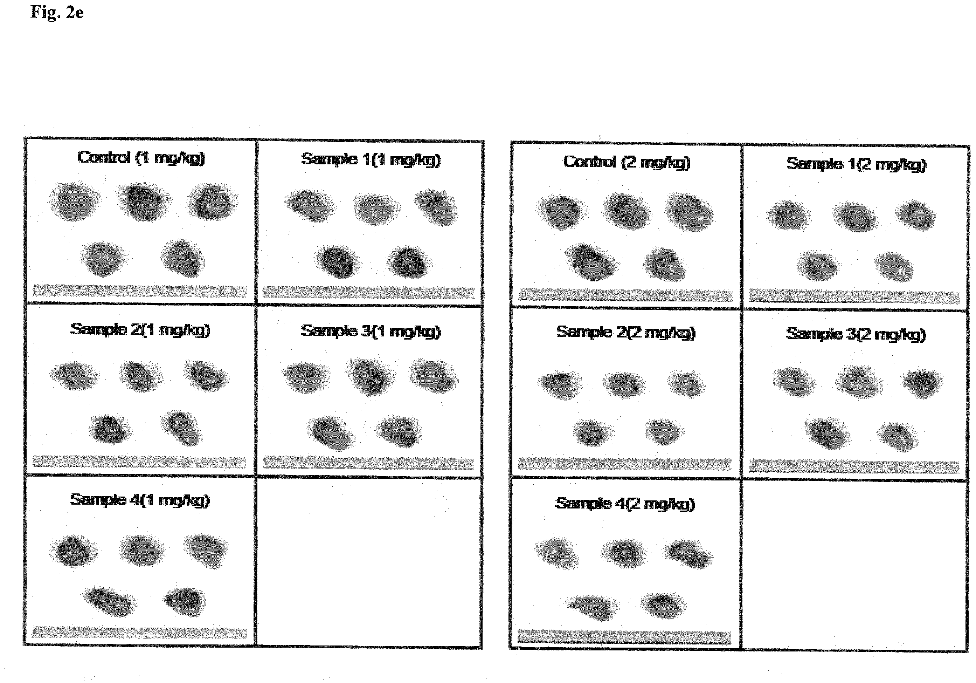

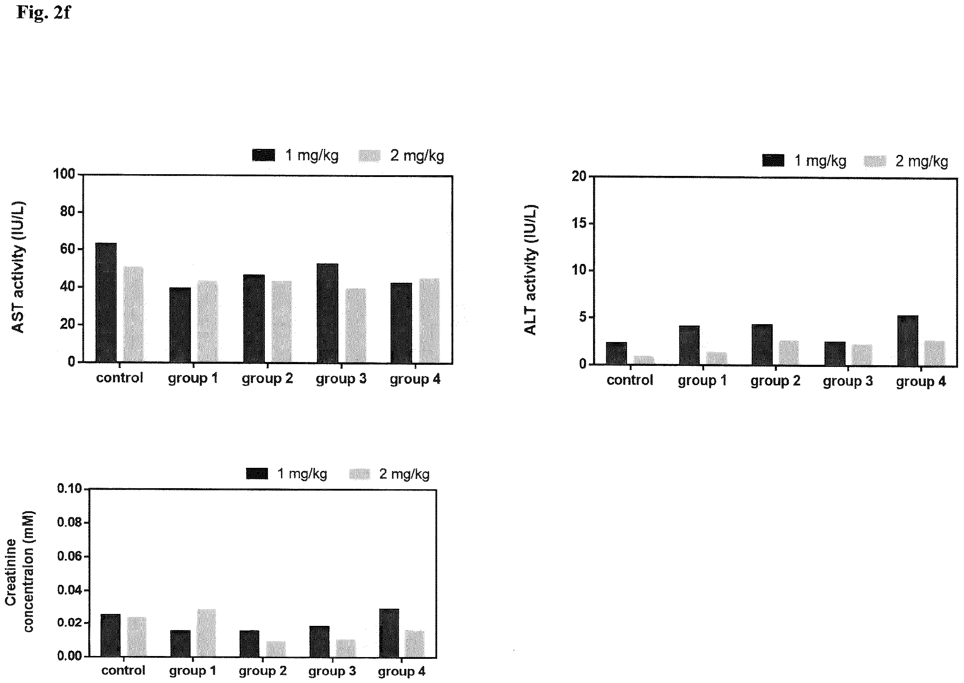

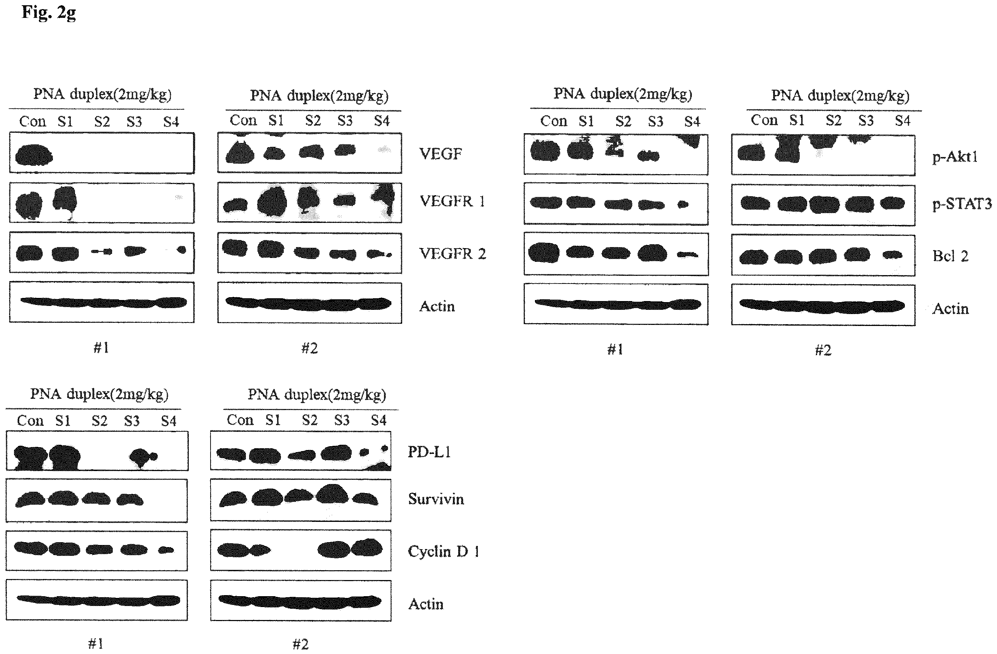

Using tumor tissues of the mice used in anticancer efficacy evaluation, two tissues of each random group were homogenized in 100 μl of RIPA buffer, and protein was isolated therefrom, and then protein expression was analyzed using anti-VEGF antibody (SantaCruz Biotech., USA), anti-VEGFR1 antibody (Cell Signaling, USA), anti-VEGFR2 antibody (Cell Signaling, USA), anti-p-Akt1 antibody (Cell Signaling, USA), anti-p-Akt1 antibody (Cell Signaling, USA), anti-Bcl2 antibody (SantaCruz Biotech., USA), anti-Survivin antibody (Cell signaling, USA), and anti-CyclinD 1 antibody (SantaCruz Biotech., USA), under the conditions disclosed in PCT/KR2017/008636. In the animal experiment performed by administration into the tail vein of the mice transplanted with human breast cancer cells, unusual general conditions and a statistically significant loss in body weight were not observed in all the sample-administered groups, unlike the negative control group, during the test period from the day after administration to the last day (FIG. 2b). In addition, in the case of the tumor volume on the last day (day 21), the groups administered 1 mg/kg of samples 1, 2, 3 and 4 showed tumor growth inhibition rates of 18.4% ($p<0.01$), 26.0% ($p<0.001$), 17.5% ($p<0.01$) and 23.9% ($p<0.001$), respectively, compared to the group administered 1 mg/kg of the negative control, and the groups administered 2 mg/kg of samples 1, 2, 3 and 4 showed tumor growth inhibition rates of 30.1% ($p<0.001$), 41.4% ($p<0.001$), 32.6% ($p<0.001$) and 36.4% ($p<0.001$), respectively, compared to the group administered 2 mg/kg of the negative control. In the case of the tumor weight on the last day, the weight of the MDA-MB-231 tumor isolated on 21 days after the start of drug administration was measured, and as a result, it could be seen that the groups administered 1 mg/kg of samples 1, 2, 3 and 4 showed tumor weight reductions of 20.3% ($p<0.01$), 28.6% ($p<0.001$), 19.0% ($p<0.01$) and 26.4% ($p<0.001$), respectively, compared to the group administered 1 mg/kg of the negative control, and the groups administered 2 mg/kg of samples 1, 2, 3 and 4 showed tumor weight reductions of 33.3% ($p<0.001$), 43.4% ($p<0.001$), 35.2% ($p<0.001$) and 38.9% ($p<0.001$), respectively, compared to the group administered 2 mg/kg of the negative control (FIGS. 2c to 2e). Finally, blood was collected from the mice used in anticancer efficacy evaluation, and the presence or absence of hepatotoxicity markers in the blood was analyzed. As a result, no specific hepatotoxicity marker was detected in all the groups administered the samples (FIG. 2f). In addition, it could be confirmed that expression of VEGF and its downstream genes was effectively inhibited in the groups treated with the novel complexes (FIG. 2g).

**Example 3: Inhibition of PD-L1 in Tumor Tissue in Mice Transplanted with Human Breast Cancer Cells (MDA-MB-231)**

It is known that PD-L1 expressed on the surface of many types of cancer cells binds to PD-1 of T cells and continuously induces cancer cell proliferation while apoptosis is not induced by immune cells. Thus, a novel complex that targets PD-L1 in the tumor tissue of mice transplanted with human breast cancer cells showing high expression of PD-L1 was used to analyze whether it would exhibit an anticancer effect by inhibiting PD-L1.

[Table 8]

| Sequences of bioactive nucleic acid and carrier peptide nucleic acid for inhibiting PD-L1 activity | | | |
|---|---|---|---|
| Component | SEQ ID NO | Nucleotide sequence | Monomer modification |
| Bioactive nucleic acid | SEQ ID NO: 10 | 5'-GLFDIIKKIAESF-O-AT$^{(-)}$GA$^{(+)}$AAGC$^{(-)}$AA$^{(+)}$TGA$^{(-)}$T-O-K-3' | -+-+- |
| Carrier peptide nucleic acid | SEQ ID NO: 11 | 5'-K-O-AT$^{(+)}$CA$^{(+)}$T-GLFDIIKKIAESF-3' | +++ |

Example 3-1. Analysis of PD-L1 Protein Expression by Administration of Candidate Complex Comprising Bioactive Peptide Nucleic Acid and Carrier Peptide Nucleic Acid into Tail Vein of Mice Transplanted with Human Breast Cancer Cells (MDA-MB-231)

According to the same method as in Example 2-3, protein was isolated from the tumor tissue of the mice used in anticancer efficacy evaluation, and protein expression was analyzed using anti-PD-L1 antibody (Abeam, GB).

Example 3-2. Evaluation of Anticancer Efficacy in Tissue by Tail Vein Administration of Candidate Complex Comprising Bioactive Peptide Nucleic Acid and Carrier Peptide Nucleic Acid into Mice Transplanted with Human Breast Cancer Cells (MDA-MB-231)

The mouse tumor tissue used in anticancer efficacy evaluation in the same manner as in Example 2-1 was biopsied, and fixed in 4% formalin solution for one day. The fixed tissue was embedded in paraffin and sectioned to 5 um and mounted on slide glass. The mounted tissue was blocked in 0.5% BSA solution for 1 hour, treated with primary antibody solution against PD-L1, and incubated for one day. Next, the primary antibody solution was removed, and the remaining material was washed with 1X PBS, treated with secondary antibody solution, incubated at room temperature for 2 hours, and then analyzed by DAB straining.

The nucleic acid complexes used in this Example are shown in Table 9 below.

[Table 9]

| Nucleic acid complexes for evaluation of anticancer efficacy in breast cancer cells | |
|---|---|
| Group | Nucleic Complex |
| Group 1 | SEQ ID NO: 2 and SEQ ID NO: 6 |
| Group 2 | SEQ ID NO: 2 and SEQ ID NO: 8 |
| Group 3 | SEQ ID NO: 2 and SEQ ID NO: 6<br>SEQ ID NO: 10 and SEQ ID NO: 11 |
| Group 4 | SEQ ID NO: 2 and SEQ ID NO: 8<br>SEQ ID NO: 10 and SEQ ID NO: 11 |

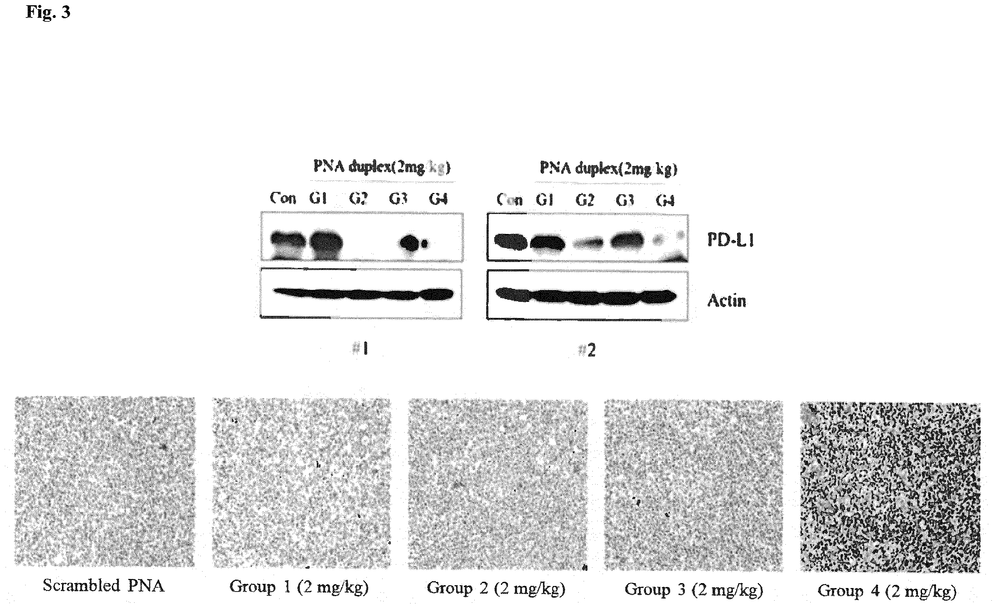

As a result, as shown in FIG. 3, it could be confirmed that, in the tumor tissue of the mice treated with the nucleic complex, protein expression decreased and expression of PD-L1 was inhibited.

**Example 4: Evaluation of Anticancer Effect by Inhibition of Androgen Receptor in Human Prostate Cancer Cells Using Novel Complex**

It is known that the normal development and preservation of the prostate depends on the role of the male hormone androgen through androgen receptor (AR), and that balance is maintained by regulation of the androgen receptor, because androgen induces apoptosis or replacement with new cells. However, it is known that, when testosterone or DHT (modified testosterone) is present, expression of androgen receptor increases, resulting in a loss of balance, which in turn causes cancer. An experiment was performed to confirm whether the novel nucleic acid complex exhibits an anticancer effect in human prostate cancer by inhibiting DHT-induced expression of androgen receptor in prostate cancer cells.

Example 4-1. Cell Culture

Human prostate cancer cells (LNCap) obtained from the ATCC (American Type Culture Collection, USA) were cultured in RPMI1640 (ATCC) (Roswell Park Memorial Institute, ATCC, USA) containing 10% (v/v) fetal bovine serum, 100 units/ml penicillin and 100 μg/ml streptomycin at 37°C under 5% (v/v) $CO_2$.

Example 4-2. Intracellular Introduction of Complex Comprising Bioactive Peptide Nucleic Acid and Carrier Peptide Nucleic Acid

Novel complexes for inhibiting androgen receptor were produced by preparing bioactive peptide nucleic acids having sequences (SEQ ID NO: 12 to SEQ ID NO: 15) complementary to androgen receptor mRNA, an essential gene for the growth and balance of prostate cells, preparing carrier peptide nucleic acids (SEQ ID NO: 16 to SEQ ID NO: 31) complementary to the bioactive peptide nucleic acids comprising a material for facilitating endosomal escape, and then hybridizing each bioactive peptide nucleic acid with each carrier peptide nucleic acid in equal amounts (see Table 10). A method for treating the complex in which the bioactive peptide nucleic acid and the carrier peptide nucleic acid are bound to each other is as described in PCT/KR2017/008636.

[Table 10]

| Sequences of bioactive nucleic acids and carrier peptide nucleic acids for inhibiting androgen receptor activity | | | |
|---|---|---|---|
| Component | SEQ ID NO | Nucleotide sequence | Monomer modification |
| Bioactive nucleic acid | SEQ ID NO: 12 | 5'-T$^{(-)}$CA$^{(+)}$ATAG$^{(-)}$TGCA$^{(+)}$AT$^{(-)}$CA-O-K-3' | -+-+- |
| | SEQ ID NO: 13 | 5'-CT$^{(-)}$CA$^{(+)}$ATG$^{(-)}$GCT$^{(+)}$TCC$^{(-)}$AG-O-K-3' | -+-+- |
| | SEQ ID NO: 14 | 5'-GLFDIIKKIAESF-O-T$^{(-)}$CA$^{(+)}$ATAG$^{(-)}$TGCA$^{(+)}$AT$^{(-)}$CA-O-K-3' | -+-+- |
| | SEQ ID NO: 15 | 5'-GLFDIIKKIAESF-O-CT$^{(-)}$CA$^{(+)}$ATG$^{(-)}$GCT$^{(+)}$TCC$^{(-)}$AG-O-K-3' | -+-+- |
| Carrier peptide nucleic acid | SEQ ID NO: 16 | 5'-K-O-TG$^{(+)}$AT$^{(+)}$T-3' | ++ |
| | SEQ ID NO: 17 | 5'-TT$^{(+)}$AG$^{(+)}$T-O-K-3' | ++ |
| | SEQ ID NO: 18 | 5'-TGAT$^{(+)}$TGCA$^{(+)}$CTAT$^{(+)}$TGA-O-K-3' | +++ |
| | SEQ ID NO: 19 | 5'-AGT$^{(+)}$TATC$^{(+)}$ACGT$^{(+)}$TAGT-O-K-3' | +++ |
| | SEQ ID NO: 20 | 5'-K-O-TG$^{(+)}$AT$^{(+)}$T-O-Histidine(10)-3' | ++ |
| | SEQ ID NO: 21 | 5'-Histidine(10)-O-TT$^{(+)}$AG$^{(+)}$T-O-K-3' | ++ |
| | SEQ ID NO: 22 | 5'-Histidine(10)-TGAT$^{(+)}$TGCA$^{(+)}$CTAT$^{(+)}$TGA-O-K-3' | +++ |
| | SEQ ID NO: 23 | 5'-Histidine(10)-AGT$^{(+)}$TATC$^{(+)}$ACGT$^{(+)}$TAGT-O-K-3' | +++ |
| | SEQ ID NO: 24 | 5'-K-O-CT$^{(+)}$GG$^{(+)}$A-3' | ++ |
| | SEQ ID NO: 25 | 5'-AG$^{(+)}$GT$^{(+)}$C-O-K-3' | ++ |
| | SEQ ID NO: 26 | 5'-CTGG$^{(+)}$AAGC$^{(+)}$CATT$^{(+)}$GAG-O-K-3' | +++ |
| | SEQ ID NO: 27 | 5'-GAGT$^{(+)}$TACC$^{(+)}$GAAG$^{(+)}$GTC-O-K-3' | +++ |

(continued)

| Sequences of bioactive nucleic acids and carrier peptide nucleic acids for inhibiting androgen receptor activity | | | |
|---|---|---|---|
| Component | SEQ ID NO | Nucleotide sequence | Monomer modification |
| | SEQ ID NO: 28 | 5'-K-O-CT$^{(+)}$GG$^{(+)}$A-O-Histidine(10)-3' | ++ |
| | SEQ ID NO: 29 | 5'-Histidine(10)-O-AG$^{(+)}$GT$^{(+)}$C-O-K-3' | ++ |
| | SEQ ID NO: 30 | 5'-Histidine(10)-CTGG$^{(+)}$AAGC$^{(+)}$CATT$^{(+)}$GAG-O-K-3' | +++ |
| | SEQ ID NO: 31 | 5'-Histidine(10)-GAGT$^{(+)}$TACC$^{(+)}$GAAG$^{(+)}$TC-O-K-3' | +++ |

Example 4-3. Analysis of Gene Expression by Western Blot Assay

To analyze the degree to which the novel complex inhibits androgen receptor, the cells cultured in Example 4-1 were seeded into a 6-well plate at a density of $1\times10^5$ cells/well, and treated with DHT (modified testosterone) to increase expression of androgen receptor. After 4 hours, the cells were treated with the complex, and then cultured for 72, 96, 120, 144 and 168 hours. After each culture time, protein expression was analyzed using anti-androgen receptor antibody (Cell Signaling, USA) and anti-p-Akt1 (Cell Signaling, USA) under the conditions disclosed in PCT/KR2017/008636.

The nucleic acid complexes used in this Example are shown in Table 11 below.

[Table 11]

| Nucleic acid complexes for analysis of expression of androgen receptor and p-Akt1 gene | |
|---|---|
| No. | Nucleic acid complex |
| 1 | SEQ ID NO: 12 and SEQ ID NO: 16 |
| 2 | SEQ ID NO: 12 and SEQ ID NO: 17 |
| 3 | SEQ ID NO: 12 and SEQ ID NO: 18 |
| 4 | SEQ ID NO: 12 and SEQ ID NO: 19 |
| 5 | SEQ ID NO: 13 and SEQ ID NO: 24 |
| 6 | SEQ ID NO: 13 and SEQ ID NO: 25 |
| 7 | SEQ ID NO: 13 and SEQ ID NO: 26 |
| 8 | SEQ ID NO: 13 and SEQ ID NO: 27 |
| 11 | SEQ ID NO: 14 and SEQ ID NO: 20 |
| 12 | SEQ ID NO: 14 and SEQ ID NO: 21 |
| 13 | SEQ ID NO: 14 and SEQ ID NO: 22 |
| 14 | SEQ ID NO: 14 and SEQ ID NO: 23 |
| 15 | SEQ ID NO: 15 and SEQ ID NO: 28 |
| 16 | SEQ ID NO: 15 and SEQ ID NO: 29 |
| 17 | SEQ ID NO: 15 and SEQ ID NO: 30 |
| 18 | SEQ ID NO: 15 and SEQ ID NO: 31 |

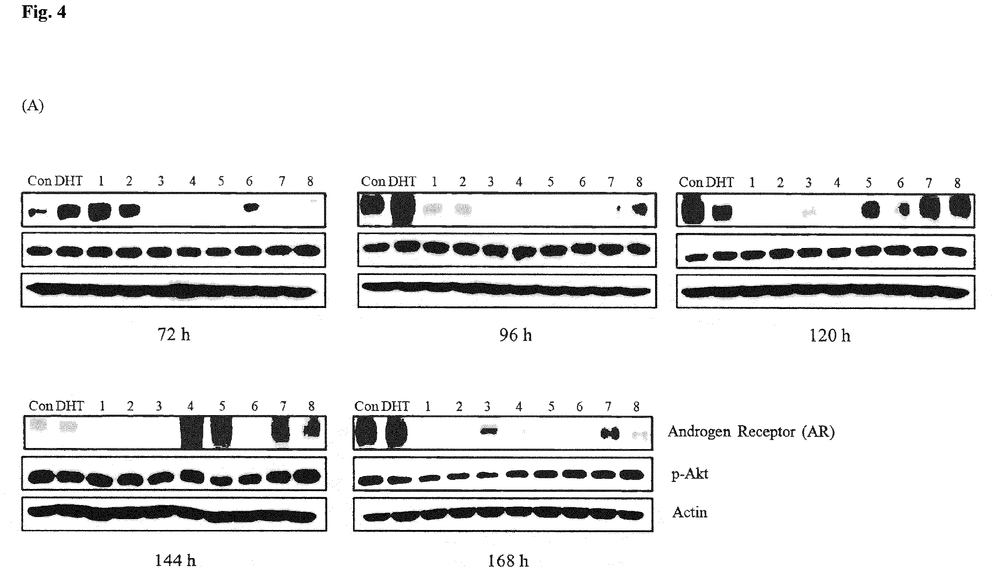

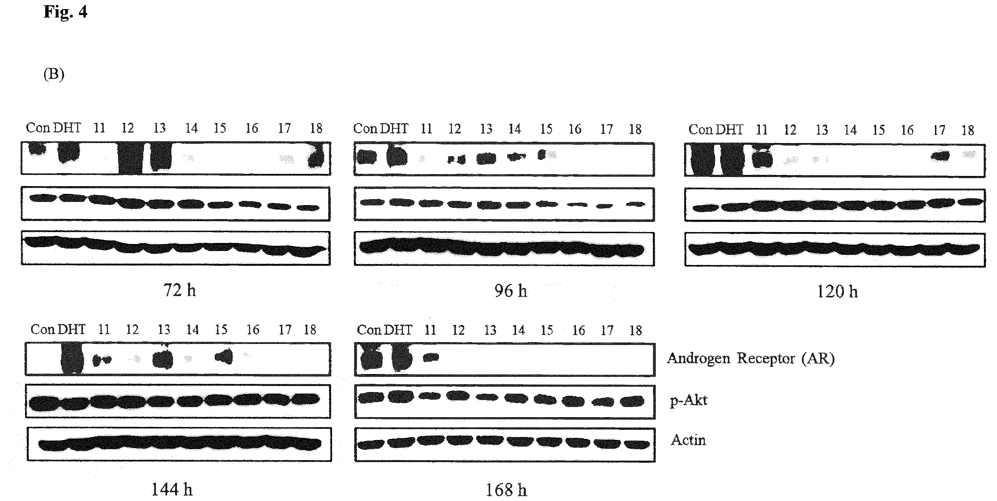

As a result, as shown in FIG. 4, it was confirmed that protein expression of androgen receptor whose expression was increased by DHT and the downstream gene p-Akt1 was inhibited.

**Example 5: Evaluation of Anticancer Effect by Inhibition of Clusterin in Human Prostate Cancer Cells Using Novel Complex**

Clusterin, also called apolipoprotein J, is a heterodimeric glycoprotein present in the body fluids. It is known that clusterin is induced and expressed in almost all types of cells studied so far, and is involved in various normal biological processes, such as sperm maturation, lipid transport, tissue reconstitution, cell membrane recycling, cell-cell or cell-matrix interactions, and apoptosis. The potent tumorigenic capacity of clusterin involved in the development and progression of various malignant tumors in humans has been reported, and it is known that clusterin stimulates cancer cell growth by inhibiting expression of Bax, which regulates apoptosis, and is also involved in cancer cell resistance. An experiment was performed to examine whether the novel complex exhibits an anticancer effect by inhibiting overexpression of clusterin in human prostate cancer cells.

Example 5-1. Cell Culture

Prostate cancer cells (PC-3) obtained from the ATCC (American Type Culture Collection, USA) were cultured in F12K culture medium (Kaighn's Modification of Ham's F-12 Medium, Gibco, USA) containing 10% (v/v) fetal bovine serum, 100 units/ml penicillin and 100 ug/ml streptomycin at 37°C under 5% (v/v) $CO_2$.

Example 5-2. Intracellular Introduction of Complex Comprising Bioactive Peptide Nucleic Acid and Carrier Peptide Nucleic Acid

Novel complexes for inhibiting Clusterin were produced by preparing bioactive peptide nucleic acids having sequences (SEQ ID NO: 32 to SEQ ID NO: 35) complementary to VEGF mRNA, an essential gene for vascular growth, preparing carrier peptide nucleic acids (SEQ ID NO: 36 to SEQ ID NO: 51) complementary to the bioactive peptide nucleic acids comprising a material for facilitating endosomal escape, and then hybridizing each bioactive peptide nucleic acid with each carrier peptide nucleic acid in equal amounts (see Table 12). A method for treating the complex in which the bioactive peptide nucleic acid and the carrier peptide nucleic acid are bound to each other is as described in PCT/KR2017/008636.

[Table 12]

| Sequences of bioactive nucleic acids and carrier peptide nucleic acids for inhibiting Clusterin activity | | | |
|---|---|---|---|
| Component | SEQ ID NO | Nucleotide sequence | Monomer modification |
| Bioactive nucleic acid | SEQ ID NO: 32 | 5'-TAC$^{(-)}$TCA$^{(+)}$AG$^{(-)}$CTAC$^{(-)}$AT$^{(-)}$TCA-O-K-3' | -+-+- |
| | SEQ ID NO: 33 | 5'-GA$^{(-)}$GCC$^{(+)}$AC$^{(-)}$TTCT$^{(+)}$GC$^{(-)}$A-O-K-3' | -+-+- |
| | SEQ ID NO: 34 | 5'-GLFDIIKKIAESF-O-TAC$^{(-)}$TCA$^{(+)}$AG$^{(-)}$CTAC$^{(+)}$AT$^{(-)}$TCA-O-K-3' | -+-+- |
| | SEQ ID NO: 35 | 5'-GLFDIIKKIAESF-O-GA$^{(-)}$GCC$^{(+)}$AC$^{(-)}$TTCT$^{(+)}$GC$^{(-)}$A-O-K-3' | -+-+- |
| Carrier peptide nucleic acid | SEQ ID NO: 36 | 5'-K-O-A$^{(+)}$TG$^{(+)}$TC-3' | ++ |
| | SEQ ID NO: 37 | 5'-CT$^{(+)}$GT$^{(+)}$A-O-K-3' | ++ |
| | SEQ ID NO: 38 | 5'-ATGT$^{(+)}$CCAA$^{(+)}$TCAG$^{(+)}$GGA-O-K-3' | +++ |
| | SEQ ID NO: 39 | 5'-AGG$^{(+)}$GACT$^{(+)}$AACC$^{(+)}$TGTA-O-K-3' | +++ |
| | SEQ ID NO: 40 | 5'-K-O-A$^{(+)}$TG$^{(+)}$TC-O-Histidine(10)-3' | ++ |
| | SEQ ID NO: 41 | 5'-Histidine(10)-O-CT$^{(+)}$GT$^{(+)}$A-O-K-3' | ++ |

(continued)

| Sequences of bioactive nucleic acids and carrier peptide nucleic acids for inhibiting Clusterin activity | | | |
|---|---|---|---|
| Component | SEQ ID NO | Nucleotide sequence | Monomer modification |
| | SEQ ID NO: 42 | 5'-Histidine(10)-ATGT$^{(+)}$CCAA$^{(+)}$TCAG$^{(+)}$GGA-O-K-3' | +++ |
| | SEQ ID NO: 43 | 5'-Histidine(10)-AGG$^{(+)}$GACT$^{(+)}$AACC$^{(+)}$TGTA-O-K-3' | +++ |
| | SEQ ID NO: 44 | 5'-K-O-TG$^{(+)}$CA$^{(+)}$G-3' | ++ |
| | SEQ ID NO: 45 | 5'-GA$^{(+)}$CG$^{(+)}$T-O-K-3' | ++ |
| | SEQ ID NO: 46 | 5'-TG$^{(+)}$CAGA$^{(+)}$AGTG$^{(+)}$GCTC-O-K-3' | +++ |
| | SEQ ID NO: 47 | 5'-CTC$^{(+)}$GGTGA$^{(+)}$AGAC$^{(+)}$GT-O-K -3' | +++ |
| | SEQ ID NO: 48 | 5'-K-O-TG$^{(+)}$CA$^{(+)}$G-O-Histidine(10)-3' | ++ |
| | SEQ ID NO: 49 | 5'-Histidine(10)-O-GA$^{(+)}$CG$^{(+)}$T-O-K-3' | ++ |
| | SEQ ID NO: 50 | 5'-Histidine(10)-TG$^{(+)}$CAGA$^{(+)}$AGTG$^{(+)}$GCTC-O-K-3' | +++ |
| | SEQ ID NO: 51 | 5'-Histidine(10)-CTC$^{(+)}$GGTGA$^{(+)}$AGAC$^{(+)}$GT-O-K-3' | +++ |

Example 5-3. Analysis of Cell Viability in Human Prostate Cancer Cells

To analyze the degree to which the novel complex inhibits vascular endothelial growth factor, the cells cultured in Example 5-1 were seeded into a 96-well plate at a density of $6\times10^3$ cells/well, treated with the complex, and then cultured for 24, 48, 72, 96 and 120 hours. After each culture time, cell viability was analyzed under the experimental conditions disclosed in PCT/KR2017/008636.

The nucleic acid complexes used in this Example are shown in Table 13 below.

[Table 13]

| Nucleic acid complexes for analysis of cell viability in prostate cancer cells | |
|---|---|
| Name | Nucleic acid complex |
| Original PNA duplex 1 | SEQ ID NO: 32 and SEQ ID NO: 36 |
| Original PNA duplex 2 | SEQ ID NO: 32 and SEQ ID NO: 37 |
| Original PNA duplex 3 | SEQ ID NO: 32 and SEQ ID NO: 38 |
| Original PNA duplex 4 | SEQ ID NO: 32 and SEQ ID NO: 39 |
| Original PNA duplex 5 | SEQ ID NO: 33 and SEQ ID NO: 44 |
| Original PNA duplex 6 | SEQ ID NO: 33 and SEQ ID NO: 45 |
| Original PNA duplex 7 | SEQ ID NO: 33 and SEQ ID NO: 46 |
| Original PNA duplex 8 | SEQ ID NO: 33 and SEQ ID NO: 47 |
| Modified PNA duplex 1 | SEQ ID NO: 34 and SEQ ID NO: 40 |
| Modified PNA duplex 2 | SEQ ID NO: 34 and SEQ ID NO: 41 |

(continued)

| Nucleic acid complexes for analysis of cell viability in prostate cancer cells | |
|---|---|
| Name | Nucleic acid complex |
| Modified PNA duplex 3 | SEQ ID NO: 34 and SEQ ID NO: 42 |
| Modified PNA duplex 4 | SEQ ID NO: 34 and SEQ ID NO: 43 |
| Modified PNA duplex 5 | SEQ ID NO: 35 and SEQ ID NO: 48 |
| Modified PNA duplex 6 | SEQ ID NO: 35 and SEQ ID NO: 49 |
| Modified PNA duplex 7 | SEQ ID NO: 35 and SEQ ID NO: 50 |
| Modified PNA duplex 8 | SEQ ID NO: 35 and SEQ ID NO: 51 |

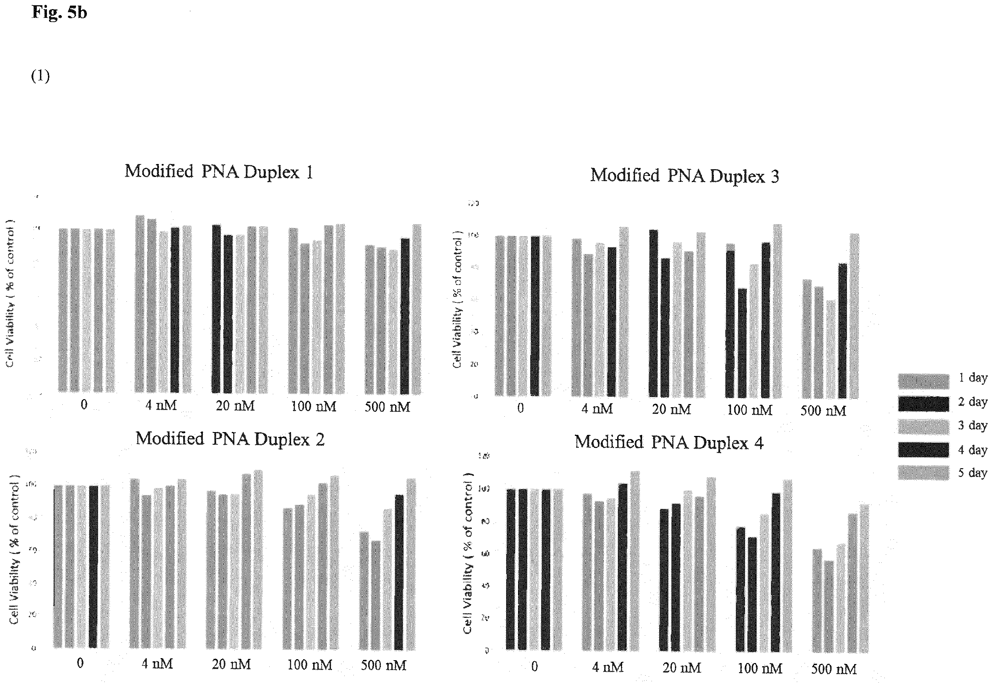

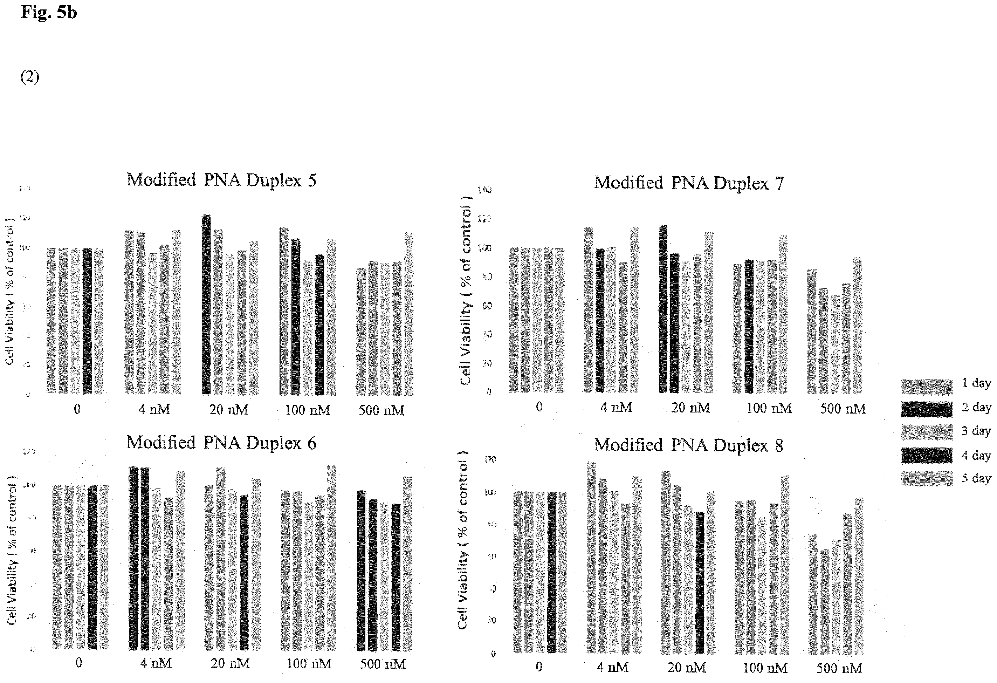

As a result, as shown in FIGS. 5a and 5b, it was confirmed in the human prostate cancer cells that the novel complex reduced cell viability by inhibiting clusterin.

Example 5-4. Analysis of Gene Expression by Western Blot Assay

To analyze the degree to which the novel complex inhibits clusterin, the cells cultured in Example 5-1 were seeded into a 6-well plate at a density of $1\times10^5$ cells/well, treated with the complex, and then cultured for 24, 48, 72, 96 and 120 hours. After each culture time, protein expression was analyzed using anti-clusterin (Abeam, GB) and anti-Bax (SantaCruz Biotech., USA) under the conditions disclosed in PCT/KR2017/008636.

The nucleic acid complexes used in this Example are shown in Table 14 below.

[Table 14]

| Nucleic acid complexes for analysis of expression of clusterin and Bax genes | |
|---|---|
| No. | Nucleic acid complex |
| 1 | SEQ ID NO: 32 and SEQ ID NO: 36 |
| 2 | SEQ ID NO: 32 and SEQ ID NO: 37 |
| 3 | SEQ ID NO: 32 and SEQ ID NO: 38 |
| 4 | SEQ ID NO: 32 and SEQ ID NO: 39 |
| 5 | SEQ ID NO: 33 and SEQ ID NO: 44 |
| 6 | SEQ ID NO: 33 and SEQ ID NO: 45 |
| 7 | SEQ ID NO: 33 and SEQ ID NO: 46 |
| 8 | SEQ ID NO: 33 and SEQ ID NO: 47 |
| 11 | SEQ ID NO: 34 and SEQ ID NO: 40 |
| 12 | SEQ ID NO: 34 and SEQ ID NO: 41 |
| 13 | SEQ ID NO: 34 and SEQ ID NO: 42 |
| 14 | SEQ ID NO: 34 and SEQ ID NO: 43 |
| 15 | SEQ ID NO: 35 and SEQ ID NO: 48 |
| 16 | SEQ ID NO: 35 and SEQ ID NO: 49 |
| 17 | SEQ ID NO: 35 and SEQ ID NO: 50 |
| 18 | SEQ ID NO: 35 and SEQ ID NO: 51 |

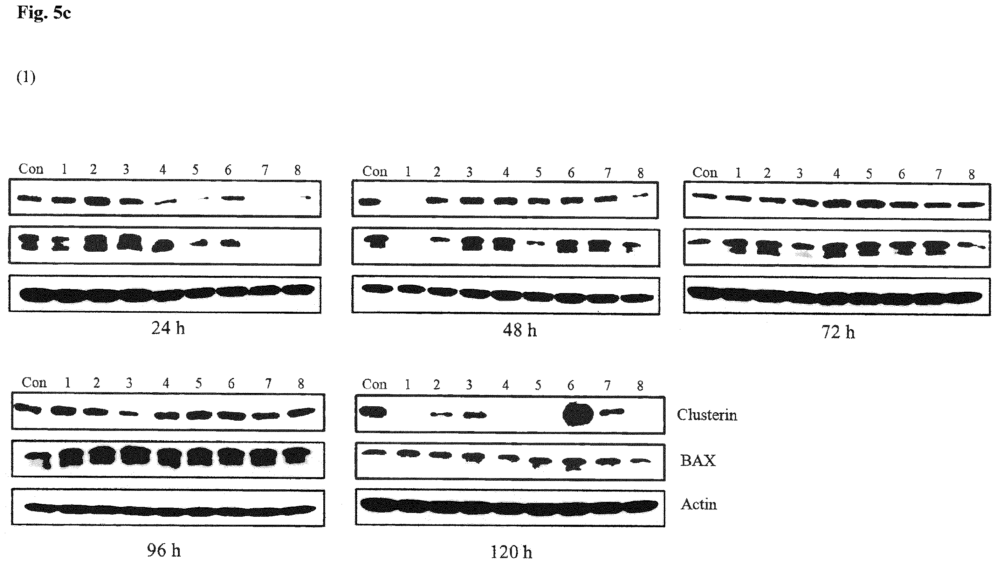

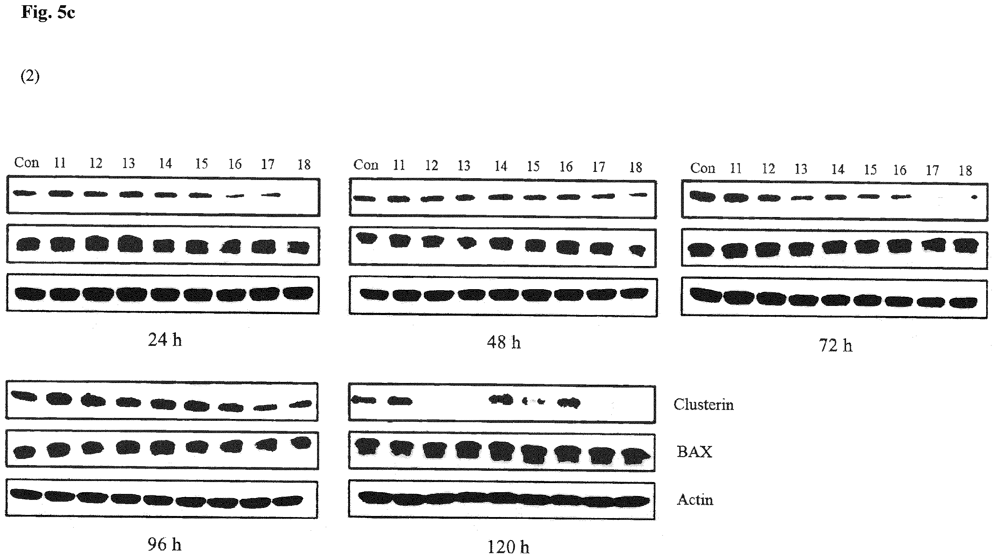

As a result, as shown in FIG. 5c, it was confirmed that protein expression of the antiapoptotic and cancer-inducing gene clusterin and the regulatory gene Bax was inhibited.

**Example 6: Examination of Inhibition of Vascular Endothelial Growth Factor in Human Retinal Pigment Epithelial Cells Using Novel Complex**

A novel complex against the vascular endothelial growth factor VEGF known to cause age-related macular degeneration in the macula was used to examine whether treatment of human retinal pigment epithelial cells with the complex would inhibit angiogenesis.

Example 6-1. Cell Culture

Human retinal pigment epithelial cells (ARPE-19) obtained from ATCC (American Type Culture Collection, USA) were cultured in DMEM-F12 culture medium (Dulbecco's Modified Eagle Medium/Nutrient Mixture F-12, Gibco, USA) containing 10% (v/v) fetal bovine serum, 100 units/ml penicillin and 100 μg/ml streptomycin at 37°C under 5% (v/v) $CO_2$.

Example 6-2. Intracellular Introduction of Complex Comprising Bioactive Peptide Nucleic Acid and Carrier Peptide Nucleic Acid

Novel complexes for inhibiting vascular endothelial growth factor were produced by preparing bioactive peptide nucleic acids having sequences (SEQ ID NO: 1 to SEQ ID NO: 3) complementary to VEGF mRNA, an essential gene for vascular growth, preparing carrier peptide nucleic acids (SEQ ID NO: 4 to SEQ ID NO: 9) complementary to the bioactive peptide nucleic acids comprising a material for facilitating endosomal escape, and then hybridizing each bioactive peptide nucleic acid with each carrier peptide nucleic acid in equal amounts (see Table 2). A method for treating the complex in which the bioactive peptide nucleic acid and the carrier peptide nucleic acid are bound to each other is as described in PCT/KR2017/008636.

Example 6-3. Analysis of Cell Viability in Human Retinal Pigment Epithelial Cells

To analyze the degree to which the novel complex inhibits vascular endothelial growth factor, the cells cultured in Example 6-1 were seeded into a 96-well plate at a density of $6\times10^3$ cells/well, treated with the complex, and then cultured for 24, 48, 72, 96 and 120 hours. After each culture time, cell viability was analyzed under the experimental conditions disclosed in PCT/KR2017/008636.

The nucleic acid complexes used in this Example are shown in Table 15 below.

[Table 15]

| Nucleic acid complexes for analysis of cell viability in retinal pigment epithelial cells | |
|---|---|
| Name | Nucleic acid complex |
| Original | SEQ ID NO: 1 and SEQ ID NO: 4 |
| PNA 1 | SEQ ID NO: 2 and SEQ ID NO: 4 |
| PNA 2 | SEQ ID NO: 3 and SEQ ID NO: 4 |
| PNA 3 | SEQ ID NO: 2 and SEQ ID NO: 5 |
| PNA 4 | SEQ ID NO: 2 and SEQ ID NO: 6 |
| PNA 5 | SEQ ID NO: 2 and SEQ ID NO: 7 |
| PNA 6 | SEQ ID NO: 2 and SEQ ID NO: 8 |

As a result, as shown in FIG. 6a, it was confirmed in the human retinal pigment epithelial cells that the novel complex inhibited cell viability by inhibiting vascular endothelial growth factor.

Example 6-4. Analysis of Gene Expression by Western Blot Assay

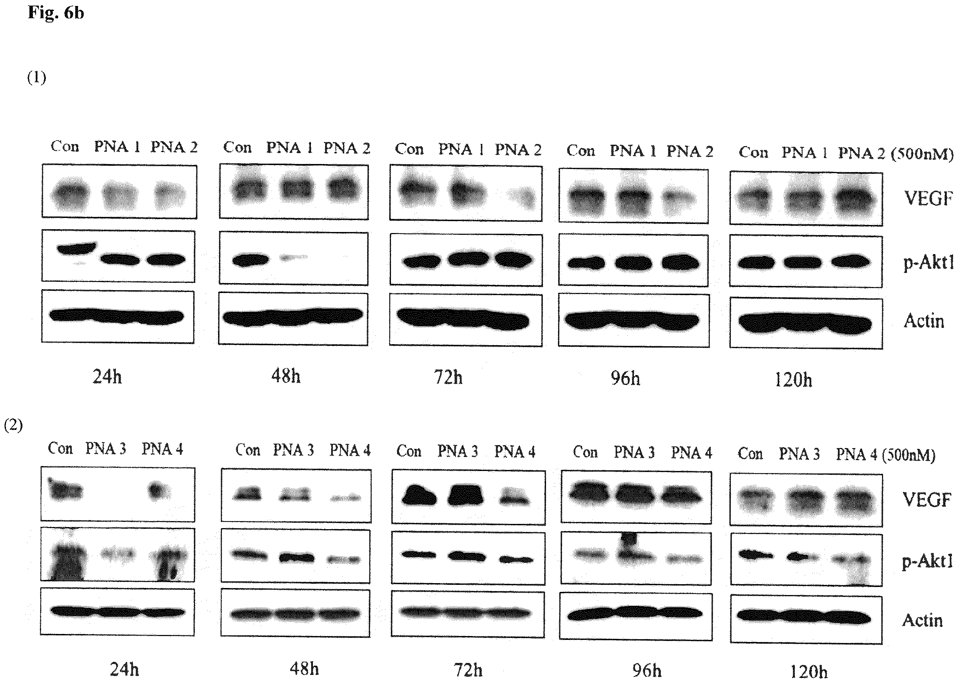

To analyze the degree to which the novel complex inhibits vascular endothelial growth factor, the cells cultured in Example 6-1 were seeded into a 6-well plate at a density of $1\times10^5$ cells/well, treated with the complex, and then cultured for 24, 48, 72, 96 and 120 hours. After each culture time, protein expression was analyzed using anti-VEGF antibody (SantaCruz Biotech., USA) and anti-p-Akt1 (Cell Signaling, USA). The nucleic acid complexes in Table 15 above were used. As shown in FIG. 6b, it was confirmed that protein expression of the vascular endothelial growth factor (VEGF) and its downstream gene p-Akt1 was inhibited.

Example 6-5. Analysis of Inhibition of Angiogenesis in Retina of Mice Using Intravitreal Injections

In order to examine the degree to which the novel complex inhibits vascular endothelial growth factor in the mouse retina, P0 and P16 newborn mice from pregnant mice (Nara Biotech Co., Korea) were injected intravitreally with 30 μg/kg of the novel complex, and after one week, were compared with a negative control group. Before the retina was isolated from the mice, 250 mg/ml of FITC-dextran (Sigma, USA) was injected behind the eye, and then the retina was isolated and analyzed with a fluorescence microscope.

The nucleic acid complexes used in this Example are shown in Table 16 below.

[Table 16]

| Nucleic acid complexes for analysis of inhibition of angiogenesis in retina | |
|---|---|
| Name | Nucleic acid complex |
| PNA duplex | SEQ ID NO: 2 and SEQ ID NO: 8 |
| Modified PNA duplex 1 | SEQ ID NO: 2 and SEQ ID NO: 6 |
| Modified PNA duplex 2 | SEQ ID NO: 2 and SEQ ID NO: 8 |

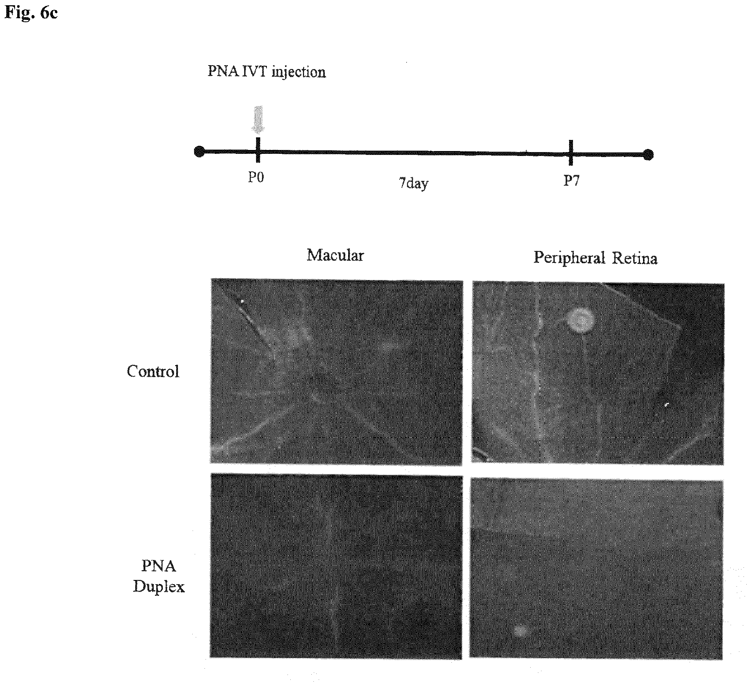

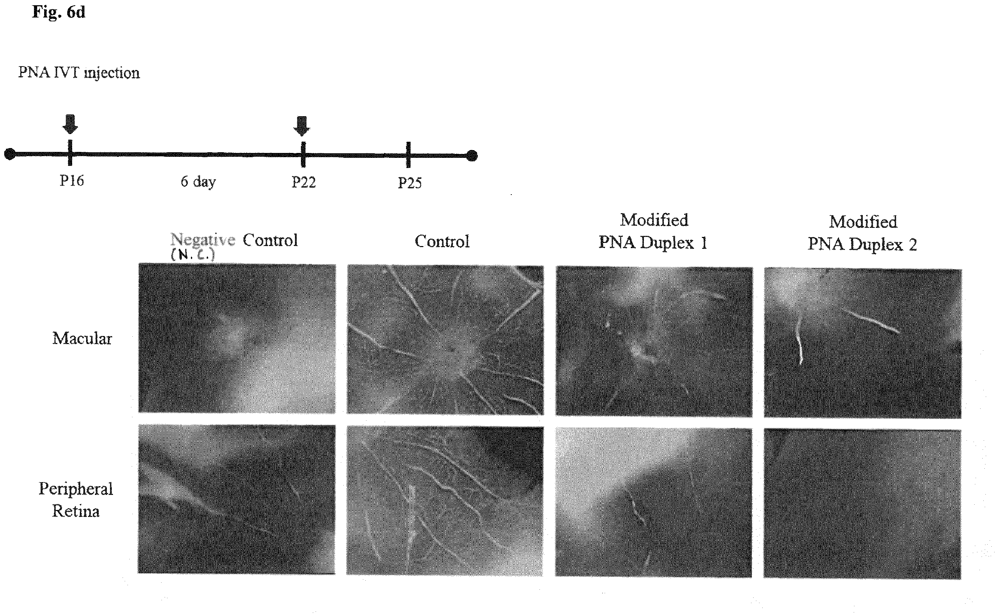

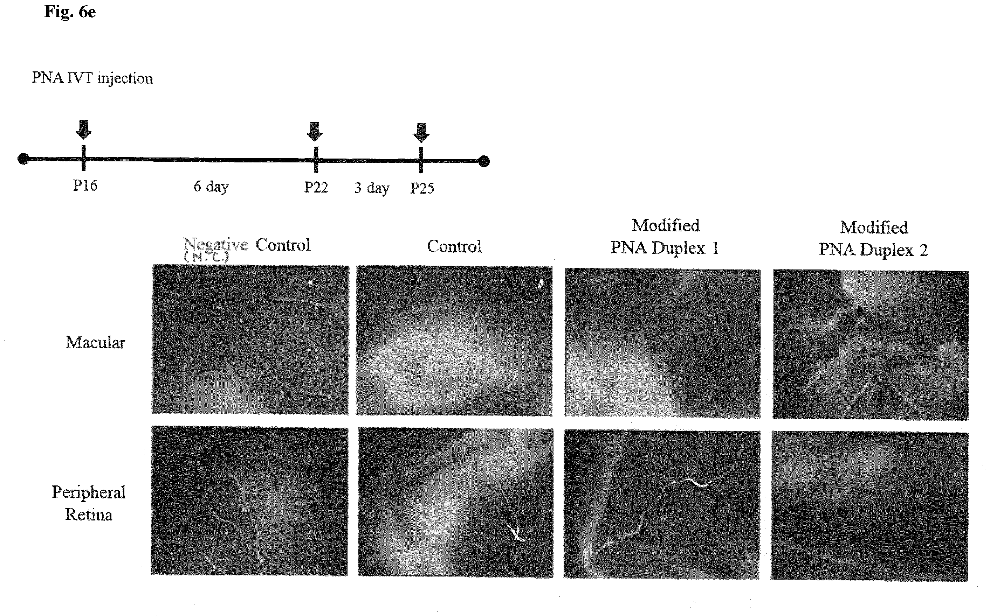

As a result, as shown in FIGS. 6c, 6d and 6e, it could be confirmed that the novel complex efficiently inhibited angiogenesis in the mouse retina by inhibiting the vascular endothelial growth factor.

Example 6-6. Analysis of Inhibition of Angiogenesis in Retina of Mice Using Eye Drops

In order to examine the degree to which the novel complex inhibits vascular endothelial growth factor in the mouse retina, P4 newborn mice from pregnant mice (Nara Biotech Co., Korea) were treated with 30 μg/kg of the novel complex as eye drops once a day for 5 days, and then compared with a negative control group. Before the retina was isolated from the mice, 250 mg/ml of FITC-dextran (Sigma, USA) was injected behind the eye, and then the retina was isolated and analyzed with a fluorescence microscope.

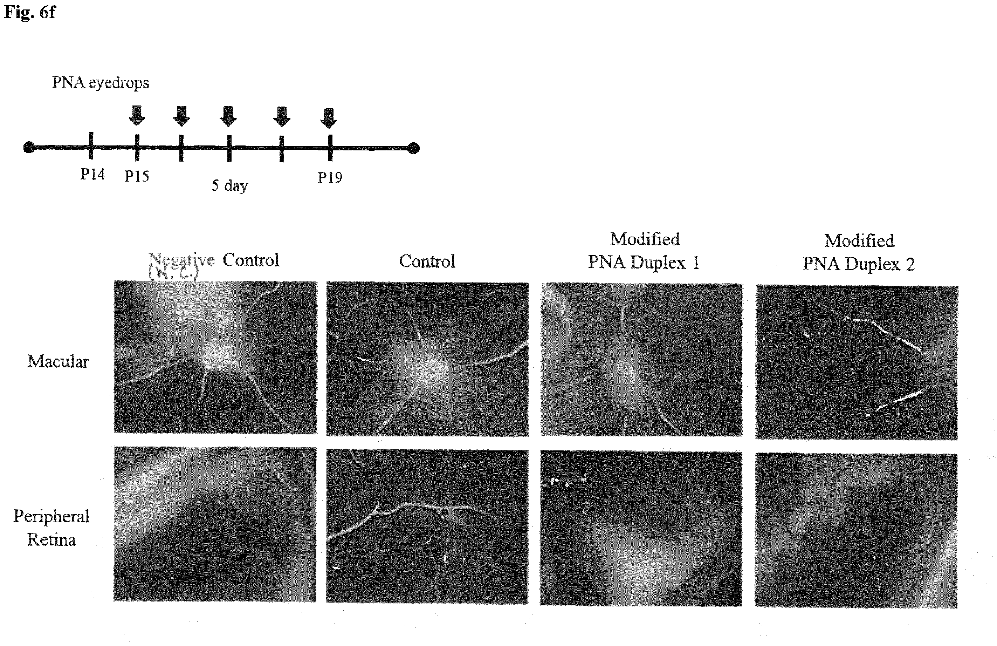

As the novel complex, a modified PNA duplex was used, which is a nucleic acid complex represented by SEQ ID NO: 2 and SEQ ID NO: 8. As a result, as shown in FIG. 6f, it could be confirmed that the use of the novel complex as eye drops efficiently inhibited angiogenesis in the mouse retina by inhibiting the vascular endothelial growth factor.

## Example 7: Examination of Inhibition of PDE4B in Human Respiratory Epithelial Cells and Lung Cancer Cells Using Novel Complex

Chronic obstructive pulmonary disease (COPD) refers to a condition in which lung function is reduced due to chronic inflammation of the airways and destruction of the parenchyma. The disease occurs mainly in middle-aged and older people who have a history of smoking, and also occurs in people with chronic inflammation. This disease is known as an important disease that causes physical disability or dysfunction in daily life due to dyspnea, is accompanied by mental problems such as depression and anxiety, and increases hospitalization rate and death. An inhibitor of PDE4 was used for treatment of COPD, but it is not currently used because it is accompanied by side effects. In recent years, it has been known that inhibition of PDE4B, a member of the PDE4 family, exhibits an anti-inflammatory effect by inhibiting degradation of cAMP. Thus, an experiment was performed to examine the novel complex exhibits a therapeutic effect against COPD by inhibiting PDE4B.

Example 7-1. Cell Culture

Human respiratory epithelial cells (NCI-H292) obtained from the ATCC (American Type Culture Collection, USA), were cultured in RPMI1640 (ATCC) (Roswell Park Memorial Institute, ATCC, USA) and human lung cancer cells (A549) were cultured in F12K culture medium (Kaighn's Modification of Ham's F-12 Medium, Gibco, USA), respectively, each containing 10% (v/v) fetal bovine serum, 100 units/ml penicillin and 100 μg/ml streptomycin, at 37°C under 5% (v/v) $CO_2$.

Example 7-2. Intracellular Introduction of Complex Comprising Bioactive Peptide Nucleic Acid and Carrier Peptide Nucleic Acid

Novel complexes for inhibiting PDE4B were produced by preparing bioactive peptide nucleic acids having sequences (SEQ ID NO: 52 to SEQ ID NO: 55) complementary to PDE4B mRNA, which is associated with degradation of cAMP (an important inflammation inducer), preparing carrier peptide nucleic acids (SEQ ID NO: 56 to SEQ ID NO: 63) complementary to the bioactive peptide nucleic acids comprising a material for facilitating endosomal escape, and then hybridizing each bioactive peptide nucleic acid with each carrier peptide nucleic acid in equal amounts (see Table 17). A method for treating the complex in which the bioactive peptide nucleic acid and the carrier peptide nucleic acid are bound to each other is as described in PCT/KR2017/008636.

[Table 17]

| Sequences of bioactive nucleic acids and carrier peptide nucleic acids for inhibiting PDE4B activity | | | |
|---|---|---|---|
| Component | SEQ ID NO | Nucleotide sequence | Monomer modification |
| Bioactive nucleic acid | SEQ ID NO: 52 | 5'-TAC$^{(-)}$TCA$^{(+)}$AG$^{(-)}$CTAC$^{(+)}$AT$^{(-)}$TCA-O-K-3' | -+-+- |
| | SEQ ID NO: 53 | 5'-TC$^{(-)}$CAC$^{(+)}$AT$^{(-)}$CAA$^{(+)}$AGC$^{(-)}$ACT-O-K-3' | -+-+- |
| | SEQ ID NO: 54 | 5'-GLFDIIKKIAESF-O-TAC$^{(-)}$TCA$^{(+)}$AG$^{(-)}$CTAC$^{(+)}$AT$^{(-)}$TCA-O-K-3' | -+-+- |
| | SEQ ID NO: 55 | 5'-GLFDIIKKIAESF-O-TC$^{(-)}$CAC$^{(+)}$AT$^{(-)}$CAA$^{(+)}$AGC$^{(-)}$ACT-O-K -3' | -+-+- |
| Carrier peptide nucleic acid | SEQ ID NO: 56 | 5'-K-O-TC$^{(+)}$AA$^{(+)}$T-3' | ++ |
| | SEQ ID NO: 57 | 5'-ATGA$^{(+)}$GTTCGA$^{(+)}$TGTA$^{(+)}$AGT-O-K-3' | ++ |
| | SEQ ID NO: 58 | 5'-K-O-TC$^{(+)}$AA$^{(+)}$T-O-Histidine(10)-3' | +++ |
| | SEQ ID NO: 59 | 5'-Histidine(10)-ATGA$^{(+)}$GTTCGA$^{(+)}$TGTA$^{(+)}$AGT-O-K -3' | +++ |
| | SEQ ID NO: 60 | 5'-K-O-AG$^{(+)}$TG$^{(+)}$C-3' | ++ |
| | SEQ ID NO: 61 | 5'-AGGT$^{(+)}$GTAGT$^{(+)}$TTCG$^{(+)}$TGA-O-K-3' | ++ |
| | SEQ ID NO: 62 | 5'-K-O-AG$^{(+)}$TG$^{(+)}$C-O-Histidine(10)-3' | +++ |
| | SEQ ID NO: 63 | 5'-Histidine(10)-AGGT$^{(+)}$GTAGT$^{(+)}$TTCG$^{(+)}$TGA-O-K -3' | +++ |

Example 7-3. Analysis of Gene Expression by Western Blot Assay

To analyze the degree to which the novel complex inhibits PDE4B, the cells cultured in Example 7-1 were seeded into a 6-well plate at a density of $1\times10^5$ cells/well, and treated with the complex. After 4 hours, pathogenesis models were prepared by treating the human respiratory epithelial cells with CSE (Sigma, USA) and treating the human lung cancer cells with LPS (Sigma, USA), and were cultured for 72, 96 and 120 hours. After each culture time, protein expression was analyzed using anti-PDE4B antibody (Abeam, GB), anti-IL-6 antibody (Cell Signaling, USA) and anti-TNF-$\alpha$ antibody (SantaCruz Biotech., USA) under the conditions disclosed in PCT/KR2017/008636.

The nucleic acid complexes used in this Example are shown in Table 18 below.

[Table 18]

| Nucleic acid complexes for analysis of inhibition of PDE4B expression | |
|---|---|
| No. | Nucleic acid complex |
| 1 | SEQ ID NO: 52 and SEQ ID NO: 56 |
| 2 | SEQ ID NO: 52 and SEQ ID NO: 57 |

(continued)

| Nucleic acid complexes for analysis of inhibition of PDE4B expression | |
|---|---|
| No. | Nucleic acid complex |
| 3 | SEQ ID NO: 54 and SEQ ID NO: 58 |
| 4 | SEQ ID NO: 54 and SEQ ID NO: 59 |
| 5 | SEQ ID NO: 53 and SEQ ID NO: 60 |
| 6 | SEQ ID NO: 53 and SEQ ID NO: 61 |
| 7 | SEQ ID NO: 55 and SEQ ID NO: 62 |
| 8 | SEQ ID NO: 55 and SEQ ID NO: 63 |

As a result, as shown in FIG. 7, it was confirmed that the novel complex inhibited protein expression of PDE4B whose expression was increased by CSE and LPS, and protein expression IL-6 and TNF-A, which are representative inflammation markers in COPD.

**Example 8: Examination of Inhibition of TLR2 in Atopic Dermatitis Using Novel Complex**

To analyze the therapeutic effect of the nucleic acid complex against atopic dermatitis, TLR2 (Toll-Like Receptor 2) was used as a target gene. TLR2 is a gene that is expressed when allergens or bacteria penetrate the skin. TLR2 is overexpressed in atopic dermatitis patients, and exacerbates atopic dermatitis due to increased inflammation caused by inflammatory cytokines in the skin. For this reason, TLR2 is considered an important target in atopic dermatitis.

Example 8-1: Cell Culture

Human keratinocytes (HaCaT) obtained from the CLS (CLS Cell Lines Service, Germany) were cultured in DMEM culture medium (Dulbecco Modified Eagle Medium, Welgene, Korea) containing 10% (v/v) fetal bovine serum, 100 units/ml penicillin and 100 pg/ml streptomycin at 37°C under 5% (v/v) $CO_2$. To prepare a cell model mimicking atopic dermatitis, the cells were treated 5 ng/mL house dust mite extract and 5 μM DNCB (2-dinitrochlorobenzene) and incubated for one day.

Example 8-2. Intracellular Introduction of Complex Comprising Bioactive Peptide Nucleic Acid and Carrier Peptide Nucleic Acid

Novel complexes for inhibiting TLR2 were produced by preparing bioactive peptide nucleic acids having sequences (SEQ ID NO: 64 and SEQ ID NO: 65) complementary to TLR2 mRNA, preparing carrier peptide nucleic acids (SEQ ID NO: 66 to SEQ ID NO: 71) complementary to the bioactive peptide nucleic acids comprising a material for facilitating endosomal escape, and then hybridizing each bioactive peptide nucleic acid with each carrier peptide nucleic acid in equal amounts (see Table 19). A method for treating the complex in which the bioactive peptide nucleic acid and the carrier peptide nucleic acid are bound to each other is as described in PCT/KR2017/008636.

[Table 19]

| Sequences of bioactive nucleic acids and carrier peptide nucleic acids for inhibiting TLR2 activity | | | |
|---|---|---|---|
| Component | SEQ ID NO | Nucleotide sequence | Monomer modification |
| Bioactive nucleic acid | SEQ ID NO: 64 | 5'-A$^{(-)}$TGT$^{(+)}$AGG$^{(-)}$TG$^{(+)}$ATCC$^{(-)}$TGTT-O-K-3' | -+-+- |
| | SEQ ID NO: 65 | 5'-GLFDIIKKIAESF-O-A$^{(-)}$TGT$^{(+)}$AGG$^{(-)}$TG$^{(+)}$ATCC$^{(-)}$TGTT-O-K -3' | -+-+- |

(continued)

| Sequences of bioactive nucleic acids and carrier peptide nucleic acids for inhibiting TLR2 activity | | | |
|---|---|---|---|
| Component | SEQ ID NO | Nucleotide sequence | Monomer modification |
| Carrier peptide nucleic acid | SEQ ID NO: 66 | 5'-K-O-A$^{(+)}$AC$^{(+)}$AG-3' | ++ |
| | SEQ ID NO: 67 | 5'-K-O-A$^{(+)}$AC$^{(+)}$AG-O-Histidine(10)-3' | ++ |
| | SEQ ID NO: 68 | 5'-Histidine(10)-O-G$^{(+)}$AC$^{(+)}$AA-O-K-3' | ++ |
| | SEQ ID NO: 69 | 5'-T$^{(+)}$ACATCC$^{(+)}$ACTAGG$^{(+)}$ACAA-O-K-3' | +++ |
| | SEQ ID NO: 70 | 5'-K-O-T$^{(+)}$ACAGGA$^{(+)}$TCACCT$^{(+)}$ACAT-O-Histidine(10)-3' | +++ |
| | SEQ ID NO: 71 | 5'-Histidine(10)-O-T$^{(+)}$ACATCC$^{(+)}$ACTAGG$^{(+)}$ACAA-O-K-3' | +++ |

Example 8-3: Change in Cell Viability in Human Keratinocytes

To analyze the degree to which the novel complex inhibits TLR2, the cells cultured in Example 8-1 were seeded into a 96-well plate at a density of $6\times10^3$ cells/well, treated with the complex, and then cultured for 24, 48 and 72 hours. After each culture time, cell viability was analyzed under the experimental conditions disclosed in PCT/KR2017/008636.

As a result, as shown in FIG. 8a, it was confirmed in the human keratinocytes that the novel complex reduced cell viability by inhibiting TLR2.

Example 8-4: Analysis of Gene Expression by Western Blot Assay

To analyze the degree to which the novel complex inhibits TLR2, the cells cultured in Example 8-1 were seeded into a 6-well plate at a density of $1\times10^5$ cells/well, treated with the complex, and then cultured for 24, 48 and 72 hours. After each culture time, protein expression was analyzed using anti-TLR2 antibody (SantaCruz Biotech., USA), anti-p-NF-kB (Cell Signaling, USA), anti-MyD88 (Cell Signaling, USA) and anti-TARC (Abeam, GB) under the conditions disclosed in PCT/KR2017/008636.

The nucleic acid complexes used in this Example are shown in Table 20 below.

[Table 20]

| Nucleic acid complexes for analysis of TLR2 inhibition | |
|---|---|
| No. | Nucleic acid complex |
| 1 | SEQ ID NO: 64 and SEQ ID NO: 65 |
| 2 | SEQ ID NO: 64 and SEQ ID NO: 66 |
| 3 | SEQ ID NO: 64 and SEQ ID NO: 67 |
| 4 | SEQ ID NO: 65 and SEQ ID NO: 69 |
| 5 | SEQ ID NO: 65 and SEQ ID NO: 70 |
| 6 | SEQ ID NO: 65 and SEQ ID NO: 71 |

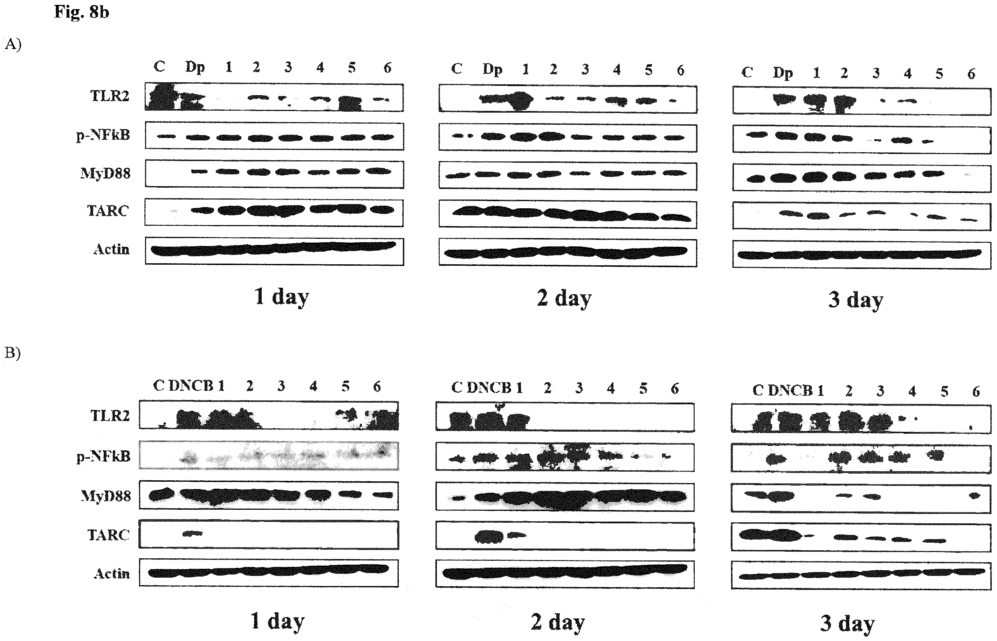

As a result, as shown in FIG. 8b, it was confirmed that treatment with the nucleic acid complex inhibited expression of TLR2 and downstream genes.

Example 8-5: Analysis of Effect against Atopic Dermatitis Phenotype in Atopic Dermatitis-Induced Animal Model Prepared Using House Dust Mite Extract and DNCB

An animal model with atopic dermatitis induced by house dust mites was prepared by shaving the back of NC/Nga mice and applying 100 mg of AD cream (house dust mite extract cream, Biostir, Japan) twice a week for a total of 3 weeks.

In addition, an animal model with atopic dermatitis induced by sick house syndrome was prepared by shaving the back of Balb/C mice and applying 50 μM DNCB twice a week for a total of 3 weeks. Each animal model was treated with a cream formulation of the nucleic complex a total of three times a week, and imaged, and the degree of hair growth on the back was measured by Image J.

The nucleic acid complexes used in this Example are shown in Table 21 below.

[Table 21]

| Nucleic acid complexes for analysis of atopic dermatitis phenotype | |
|---|---|
| Name | Nucleic acid complex |
| PNA 1 | SEQ ID NO: 65 and SEQ ID NO: 67 |
| PNA 2 | SEQ ID NO: 65 and SEQ ID NO: 70 |

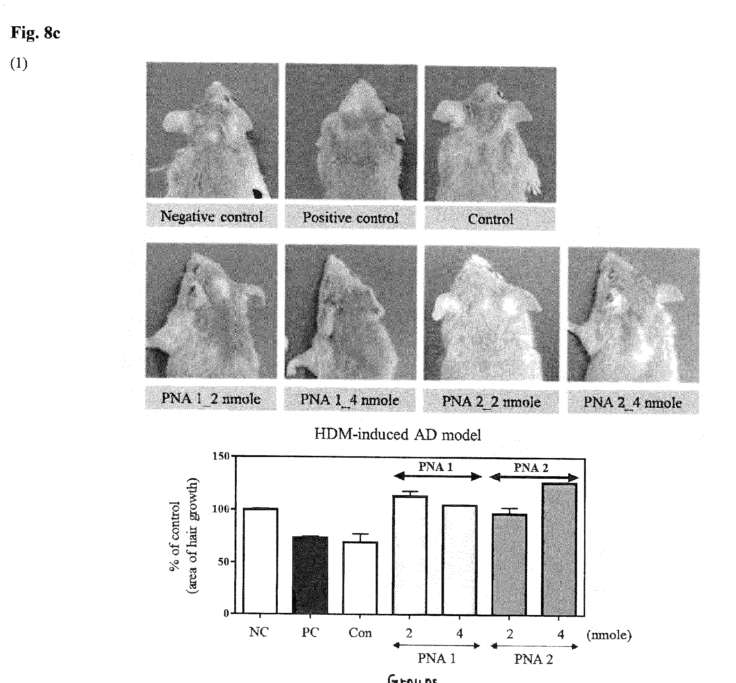

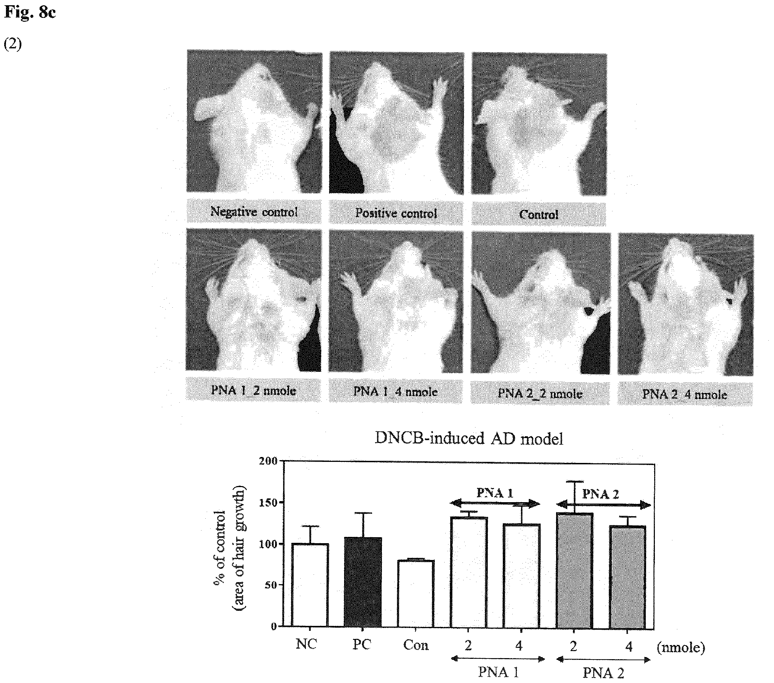

As a result, it was confirmed that the atopic dermatitis phenotype decreased in the group treated with the novel nucleic acid complex (FIG. 8c).

Example 8-6: Analysis of Changes in IgE and TARC Concentrations in Serum

On the last day of the animal experiment performed under the conditions of Example 8-5, mouse blood is collected through the orbital vein, left to stand at room temperature for 2 hours or more, and centrifuged at 14,000 rpm for 15 min, and the serum was collected. The concentrations of IgE and TARC in the collected serum were measured using the experimental methods provided in an IgE ELISA kit (KOMABIOTECH Inc., Korea) and a TARC ELISA kit (R&D System, USA).

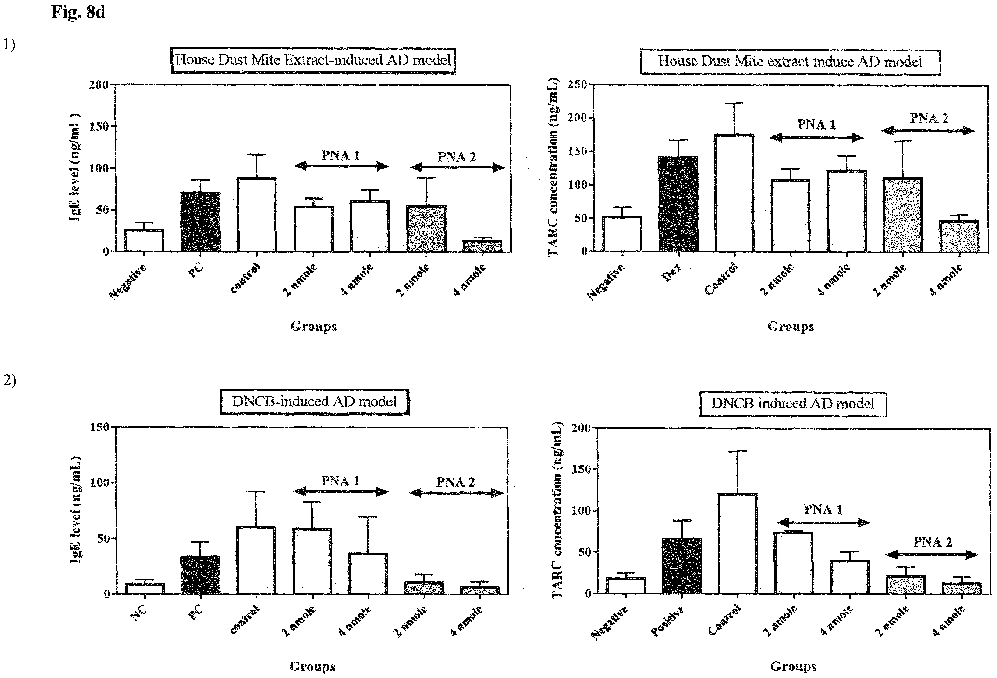

The nucleic acid complexes shown in Table 21 above were used. As a result, it could be confirmed that the concentrations of IgE and TARC in the group treated with the novel nucleic acid complex decreased to levels similar to those in the negative control group, unlike those in the control group with induced atopic dermatitis (FIG. 8d).

Example 8-7: Analysis of Phenotype in Atopy-Induced Animal Model by H&E Staining

On the last day of the animal experiment performed under the conditions of Example 8-5, the mouse ear tissue was biopsied and fixed in 4% formalin solution for one day. The fixed tissue was embedded in paraffin, sectioned to 5 μm, and mounted on a glass slide. The mounted tissue was stained with Hematoxylin:Eoin staining solution for a predetermined time, washed with 1X PBS, and then analyzed with a microscope.

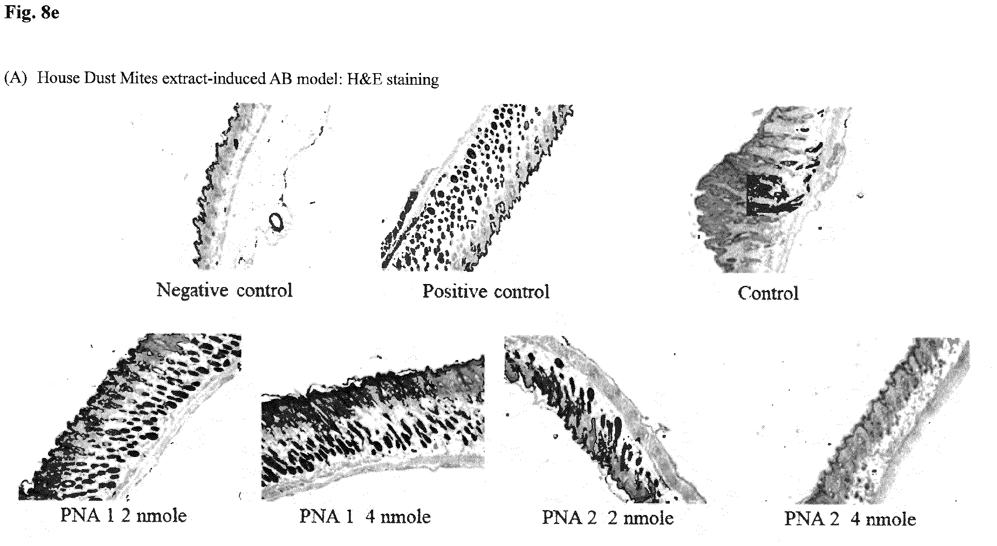

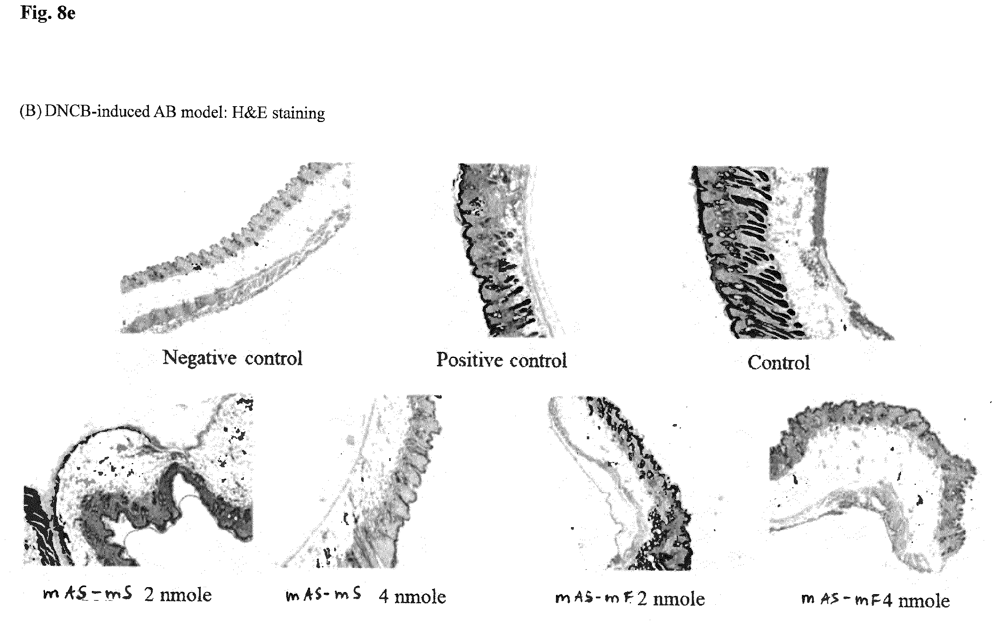

The nucleic acid complexes shown in Table 21 above were used. As a result, it could be confirmed that abnormal growth of epidermis in the group treated with the novel nucleic acid complex decreased compared to that in the psoriasis-derived control group (FIG. 8e).

Example 8-8: Analysis of Inflammatory Marker in Tissue of Atopy-Induced Animal Model by Immunostaining

On the last day of the animal experiment performed under the conditions of Example 8-5, the mouse back tissue was biopsied and fixed in 4% formalin solution for one day. The fixed tissue was embedded in paraffin, sectioned to 5 um, and mounted on a glass slide. The mounted tissue was blocked in 0.5% BSA solution for 1 hour, treated with primary antibody solution against CD3, and incubated for one day. Next, the primary antibody solution was removed, and the remaining material was washed with 1X PBS, treated with secondary antibody solution, incubated at room temperature for 2 hours, and then analyzed by DAB straining.

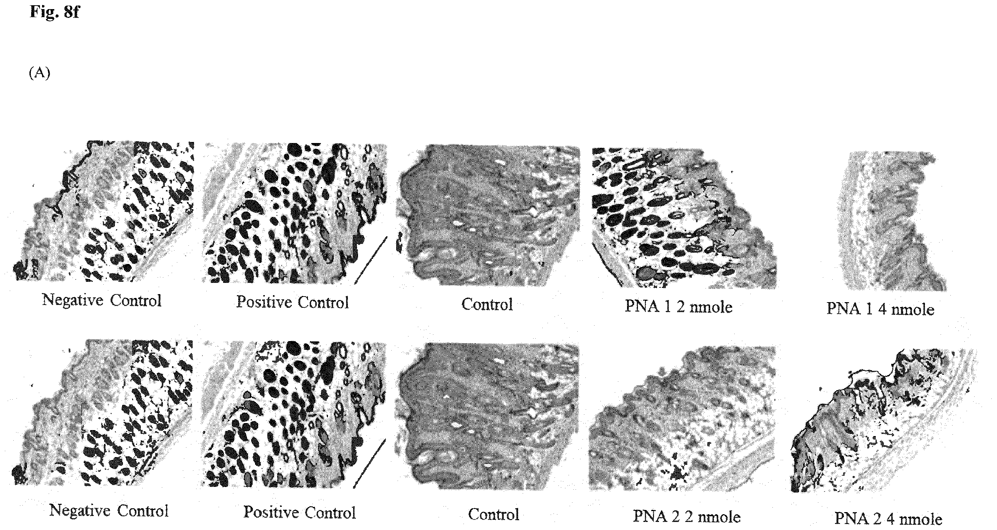

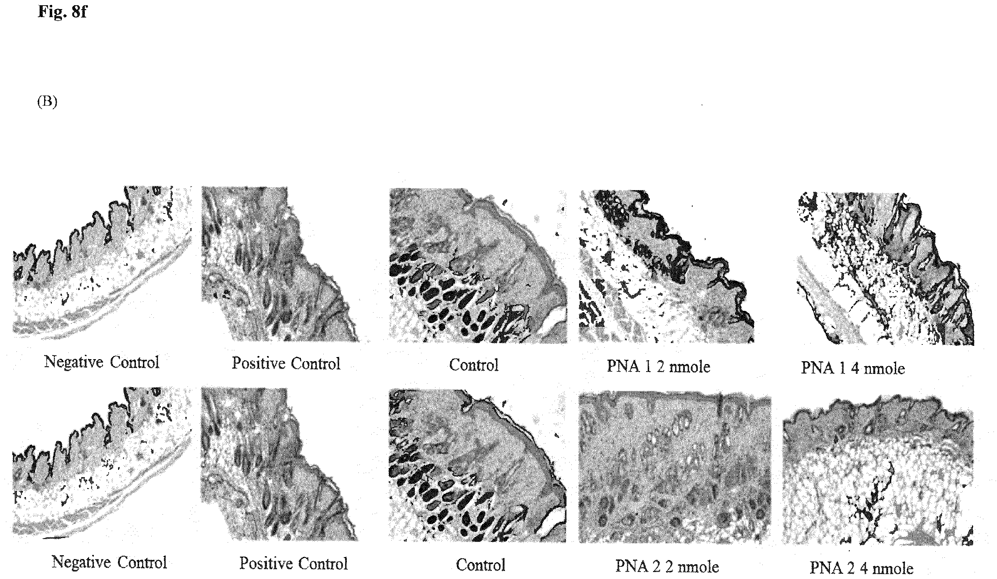

The nucleic acid complexes shown in Table 21 above were used. As a result, the inflammatory marker CD3 in the tissue decreased in the group treated with the novel nucleic acid complex compared to the atopy-induced control group (FIG. 8f) .

**Example 9: Examination of Inhibition of Smad3 in Skin Regeneration Using Novel Complex**

To analyze the skin regeneration effect of the nucleic acid complex, Smad3 was used as a target gene. Smad3 is a protein that is overexpressed in wounded skin, and is considered an important target in skin regeneration.

Example 9-1: Cell Culture

Human keratinocytes (HaCaT) obtained from the CLS (CLS Cell Lines Service, Germany) were cultured in DMEM culture medium (Dulbecco Modified Eagle Medium, Welgene, Korea) containing 10% (v/v) fetal bovine serum, 100 units/ml penicillin and 100 μg/ml streptomycin at 37°C under 5% (v/v) $CO_2$.

Example 9-2. Intracellular Introduction of Complex Comprising Bioactive Peptide Nucleic Acid and Carrier Peptide Nucleic Acid

Novel complexes for inhibiting Smad3 were produced by preparing a bioactive peptide nucleic acid having a sequence (SEQ ID NO: 72 or SEQ ID NO: 73) complementary to Smad3 mRNA, which is overexpressed in wounded portions, preparing carrier peptide nucleic acids (SEQ ID NO: 74 to SEQ ID NO: 78) complementary to the bioactive peptide nucleic acids comprising a material for facilitating endosomal escape, and then hybridizing each bioactive peptide nucleic acid with each carrier peptide nucleic acid in equal amounts (see Table 22). A method for treating the complex in which the bioactive peptide nucleic acid and the carrier peptide nucleic acid are bound to each other is as described in PCT/KR2017/008636.

[Table 22]

| Sequences of bioactive nucleic acids and carrier peptide nucleic acids for inhibiting Smad3 activity | | | |
|---|---|---|---|
| Component | SEQ ID NO | Nucleotide sequence | Monomer modification |
| Bioactive nucleic acid | SEQ ID NO: 72 | 5'-TG$^{(-)}$TCA$^{(+)}$AGCC$^{(-)}$ACT$^{(+))}$GC$^{(-)}$A-O-K-3' | -+-+- |
| | SEQ ID NO: 73 | 5'-GLFDIIKKIAESF-O-TG$^{(-)}$TCA$^{(+)}$AGCC$^{(-)}$ACT$^{(+)}$GC$^{(-)}$A-O-K-3' | -+-+- |
| Carrier peptide | SEQ ID NO: 74 | 5'-K-O-T$^{(+)}$GCA$^{(+)}$G-3' | ++ |
| nucleic acid | SEQ ID NO: 75 | 5'-K-O-T$^{(+)}$GCA$^{(+)}$G-O-Histidine(10)-3' | ++ |
| | SEQ ID NO: 76 | 5'-AC$^{(+)}$AGTTCGG$^{(+)}$TGACG$^{(+)}$T-O-K-3' | +++ |
| | SEQ ID NO: 77 | 5'-K-O-T$^{(+)}$GCAGT$^{(+)}$GGCTTGA$^{(+)}$CA-O-Histidine(10)-3' | +++ |
| | SEQ ID NO: 78 | 5'-Histidine(10)-O-AC$^{(+)}$AGTTCGG$^{(+)}$TGACG$^{(+)}$T-O-K-3' | +++ |

Example 9-3: Analysis of Cell Migration by Wound Healing Assay

To analyze the degree to which the novel complex inhibits Smad3, the cells cultured in Example 9-1 were seeded into a 24-well plate at a density of $1\times10^4$ cells/well, and treated with the complex, and then cultured for 24 and 48 hours. After each culture time, the effect of the complex on cell migration in the artificially wounded portion of the human keratinocytes was analyzed.

The nucleic acid complexes used in this Example are shown in Table 23 below.

[Table 23]

| Nucleic acid complexes for analysis of cell migration in keratinocytes | |
|---|---|
| Name | Nucleic acid complex |
| PNA 1 | SEQ ID NO: 73 and SEQ ID NO: 75 |
| PNA 2 | SEQ ID NO: 73 and SEQ ID NO: 77 |
| PNA 3 | SEQ ID NO: 73 and SEQ ID NO: 78 |

As a result, as shown in FIG. 9a, it was confirmed that the novel complex increased cell migration.

Example 9-4: Analysis of Gene Expression by Western Blot Assay

To analyze the degree to which the novel complex inhibits Smad3, the cells cultured in Example 9-1 were seeded into a 6-well plate at a density of $1\times10^5$ cells/well, treated with the complex, and tthen cultured for 24, 48 and 72 hours. After each culture time, protein expression was analyzed using anti-p-smad3 antibody (Cell Signaling, USA) under the conditions disclosed in PCT/KR2017/008636.

The nucleic acid complexes used in this Example are shown in Table 24 below.

[Table 24]

| Nucleic acid complexes for analysis of Smad3 inhibition | |
|---|---|
| No. | Nucleic acid complex |
| 1 | SEQ ID NO: 73 and SEQ ID NO: 74 |
| 2 | SEQ ID NO: 73 and SEQ ID NO: 75 |
| 3 | SEQ ID NO: 73 and SEQ ID NO: 76 |
| 4 | SEQ ID NO: 73 and SEQ ID NO: 77 |
| 5 | SEQ ID NO: 73 and SEQ ID NO: 78 |
| 6 | SEQ ID NO: 72 and SEQ ID NO: 74 |
| 7 | SEQ ID NO: 72 and SEQ ID NO: 75 |
| 8 | SEQ ID NO: 72 and SEQ ID NO: 76 |
| 9 | SEQ ID NO: 72 and SEQ ID NO: 77 |
| 10 | SEQ ID NO: 72 and SEQ ID NO: 78 |

As a result, as shown in FIG. 9b, it could be confirmed that the novel complex decreased expression of smad3 in human keratinocytes.

**Example 10: Examination of Inhibition of TIEG1 in Keloids Using Novel Complex**

To analyze the therapeutic effect of the novel nucleic acid complex against keloids, TIEG1 was used as a target protein. TIEG1 is a protein that is overexpressed in the skin tissue of keloid patients, and is considered an important target in the treatment of keloids.

Example 10-1: Cell Culture

Human keloid fibroblasts (KEL FIB) were cultured in DMEM culture medium (Dulbecco Modified Eagle Medium, Welgene, Korea) containing 10% (v/v) fetal bovine serum, 100 units/ml penicillin and 100 μg/ml streptomycin at 37°C under 5% (v/v) $CO_2$.

Example 10-2. Intracellular Introduction of Complex Comprising Bioactive Peptide Nucleic Acid and Carrier Peptide Nucleic Acid

Novel complexes for inhibiting TIEG1 were produced by preparing a bioactive peptide nucleic acid having a sequence (SEQ ID NO: 79 or SEQ ID NO: 80) complementary to TIEG1 mRNA, which is overexpressed in keloid parts, preparing carrier peptide nucleic acids (SEQ ID NO: 81 to SEQ ID NO: 84) complementary to the bioactive peptide nucleic acid comprising a material for facilitating endosomal escape, and then hybridizing each bioactive peptide nucleic acid with each carrier peptide nucleic acid in equal amounts (see Table 25). A method for treating the complex in which the bioactive peptide nucleic acid and the carrier peptide nucleic acid are bound to each other is as described in PCT/KR2017/008636.

[Table 25]

| Sequences of bioactive nucleic acids and carrier peptide nucleic acids for inhibiting TIEG1 activity | | | |
|---|---|---|---|
| Component | SEQ ID NO | Nucleotide sequence | Monomer modification |
| Bioactive nucleic acid | SEQ ID NO: 79 | 5'-GC$^{(-)}$TTC$^{(+)}$TAC$^{(-)}$AG$^{(+)}$Crr$^{(-)}$CA-O-K-3' | -+-+- |
| | SEQ ID NO: 80 | 5'-GLFDIIKKIAESF-O-GC$^{(-)}$TTC$^{(+)}$TAC$^{(-)}$AG$^{(+)}$CTT$^{(-)}$CA-O-K -3' | -+-+- |
| Carrier peptide | SEQ ID NO: 81 | 5'-K-O-TG$^{(+)}$AA$^{(+)}$G-3' | ++ |
| nucleic acid | SEQ ID NO: 82 | 5'-K-O-TG$^{(+)}$AA$^{(+)}$G-O-Histidine(10) -3' | ++ |
| | SEQ ID NO: 83 | 5'-K-O-TG$^{(+)}$AAGCTG$^{(+)}$TAGAA$^{(+)}$GC -3' | +++ |
| | SEQ ID NO: 84 | 5'-K-O-TG$^{(+)}$AAGCTG$^{(+)}$TAGAA$^{(+)}$GC-O-Histidine(10) 3' | +++ |

Example 10-3: Analysis of Cell Viability in Human Keloid Fibroblasts

To analyze the degree to which the novel complex inhibits TIEG1, the cells cultured in Example 10-1 were seeded into a 96-well plate at a density of $6\times10^3$ cells/well, and treated with the complex, and then cultured for 24, 48, 72 and 96 hours. After each culture time, cell viability was analyzed under the experimental conditions disclosed in PCT/KR2017/008636.

Nucleic acid complexes (antisense PNA) represented by SEQ ID NO: 79 and each of SEQ ID NOs: 81 to 84, and Nucleic acid complexes (modified antisense PNA) represented by SEQ ID NO: 80 and each of SEQ ID NOs: 81 to 84, were used. As a result, as shown in FIG. 10a, it was confirmed in the human keloid fibroblasts that the novel complex reduced cell viability by inhibiting TIEG1.

Example 10-4: Analysis of Gene Expression by Western Blot Assay

To analyze the degree to which the novel complex inhibits TIEG1, the cells cultured in Example 10-1 were seeded into a 6-well plate at a density of $1\times10^5$ cells/well, treated with the complex, and then cultured for 24, 48 and 72 hours. After each culture time, protein expression was analyzed using anti-TIEG1 antibody (SantaCruz Biotech., USA), anti-p-smad2 (Cell Signaling, USA) and anti-smad7 (SantaCruz Biotech., USA) under the conditions disclosed in PCT/KR2017/008636.

The nucleic acid complexes used in this Example are shown in Table 26 below.

[Table 26]

| Nucleic acid complexes for analysis of TIEG1 inhibition | |
|---|---|
| No. | Nucleic acid complex |
| 1 | SEQ ID NO: 79 and SEQ ID NO: 81 |
| 2 | SEQ ID NO: 79 and SEQ ID NO: 82 |
| 3 | SEQ ID NO: 79 and SEQ ID NO: 83 |
| 4 | SEQ ID NO: 79 and SEQ ID NO: 84 |
| 5 | SEQ ID NO: 80 and SEQ ID NO: 81 |
| 6 | SEQ ID NO: 80 and SEQ ID NO: 82 |
| 7 | SEQ ID NO: 80 and SEQ ID NO: 83 |
| 8 | SEQ ID NO: 80 and SEQ ID NO: 84 |

As a result, as shown in FIG. 10b, it could be confirmed that the novel complex decreased and increased the expression of TIEG1 and downstream genes in human fibroblasts.

**Industrial Applicability**

The nucleic acid complex of Structural Formula (1) according to the present invention, which comprises the bioactive nucleic acid and the carrier peptide nucleic acid, may increase the stability of the bioactive nucleic acid, reduce the loss of the bioactive nucleic acid, such as precipitation caused by self-aggregation, increase the intracellular delivery efficiency of the bioactive nucleic acid, and easily regulate target gene expression.

In particular, since binding between the bioactive nucleic acid and the carrier peptide nucleic in the nucleic acid complex according to the present invention is separated only in the presence of a target gene targeted by the bioactive nucleic acid, the bioactive nucleic acid has high selectivity and specificity for the target gene compared to intracellular introduction of the bioactive nucleic acid alone. In addition, since the complex may be produced by adjusting binding affinity through various structural combinations between the bioactive nucleic acid and the carrier peptide nucleic acid, it is possible to control the point in time when the bioactive nucleic acid acts inside or outside cells.

In addition, as the nucleic acid complex comprises the material for facilitating endosomal escape, the nucleic acid complex entering cells by endocytosis may effectively exhibit activity in the cytoplasm.

**Claims**

1. A nucleic acid complex having a structure of the following Structural Formula (1):

**Structural Formula (1)**

$$[\ mA \equiv mC^{(+)}\ ]$$

wherein,

A represents a bioactive nucleic acid having either a sequence capable of binding to a target gene or a target gene sequence;
C represents a carrier peptide nucleic acid capable of binding to the bioactive nucleic acid;
'=' represents complementary binding between the bioactive nucleic acid and the carrier peptide nucleic acid;
'm' represents a material for facilitating endosomal escape of the bioactive nucleic acid and the carrier peptide nucleic acid, wherein the material for facilitating endosomal escape is GLFDIIKKIAESF (SEQ ID NO: 86) or histidine(10);
the bioactive nucleic acid represented by A is generally negatively charged or neutral;
$C^{(+)}$ indicates that the carrier peptide nucleic acid is generally positively charged; and
the carrier peptide nucleic acid comprises one or more peptide nucleic acid monomers modified such that the carrier peptide nucleic acid is generally positively charged.

2. The nucleic acid complex of claim 1, wherein the bioactive nucleic acid is selected from the group consisting of DNA, RNA, LNA, PNA, and modified nucleic acids.

3. The nucleic acid complex of claim 1, wherein the material for facilitating endosomal escape is bound to the 5'-end and/or 3'-end of each of the bioactive nucleic acid and the carrier peptide nucleic acid.

4. The nucleic acid complex of claim 1, wherein each of the bioactive nucleic acid and the carrier peptide nucleic acid comprises 2 to 50 nucleic acid monomers.

5. The nucleic acid complex of claim 1, wherein the carrier peptide nucleic acid is composed of a nucleotide sequence which is partially or completely complementary to the bioactive nucleic acid.

6. The nucleic acid complex of claim 5, wherein the carrier peptide nucleic acid composed of the nucleotide sequence which is partially complementary to the bioactive nucleic acid comprises one or more universal bases.

7. The nucleic acid complex of claim 1, wherein the carrier peptide nucleic acid comprises one or more gamma- or alpha-backbone-modified peptide nucleic acid monomers so as to be generally positively charged.

**8.** The nucleic acid complex of claim 7, wherein the gamma- or alpha-backbone-modified peptide nucleic acid monomers comprise, in a backbone thereof, one or more positively charged amino acids selected from the group consisting of lysine, arginine, histidine, diamino butyric acid, ornithine, and an amino acid analogue, so as to be electrically positive.

**9.** The nucleic acid complex of claim 7, wherein the gamma- or alpha-backbone-modified peptide nucleic acid monomers comprise, in a backbone thereof, glutamic acid or aspartic acid, or an amino acid analogue which is a negatively charged amino acid.

**10.** The nucleic acid complex of claim 7, wherein the peptide nucleic acid monomers comprise a larger number of monomers having a positively charged amino acid than monomers having a negatively charged amino acid such that the carrier peptide nucleic acid is generally positively charged.

**11.** The nucleic acid complex of claim 1, wherein at least one substance selected from the group consisting of a hydrophobic moiety, a hydrophilic moiety, a target antigen-specific antibody, an aptamer, a quencher, a fluorescent marker and a luminescent marker is bound to the bioactive nucleic acid and/or the carrier peptide nucleic acid.

**12.** The nucleic acid complex of claim 11, wherein the binding of at least one substance, selected from the group consisting of the hydrophobic moiety, the hydrophilic moiety, the target antigen-specific antibody, the aptamer, the quencher, the fluorescent marker, and the luminescent marker, to the bioactive nucleic acid and/or the carrier peptide nucleic acid, is via a single covalent bond or a linker-mediated covalent bond.

**13.** The nucleic acid complex of claim 1, wherein the binding between the bioactive nucleic acid and the carrier peptide nucleic acid is parallel binding, partial specific binding or antiparallel binding according to 5'-directionality and 3'-directionality of each of the nucleic acids.

**14.** The nucleic acid complex of claim 1, wherein the binding affinity between the bioactive nucleic acid and the carrier peptide nucleic acid is lower than the binding affinity between the bioactive nucleic acid and a gene targeted by the bioactive nucleic acid.

**15.** The nucleic acid complex of claim 14, wherein the carrier peptide nucleic acid has at least one peptide nucleobase selected from the group consisting of a linker, a universal base, and a peptide nucleobases which has base not complementary to the corresponding bases of the bioactive nucleic acid.

**16.** The nucleic acid complex of claim 15, wherein the universal base is any one or more selected from the group consisting of inosine PNA, indole PNA, nitroindole PNA, and abasic PNA, which are bases that bind to natural bases, including adenine, guanine, cytosine, thymine, and uracil, without selectivity, and have lower binding affinity than complementary binding affinity.

**17.** The nucleic acid complex of claim 14, wherein the time of separation between the bioactive nucleic acid and the carrier peptide nucleic acid or the time of binding between the bioactive nucleic acid and the gene targeted by the bioactive nucleic acid is controlled by controlling the binding affinity between the bioactive nucleic acid and the carrier peptide nucleic acid.

**18.** The nucleic acid complex of claim 1, wherein the nucleic acid complex has a particle size of 5 nm to 300 nm.

**19.** The nucleic acid complex of claim 18, wherein the particle size of the nucleic acid complex is controlled by controlling the charge balance of the nucleic acid complex.

**20.** A composition comprising the nucleic acid complex according to any one of claims 1 to 19 for use in a method of in-vivo diagnosing, preventing or treating a disease.

**21.** The composition for the use of claim 20, wherein the disease is cancer, inflammatory disease, age-related macular degeneration, diabetic retinopathy, chronic obstructive pulmonary disease (COPD), rare and severe disease, cardiovascular disease, metabolic disease, or skin disease.

**22.** Use of a composition comprising the nucleic acid complex according to any one of claims 1 to 20 in an in-vitro method of diagnosing a disease.

**23.** The composition for the use of claim 20 or 21, wherein a target gene, to which the bioactive nucleic acid contained in the nucleic acid complex binds, is

(a) any one or more selected from the group consisting of VEGF, PD-L1, androgen receptor, clusterin, TGFβR2, ERBB3, ABCB1 and PDE4B, and the disease is cancer, preferably, wherein the cancer is breast cancer, prostate cancer or lung cancer;
(b) VEGF, and the disease is age-related macular degeneration or diabetic retinopathy;
(c) PDE4B, and the disease is chronic obstructive pulmonary disease (COPD); or
(d) any one or more selected from the group consisting of TLR2, Smad3, IFI16, TLR6 and TIEG1, and the disease is skin disease, preferably wherein the skin disease is psoriasis, pigmentation-related skin diseases, atopic dermatitis, skin damage, or keloids.

**Patentansprüche**

**1.** Nukleinsäurekomplex, der eine Struktur der folgenden Strukturformel (1) aufweist:

**Strukturformel (1)**

$$[\ mA \equiv mC^{(+)}\ ]$$

wobei:

A eine bioaktive Nukleinsäure, die entweder eine Sequenz, die an ein Zielgen binden kann, oder eine Zielgensequenz aufweist, darstellt;
C eine Trägerpeptidnukleinsäure, die an die bioaktive Nukleinsäure binden kann, darstellt;
"≡" eine komplementäre Bindung zwischen der bioaktiven Nukleinsäure und der Trägerpeptidnukleinsäure darstellt;
"m" ein Material zur Erleichterung der endosomalen Flucht der bioaktiven Nukleinsäure und der Trägerpeptidnukleinsäure darstellt, wobei das Material zur Erleichterung der endosomalen Flucht GLFDIIKKIAESF (SEQ ID NO: 86) oder Histidin(10) ist;
die durch A dargestellte bioaktive Nukleinsäure im Allgemeinen negativ geladen oder neutral ist;
$C^{(+)}$ bedeutet, dass die Trägerpeptidnukleinsäure im Allgemeinen positiv geladen ist; und
die Trägerpeptidnukleinsäure ein oder mehrere Peptidnukleinsäure-Monomere umfasst, die so modifiziert sind, dass die Trägerpeptidnukleinsäure im Allgemeinen positiv geladen ist.

**2.** Nukleinsäurekomplex nach Anspruch 1, wobei die bioaktive Nukleinsäure aus der Gruppe bestehend aus DNA, RNA, LNA, PNA und modifizierten Nukleinsäuren ausgewählt ist.

**3.** Nukleinsäurekomplex nach Anspruch 1, wobei das Material zur Erleichterung der endosomalen Flucht an das 5'-Ende und/oder das 3'-Ende der bioaktiven Nukleinsäure und der Trägerpeptidnukleinsäure jeweils gebunden ist.

**4.** Nukleinsäurekomplex nach Anspruch 1, wobei jede der bioaktiven Nukleinsäure und der Trägerpeptidnukleinsäure 2 bis 50 Nukleinsäure-Monomere umfasst.

**5.** Nukleinsäurekomplex nach Anspruch 1, wobei die Trägerpeptidnukleinsäure aus einer Nukleotidsequenz, die teilweise oder vollständig komplementär zur bioaktiven Nukleinsäure ist, besteht.

**6.** Nukleinsäurekomplex nach Anspruch 5, wobei die Trägerpeptidnukleinsäure, die aus der Nukleotidsequenz besteht, die teilweise komplementär zur bioaktiven Nukleinsäure ist, eine oder mehrere Universalbasen umfasst.

**7.** Nukleinsäurekomplex nach Anspruch 1, wobei die Trägerpeptidnukleinsäure ein oder mehrere gamma- oder alpha-Rückgratmodifizierte Peptidnukleinsäure-Monomere umfasst, so dass sie im Allgemeinen positiv geladen ist.

**8.** Nukleinsäurekomplex nach Anspruch 7, wobei die gamma- oder alpha-Rückgrat-modifizierten Peptidnukleinsäure-Monomere in ihrem Rückgrat eine oder mehrere positiv geladene Aminosäuren umfassen, die aus der Gruppe bestehend aus Lysin, Arginin, Histidin, Diaminobuttersäure, Ornithin und einem Aminosäureanalogon ausgewählt sind, so dass sie elektrisch positiv sind.

**9.** Nukleinsäurekomplex nach Anspruch 7, wobei die gamma- oder alpha-Rückgrat-modifizierten Peptidnukleinsäure-Monomere in ihrem Rückgrat Glutaminsäure oder Asparaginsäure oder ein Aminosäureanalogon umfassen, das eine negativ geladene Aminosäure ist.

**10.** Nukleinsäurekomplex nach Anspruch 7, wobei die Peptidnukleinsäure-Monomere eine größere Anzahl von Monomeren mit einer positiv geladenen Aminosäure als Monomere mit einer negativ geladenen Aminosäure umfassen, so dass die Trägerpeptidnukleinsäure im Allgemeinen positiv geladen ist.

**11.** Nukleinsäurekomplex nach Anspruch 1, wobei mindestens eine Substanz, die aus der Gruppe bestehend aus einem hydrophoben Rest, einem hydrophilen Rest, einem Zielantigen-spezifischen Antikörper, einem Aptamer, einem Quencher, einem Fluoreszenzmarker und einem Lumineszenzmarker ausgewählt ist, an die bioaktive Nukleinsäure und/oder die Trägerpeptidnukleinsäure gebunden ist.

**12.** Nukleinsäurekomplex nach Anspruch 11, wobei die Bindung mindestens einer Substanz, die aus der Gruppe bestehend aus dem hydrophoben Rest, dem hydrophilen Rest, dem Zielantigen-spezifischen Antikörper, dem Aptamer, dem Quencher, dem Fluoreszenzmarker und dem Lumineszenzmarker ausgewählt ist, an die bioaktive Nukleinsäure und/oder die Trägerpeptidnukleinsäure über eine einzelne kovalente Bindung oder eine Linker-vermittelte kovalente Bindung erfolgt.

**13.** Nukleinsäurekomplex nach Anspruch 1, wobei die Bindung zwischen der bioaktiven Nukleinsäure und der Trägerpeptidnukleinsäure eine parallele Bindung, eine teilweise spezifische Bindung oder eine antiparallele Bindung entsprechend der 5'-Richtung und der 3'-Richtung jeder der Nukleinsäuren ist.

**14.** Nukleinsäurekomplex nach Anspruch 1, wobei die Bindungsaffinität zwischen der bioaktiven Nukleinsäure und der Trägerpeptidnukleinsäure geringer als die Bindungsaffinität zwischen der bioaktiven Nukleinsäure und einem von der bioaktiven Nukleinsäure zielgerichteten Gen ist.

**15.** Nukleinsäurekomplex nach Anspruch 14, wobei die Trägerpeptidnukleinsäure mindestens eine Peptidnukleobase aufweist, die aus der Gruppe bestehend aus einem Linker, einer universellen Base und einer Peptidnukleobase, deren Base zu den entsprechenden Basen der bioaktiven Nukleinsäure nicht komplementär ist, ausgewählt ist.

**16.** Nukleinsäurekomplex nach Anspruch 15, wobei die universelle Base eine Base oder mehrere Basen ist/sind, die aus der Gruppe bestehend aus Inosin-PNA, Indol-PNA, Nitroindol-PNA und abasischer PNA ausgewählt ist/sind, welche Basen sind, die an natürliche Basen, einschließlich Adenin, Guanin, Cytosin, Thymin und Uracil, ohne Selektivität binden und eine geringere Bindungsaffinität als die komplementäre Bindungsaffinität aufweisen.

**17.** Nukleinsäurekomplex nach Anspruch 14, wobei die Zeit der Trennung zwischen der bioaktiven Nukleinsäure und der Trägerpeptidnukleinsäure oder die Zeit der Bindung zwischen der bioaktiven Nukleinsäure und dem Gen, auf das die bioaktive Nukleinsäure gerichtet ist, durch Steuerung der Bindungsaffinität zwischen der bioaktiven Nukleinsäure und der Trägerpeptidnukleinsäure gesteuert wird.

**18.** Nukleinsäurekomplex nach Anspruch 1, wobei der Nukleinsäurekomplex eine Partikelgröße von 5 nm bis 300 nm aufweist.

**19.** Nukleinsäurekomplex nach Anspruch 18, wobei die Partikelgröße des Nukleinsäurekomplexes durch Steuerung des Ladungsausgleichs des Nukleinsäurekomplexes gesteuert wird.

**20.** Zusammensetzung, die den Nukleinsäurekomplex nach einem der Ansprüche 1 bis 19 umfasst, zur Verwendung in einem Verfahren zur In-vivo-Diagnose, -Prävention oder -Behandlung einer Krankheit.

**21.** Zusammensetzung für die Verwendung nach Anspruch 20, wobei die Krankheit Krebs, Entzündungskrankheit, altersbedingte Makuladegeneration, diabetische Retinopathie, chronisch obstruktive Lungenkrankheit (COPD), seltene und schwere Krankheit, kardiovaskuläre Krankheit, Stoffwechselkrankheit oder Hautkrankheit ist.

**22.** Verwendung einer Zusammensetzung umfassend den Nukleinsäurekomplex nach einem der Ansprüche 1 bis 20 in einem In-vitro-Verfahren zur Diagnose einer Krankheit.

**23.** Zusammensetzung für die Verwendung nach Anspruch 20 oder 21, wobei ein Zielgen, an das die in dem Nukleinsäurekomplex enthaltene bioaktive Nukleinsäure bindet,

(a) ein oder mehrere Zielgen(e), das/die aus der Gruppe bestehend aus VEGF, PD-L1, Androgenrezeptor, Clusterin, TGFβR2, ERBB3, ABCB1 und PDE4B ausgewählt ist/sind, ist und die Krankheit Krebs ist, vorzugsweise, wobei der Krebs Brustkrebs, Prostatakrebs oder Lungenkrebs ist;
(b) VEGF ist und die Krankheit altersbedingte Makuladegeneration oder diabetische Retinopathie ist;
(c) PDE4B ist und die Krankheit chronisch obstruktive Lungenerkrankung (COPD) ist; oder
(d) ein oder mehrere Zielgen(e), das/die aus der Gruppe bestehend aus TLR2, Smad3, IFI16, TLR6 und TIEG1 ausgewählt ist/sind, ist und die Krankheit Hautkrankheit, vorzugsweise, wobei die Hautkrankheit Psoriasis, pigmentierungsbedingte Hautkrankheiten, atopische Dermatitis, Hautschäden oder Keloide ist.

**Revendications**

**1.** Complexe d'acide nucléique ayant une structure de la formule structurelle (1) suivante :

Formule structurelle (1)

$$[\ mA \equiv mC^{(+)}\ ]$$

dans lequel,

A représente un acide nucléique bioactif ayant soit une séquence capable de se lier à un gène cible soit une séquence de gène cible ;
C représente un acide nucléique peptidique vecteur capable de se lier à l'acide nucléique bioactif ;
'≡' représente une liaison complémentaire entre l'acide nucléique bioactif et l'acide nucléique peptidique vecteur ;
'm' représente un matériau permettant de faciliter un échappement endosomal de l'acide nucléique bioactif et de l'acide nucléique peptidique vecteur, dans lequel le matériau permettant de faciliter un échappement endosomal est GLFDIIKKIAESF (SEQ ID NO: 86) ou histidine(10) ;
l'acide nucléique bioactif représenté par A est généralement chargé négativement ou neutre ;
$C^{(+)}$ indique que l'acide nucléique peptidique vecteur est généralement chargé positivement ; et
l'acide nucléique peptidique vecteur comprend un ou plusieurs monomères d'acide nucléique peptidique modifiés de telle sorte que l'acide nucléique peptidique vecteur est généralement chargé positivement.

**2.** Complexe d'acide nucléique selon la revendication 1, dans lequel l'acide nucléique bioactif est choisi dans le groupe constitué par ADN, ARN, LNA, PNA et acides nucléiques modifiés.

**3.** Complexe d'acide nucléique selon la revendication 1, dans lequel le matériau permettant de faciliter un échappement endosomal est lié à l'extrémité 5' et/ou à l'extrémité 3' de chacun parmi l'acide nucléique bioactif et l'acide nucléique peptidique vecteur.

**4.** Complexe d'acide nucléique selon la revendication 1, dans lequel chacun parmi l'acide nucléique bioactif et l'acide nucléique peptidique vecteur comprend 2 à 50 monomères d'acide nucléique.

**5.** Complexe d'acide nucléique selon la revendication 1, dans lequel l'acide nucléique peptidique vecteur est composé d'une séquence nucléotidique qui est partiellement ou complètement complémentaire à l'acide nucléique bioactif.

**6.** Complexe d'acide nucléique selon la revendication 5, dans lequel l'acide nucléique peptidique vecteur composé de la séquence nucléotidique qui est partiellement complémentaire à l'acide nucléique bioactif comprend une ou plusieurs bases universelles.

**7.** Complexe d'acide nucléique selon la revendication 1, dans lequel l'acide nucléique peptidique vecteur comprend un ou plusieurs monomères d'acide nucléique peptidique à modification de squelette en gamma ou alpha de façon à être généralement chargé positivement.

**8.** Complexe d'acide nucléique selon la revendication 7, dans lequel les monomères d'acide nucléique peptidique à modification de squelette en gamma ou alpha comprennent, dans un squelette de ceux-ci, un ou plusieurs acides aminés chargés positivement choisis dans le groupe constitué par lysine, arginine, histidine, acide diamino-butyrique, ornithine et un analogue d'acide aminé, de façon à être électriquement positifs.

**9.** Complexe d'acide nucléique selon la revendication 7, dans lequel les monomères d'acide nucléique peptidique à modification de squelette en gamma ou alpha comprennent, dans un squelette de ceux-ci, acide glutamique ou acide aspartique, ou un analogue d'acide aminé qui est un acide aminé chargé négativement.

**10.** Complexe d'acide nucléique selon la revendication 7, dans lequel les monomères d'acide nucléique peptidique comprennent un plus grand nombre de monomères ayant un acide aminé chargé positivement que de monomères ayant un acide aminé chargé négativement de telle sorte que l'acide nucléique peptidique vecteur est généralement chargé positivement.

**11.** Complexe d'acide nucléique selon la revendication 1, dans lequel au moins une substance choisie dans le groupe constitué par un fragment hydrophobe, un fragment hydrophile, un anticorps spécifique à un antigène cible, un aptamère, un agent d'inactivation, un marqueur fluorescent et un marqueur luminescent est liée à l'acide nucléique bioactif et/ou à l'acide nucléique peptidique vecteur.

**12.** Complexe d'acide nucléique selon la revendication 11, dans lequel la liaison d'au moins une substance, choisie dans le groupe constitué par le fragment hydrophobe, le fragment hydrophile, l'anticorps spécifique à un antigène cible, l'aptamère, l'agent d'inactivation, le marqueur fluorescent et le marqueur luminescent, à l'acide nucléique bioactif et/ou à l'acide nucléique peptidique vecteur, est par l'intermédiaire d'une liaison covalente simple ou d'une liaison covalente médiée par lieur.

**13.** Complexe d'acide nucléique selon la revendication 1, dans lequel la liaison entre l'acide nucléique bioactif et l'acide nucléique peptidique vecteur est une liaison parallèle, une liaison spécifique partielle ou une liaison antiparallèle selon la directionnalité 5' et la directionnalité 3' de chacun des acides nucléiques.

**14.** Complexe d'acide nucléique selon la revendication 1, dans lequel l'affinité de liaison entre l'acide nucléique bioactif et l'acide nucléique peptidique vecteur est inférieure à l'affinité de liaison entre l'acide nucléique bioactif et un gène ciblé par l'acide nucléique bioactif.

**15.** Complexe d'acide nucléique selon la revendication 14, dans lequel l'acide nucléique peptidique vecteur a au moins une nucléobase peptidique choisie dans le groupe constitué par un lieur, une base universelle et une nucléobase peptidique qui a une base non complémentaire aux bases correspondantes de l'acide nucléique bioactif.

**16.** Complexe d'acide nucléique selon la revendication 15, dans lequel la base universelle est l'une quelconque ou plusieurs quelconques choisies dans le groupe constitué par ANP inosine, ANP indole, ANP nitro-indole et ANP abasique, qui sont des bases qui se lient à des bases naturelles, comportant adénine, guanine, cytosine, thymine et uracile, sans sélectivité, et ont une affinité de liaison plus basse qu'une affinité de liaison complémentaire.

**17.** Complexe d'acide nucléique selon la revendication 14, dans lequel le temps de séparation entre l'acide nucléique bioactif et l'acide nucléique peptidique vecteur ou le temps de liaison entre l'acide nucléique bioactif et le gène ciblé par l'acide nucléique bioactif est commandé en commandant l'affinité de liaison entre l'acide nucléique bioactif et l'acide nucléique peptidique vecteur.

**18.** Complexe d'acide nucléique selon la revendication 1, dans lequel le complexe d'acide nucléique a une taille de particules de 5 nm à 300 nm.

**19.** Complexe d'acide nucléique selon la revendication 18, dans lequel la taille de particules du complexe d'acide nucléique est commandée en commandant l'équilibre de la charge du complexe d'acide nucléique.

**20.** Composition comprenant le complexe d'acide nucléique selon l'une quelconque des revendications 1 à 19 pour utilisation dans un procédé de diagnostic in vivo, de prévention ou de traitement d'une maladie.

**21.** Composition pour l'utilisation selon la revendication 20, dans laquelle la maladie est un cancer, une maladie inflammatoire, une dégénérescence maculaire liée à l'âge, une rétinopathie diabétique, une maladie pulmonaire obstructive chronique (MPOC), une maladie rare et grave, une maladie cardiovasculaire, une maladie métabolique ou une maladie de la peau.

**22.** Utilisation d'une composition comprenant le complexe d'acide nucléique selon l'une quelconque des revendications 1 à 20 dans un procédé in vitro de diagnostic d'une maladie.

**23.** Composition pour l'utilisation selon la revendication 20 ou 21, dans laquelle un gène cible, auquel l'acide nucléique bioactif contenu dans le complexe d'acide nucléique se lie, est

(a) l'un quelconque plusieurs quelconques choisis dans le groupe constitué par VEGF, PD-L1, récepteur d'androgène, clusterine, TGFβR2, ERBB3, ABCB1 et PDE4B, et la maladie est un cancer, de préférence, dans laquelle le cancer est un cancer du sein, un cancer de la prostate ou un cancer du poumon ;
(b) VEGF, et la maladie est une dégénérescence maculaire liée à l'âge ou une rétinopathie diabétique ;
(c) PDE4B, et la maladie est une maladie pulmonaire obstructive chronique (MPOC) ; ou
(d) l'un quelconque plusieurs quelconques choisis dans le groupe constitué par TLR2, Smad3, IFI16, TLR6 et TIEG1, et la maladie est une maladie de la peau, de préférence dans laquelle la maladie de la peau est le psoriasis, des maladies cutanées liées à la pigmentation, une dermatite atopique, des lésions cutanées ou des chéloïdes.

**Fig. 1a**

(1)

24 h

Original PNA 1
Modified PNA 1

Fig. 1 a

(1)

48h

Original PNA 1
Modified PNA 1

**Fig. 1a**

(1)

72 h

Original PNA 1
Modified PNA 1

**Fig. 1a**

(1)

96 h

Original PNA 1

Modified PNA 1

**Fig. 1a**

(2)

24 h

Original PNA 2
Modified PNA 2

Fig. 1a

(2)

48 h
Original PNA 2
Modified PNA 2

**Fig. 1a**

(2)

72 h

Original PNA 2
Modified PNA 2

**Fig. 1a**

(2)

96 h
Original PNA 2
Modified PNA 2

**Fig. 1b**

(1)

Cell viability (% of control)
Control
0
Original
100 nM
500 nM
PNA 1
100 nM
500 nM
PNA 2
100 nM
500 nM
1 day
2 day
3 day
4 day
5 day

Fig. 1b

(2)

Cell viability (% of control)
Control
0
100 nM
500 nM
Original
PNA 3
PNA 4
PNA 5
PNA 6
1 day
2 day
3 day
4 day
5 day

Fig. 1c

(1)

Cell viability (% of control)
120
100
80
60
40
20
0
100 nM
500 nM
100 nM
500 nM
100 nM
500 nM
Control
Original
PNA 1
PNA 2
1 day
2 day
3 day
4 day
5 day

**Fig. 1c**

(2)

Cell viability (% of control)
0
100 nM
500 nM
Control
Original
PNA 3
PNA 4
PNA 5
PNA 6
1 day
2 day
3 day
4 day
5 day

Fig. 1d

(1)

Con PNA 1 PNA 2
Con PNA 1 PNA 2
Con PNA 1 PNA 2
Con PNA 1 PNA 2 (500nM)
VEGF
p-Akt
Actin
24h
48h
72h
96h

(2)

Con PNA 3 PNA 4
Con PNA 3 PNA 4
Con PNA 3 PNA 4
Con PNA 3 PNA 4 (500nM)
VEGF
p-Akt1
Actin
24h
48h
72h
96h

Control
Original PNA
Modified PNA Duplex
24 h
48 h
72 h

**Fig. 1e**

(1)

Control
Modified PNA Duplex 1
Modified PNA Duplex 2
24 h
48 h
72 h
0.8 %
0.8 %
0.4 %
2.5 %
0.1 %
2.7 %
0.1 %
0.8 %
0.1 %
0.1 %
1.6 %
1.8 %
0.2 %
3.4%
0.1 %


**Fig. 1e**

(2)

Fig. 2a

In vivo animal test design

Weekly twice IV injection
PNA
IV injection
PNA
treatment
PNA
treatment
PNA
treatment
PNA
treatment
PNA
treatment
Xenograf
(8/2)
Grouping
(8/11)
Mice
Scarified

Mouse group (1mg/kg, 2mg/kg)

1. Control
2. Sample 1 – PNA duplex (VEGF)
3. Sample 2 – PNA duplex (VEGF)
4. Sample 3 – PNA duplex (combination – VEGF & PD-L1)
5. Sample 4 – PNA duplex (combination – VEGF & PD-L1)

Fig. 2b

(A)
Body weight changes(%)
Days after treatment
Control(1 mg/kg)
Sample 1(1 mg/kg)
Sample 2(1 mg/kg)
Sample 3(1 mg/kg)
Sample 4(1 mg/kg)
(B)
Body weight changes(%)
Days after treatment
Control(2 mg/kg)
Sample 1(2 mg/kg)
Sample 2(2 mg/kg)
Sample 3(2 mg/kg)
Sample 4(2 mg/kg)

Fig. 2c

(A)
Control(1 mg/kg)
Sample 1(1 mg/kg)
Sample 2(1 mg/kg)
Sample 3(1 mg/kg)
Sample 4(1 mg/kg)
** p<0.01
*** p<0.001
Tumor volume(Vt-Vo)
Days after treatment
(B)
Control(2 mg/kg)
Sample 1(2 mg/kg)
Sample 2(2 mg/kg)
Sample 3(2 mg/kg)
Sample 4(2 mg/kg)
*** p<0.001
Tumor volume(Vt-Vo)
Days after treatment

**Fig. 2d**

(A)
Tumor weight(mg)
1200
900
600
300
0
Control
Sample 1
Sample 2
Sample 3
Sample 4
1 mg/kg
(B)
Tumor weight(mg)
1200
900
600
300
0
Control
Sample 1
Sample 2
Sample 3
Sample 4
2 mg/kg
** p<0.01, *** p<0.001

Fig. 2e

Control (1 mg/kg)

Sample 1(1 mg/kg)

Sample 2(1 mg/kg)

Sample 3(1 mg/kg)

Sample 4(1 mg/kg)

Control (2 mg/kg)

Sample 1(2 mg/kg)

Sample 2(2 mg/kg)

Sample 3(2 mg/kg)

Sample 4(2 mg/kg)

Fig. 2f

1 mg/kg
2 mg/kg
ALT activity (IU/L)
0
5
10
15
20
control
group 1
group 2
group 3
group 4

1 mg/kg
2 mg/kg
AST activity (IU/L)
0
20
40
60
80
100
control
group 1
group 2
group 3
group 4

1 mg/kg
2 mg/kg
Creatinine
concentraion (mM)
0.00
0.02
0.04
0.06
0.08
0.10
control
group 1
group 2
group 3
group 4

Fig. 2g

PNA duplex(2mg/kg)
Con S1 S2 S3 S4
VEGF
VEGFR 1
VEGFR 2
Actin
#1
#2
p-Akt1
p-STAT3
Bcl 2
PD-L1
Survivin
Cyclin D 1

Fig. 3

PNA duplex(2mg/kg)
Con G1 G2 G3 G4
#1
PNA duplex(2mg/kg)
Con G1 G2 G3 G4
#2
PD-L1
Actin
Scrambled PNA
Group 1 (2 mg/kg)
Group 2 (2 mg/kg)
Group 3 (2 mg/kg)
Group 4 (2 mg/kg)

Fig. 4

(A)

Con DHT 1 2 3 4 5 6 7 8
72 h
96 h
120 h
144 h
168 h
Androgen Receptor (AR)
p-Akt
Actin

**Fig. 4**

(B)

Con DHT 11 12 13 14 15 16 17 18
72 h
96 h
120 h
144 h
168 h
Androgen Receptor (AR)
p-Akt
Actin

Fig. 5a

1)
Original PNA Duplex 1
Original PNA Duplex 2
Original PNA Duplex 3
Original PNA Duplex 4
Cell Viability ( % of control )
0
4 nM
20 nM
100 nM
500 nM
1 day
2 day
3 day
4 day
5 day

Fig. 5a

(2)

Original PNA Duplex 5
Original PNA Duplex 7
Original PNA Duplex 6
Original PNA Duplex 8
Cell Viability ( % of control )
0
20
40
60
80
100
120
140
0
4 nM
20 nM
100 nM
500 nM
1 day
2 day
3 day
4 day
5 day

Fig. 5b

(1)

Modified PNA Duplex 1
Modified PNA Duplex 2
Modified PNA Duplex 3
Modified PNA Duplex 4
Cell Viability ( % of control )
0
4 nM
20 nM
100 nM
500 nM
1 day
2 day
3 day
4 day
5 day

Fig. 5b

(2)

Modified PNA Duplex 5
Modified PNA Duplex 6
Modified PNA Duplex 7
Modified PNA Duplex 8
Cell Viability ( % of control )
0
4 nM
20 nM
100 nM
500 nM
1 day
2 day
3 day
4 day
5 day

**Fig. 5c**

(1)

Con 1 2 3 4 5 6 7 8
24 h
48 h
72 h
96 h
120 h
Clusterin
BAX
Actin

**Fig. 5c**

(2)

Con 11 12 13 14 15 16 17 18
24 h
48 h
72 h
96 h
120 h
Clusterin
BAX
Actin

**Fig. 6a**

(1)

Cell viability (% of control)
0
100 nM
500 nM
100 nM
500 nM
100 nM
500 nM
Control
Original
PNA 1
PNA 2
1 day
2 day
3 day
4 day
5 day

**Fig. 6a**

(2)

1 day
2 day
3 day
4 day
5 day
Cell viability (% of control)
0
Control
100 nM
500 nM
Original
100 nM
500 nM
PNA 3
100 nM
500 nM
PNA 4
Cell viability (% of control)
0
Control
100 nM
500 nM
PNA 5
100 nM
500 nM
PNA 6

**Fig. 6b**

(1)

Con PNA 1 PNA 2
Con PNA 1 PNA 2
Con PNA 1 PNA 2
Con PNA 1 PNA 2
Con PNA 1 PNA 2 (500nM)
VEGF
p-Akt1
Actin
24h
48h
72h
96h
120h
(2)
Con PNA 3 PNA 4
Con PNA 3 PNA 4
Con PNA 3 PNA 4
Con PNA 3 PNA 4
Con PNA 3 PNA 4 (500nM)
VEGF
p-Akt1
Actin
24h
48h
72h
96h
120h

**Fig. 6c**

PNA IVT injection
P0
7day
P7
Macular
Peripheral Retina
Control
PNA
Duplex

Fig. 6d

PNA IVT injection
P16
6 day
P22
P25
Negative Control
(N.C.)
Control
Modified
PNA Duplex 1
Modified
PNA Duplex 2
Macular
Peripheral
Retina

**Fig. 6e**

PNA IVT injection
P16
6 day
P22
3 day
P25
Negative Control (N.C.)
Control
Modified PNA Duplex 1
Modified PNA Duplex 2
Macular
Peripheral Retina

Fig. 6f

PNA eyedrops
P14
P15
5 day
P19
Negative Control (N.C.)
Control
Modified PNA Duplex 1
Modified PNA Duplex 2
Macular
Peripheral Retina

Fig. 7

(A)
CSE (10ug/mg)
N.C con 1 2 3 4 5 6 7 8
CSE (10ug/mg)
N.C con 1 2 3 4 5 6 7 8
CSE (10ug/mg)
N.C con 1 2 4 3 5 6 7 8 (500nM)
PDE4B
IL-6
Actin
72h
96h
120h
(B)
LPS (10ug/mg)
N.C con 1 2 3 4 5 6 7 8
LPS (10ug/mg)
N.C con 1 2 3 4 5 6 7 8
LPS (10ug/mg)
N.C con 1 2 3 4 5 6 7 8 (500nM)
PDE4B
TNFα
Actin
72h
96h
120h

Fig. 8a

Dp 10 μg/mL
Cell viability (% of control)
0
20
40
60
80
100
120
Control
100 nM
500 nM
100 nM
500 nM
100 nM
500 nM
Antisense PNA / Modified Carrier PNA
1 day
2 day
3 day
Dp 10 μg/mL
Cell viability (% of control)
Control
100 nM
500 nM
100 nM
500 nM
100 nM
500 nM
Modified Antisense PNA / Modified Carrier PNA
1 day
2 day
3 day
Inducer: Dp (HDM extract)

Fig. 8b

A)
C Dp 1 2 3 4 5 6
TLR2
p-NFkB
MyD88
TARC
Actin
1 day
2 day
3 day
B)
C DNCB 1 2 3 4 5 6
TLR2
p-NFkB
MyD88
TARC
Actin
1 day
2 day
3 day

**Fig. 8c**

(1)

Negative control
Positive control
Control
PNA 1_2 nmole
PNA 1_4 nmole
PNA 2_2 nmole
PNA 2_4 nmole
HDM-induced AD model
% of control
(area of hair growth)
150
100
50
0
PNA 1
PNA 2
NC
PC
Con
2
4
2
4
(nmole)
PNA 1
PNA 2

**Fig. 8c**

(2)

Negative control
Positive control
Control
PNA 1_2 nmole
PNA 1_4 nmole
PNA 2_2 nmole
PNA 2_4 nmole
DNCB-induced AD model
% of control
(area of hair growth)
200
150
100
50
0
NC
PC
Con
2
4
2
4
(nmole)
PNA 1
PNA 2

Fig. 8d

1)
House Dust Mite Extract-induced AD model
IgE level (ng/mL)
200
150
100
50
0
PNA 1
PNA 2
Negative
PC
control
2 nmole
4 nmole
2 nmole
4 nmole
Groups
House Dust Mite extract induce AD model
TARC concentration (ng/mL)
250
200
150
100
50
0
PNA 1
PNA 2
Negative
Dex
Control
2 nmole
4 nmole
2 nmole
4 nmole
Groups
2)
DNCB-induced AD model
IgE level (ng/mL)
150
100
50
0
PNA 1
PNA 2
NC
PC
control
2 nmole
4 nmole
2 nmole
4 nmole
Groups
DNCB induced AD model
TARC concentration (ng/mL)
200
150
100
50
0
PNA 1
PNA 2
Negative
Positive
Control
2 nmole
4 nmole
2 nmole
4 nmole
Groups

**Fig. 8e**

(A) House Dust Mites extract-induced AB model: H&E staining

Negative control

Positive control

Control

PNA 1 2 nmole

PNA 1 4 nmole

PNA 2 2 nmole

PNA 2 4 nmole

Fig. 8e

(B) DNCB-induced AB model: H&E staining

Negative control
Positive control
Control
mAS-mS 2 nmole
mAS-mS 4 nmole
mAS-mF 2 nmole
mAS-mF4 nmole

**Fig. 8f**

(A)

Negative Control
Positive Control
Control
PNA 1 2 nmole
PNA 1 4 nmole
Negative Control
Positive Control
Control
PNA 2 2 nmole
PNA 2 4 nmole

Fig. 8f

(B)

Negative Control
Positive Control
Control
PNA 1 2 nmole
PNA 1 4 nmole
Negative Control
Positive Control
Control
PNA 2 2 nmole
PNA 2 4 nmole

Fig. 9a

1 Day
2 Day
Control
TGFβ-1
PNA 1
PNA 2
PNA 3

Fig. 9b

Dose: 500 nM

TGFß-1 5 ng/ml PNA duplex

C T 1 2 3 4 5 6 7 8 9 10
p-smad3
Actin
24 h
48 h
72 h

**Fig. 10a**

A)
Cell viability (% of control)
120
100
80
60
40
20
0
Control
100 nM
500 nM
100 nM
500 nM
100 nM
500 nM
100 nM
500 nM
Antisense PNA / Various Carrier PNA
1 day
2 day
3 day
4 day
B)
Cell viability (% of control)
120
100
80
60
40
20
0
Control
100 nM
500 nM
100 nM
500 nM
100 nM
500 nM
100 nM
500 nM
Modified Antisense PNA / Various Carrier PNA
1 day
2 day
3 day
4 day

Fig. 10b

TIEG1
p-smad2
smad7
Actin
C 1 2 3 4 5 6 7 8
24 h
48 h
72 h

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5719262 A
- WO 9913719 A1
- WO 2017015109 A1
- KR 2017008636 W
- US 4235871 A
- US 4501728 A
- US 4837028 A
- US 5019369 A


### Non-patent literature cited in the description


- **KOLE R. et al.** *Nature Rev. Drug Discov.*, 2012, vol. 11, 125-140
- **WILSON C. et al.** *Curr. Opin. Chem. Bio.*, 2006, vol. 10, 607-614
- **JOERGENSEN M. et al.** *Oligonucleotides*, 2011, vol. 21, 29-37
- **COUTO L. B. et al.** *Curr. Opin. Pharmacol.*, 2010, vol. 5, 534-542
- **ZHI D. et al.** *Bioconjug. Chem.*, 2013, vol. 24, 487-519
- **BUYENS K. et al.** *J. Control Release*, 2012, vol. 158, 362-70
- **ROSSI, J. J. et al.** *Gene Ther.*, 2006, vol. 13, 583-584
- **YOUSEFI A. et al.** *J. Control Release*, 2013, vol. 170, 209-18
- **TRABULO S. et al.** *Curr. Pharm. Des.*, 2013, vol. 19, 2895-923
- **D. W. PACK** ; **A. S. HOFFMAN** ; **S. PUN** ; **P. S. STAYTON**. Design and development of polymers for gene delivery. *Nat. Rev. Drug. Discov.*, 2005, vol. 4, 581-593
- Remington's Pharmaceutical Science. Mack Publishing Company
- **SZOKA et al.** *Ann. Rev. Biophys. Bioeng.*, 1980, vol. 9, 467
- **BERKMAN et al.** *J Clin Invest.*, 1993, vol. 91, 153-159
- **FERRARA.N. et al.** *Nat Rev Cancer*, 2002, vol. 2, 795-803
- **ZENG H. et al.** *J Biol. Chem.*, 2001, vol. 276, 26969-26976